# EUROPEAN PATENT APPLICATION

(11) **EP 3 815 778 A1**
(43) Date of publication of application: **05.05.2021**
(21) Application number: 19207015.9
(22) Date of filing: 04.11.2019
(51) Int. Cl.: B01J 19/08, B01J 19/00, B01J 19/12, B01J 20/34, B01J 38/32

(54) **DEVICE FOR RAPID AUTOMATED SYNTHESIS OF OLIGO- AND POLYSACCHARIDES**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention generally relates to automated synthesis technology, and more particularly, to a device and method for automated synthesis of oligo- and polysaccharides on a solid support. In particular the present invention relates to a device for automated synthesis of oligo- and polysaccharides on a solid support comprising a reaction vessel, a reagent storing component, a reagent delivery system, a cooling device for cooling reaction vessel, and a pre-cooling device for pre-cooling the reagents to be supplied.

## Description

### Field of the invention

The present invention generally relates to automated synthesis technology, and more particularly, to a device and method for automated synthesis of oligo- and polysaccharides on a solid support. In particular the present invention relates to a device for automated synthesis of oligo- and polysaccharides on a solid support comprising a reaction vessel, a reagent storing component, a reagent delivery system, a cooling device for cooling the reaction vessel, and a pre-cooling device for pre-cooling the reagents to be supplied.

### Background of the invention

Certain biopolymers, including polypeptides and polynucleotides are routinely synthesized by solid-phase methods in which individual subunits are added stepwise to a growing chain tethered to a solid support. Those approaches can be carried out using commercially available synthesizers in an automated or semi-automated manner. However, those general synthesizers do not allow the efficient synthesis of oligosaccharides on a solid support and typically result in poor yields and selectivity, due to the lack of control of reaction temperature.

Polysaccharides are the most abundant biomolecules on Earth and are essential for many vital biological functions. Energy, structural support, cell proliferation and differentiation, host-pathogen recognition and cancer invasion are among the numerous areas that oligosaccharides and glycoconjugates participate and regulate. Because biochemical techniques to purify pure oligosaccharides from natural sources are difficult and inefficient, chemical and enzymatic approaches, or combinations thereof, are often the only reliable methods to access pure oligosaccharides. Efforts to reduce the time and resources spent on traditional chemical syntheses have focused mainly on automated glycan assembly (AGA) on solid supports.

The advantages of solid-support synthesis have been appreciated since the development of peptide synthesis by Merrifield and oligonucleotide synthesis on polymer supports developed by Caruthers. Automated synthesis machines carry out the reaction sequences and the reactions are carried out in high yields by using excess reagents, which are simply removed by washing steps. Separation from the solid carrier and purification as post-automation steps are typically standardized. The keys to successful automated synthesis are reliable building blocks and resins, high-yield couplings and reliable instrumentation.

Since the introduction of AGA by Seeberger in 2001, many aspects of the synthesis process have been systematically improved. Synthetic glycans of increasing length and complexity up to a chain length of 50 units were assembled with the help of AGA. The quality control of the stereo- and regiochemical composition of synthetic products was greatly accelerated by the use of ion mobility mass spectrometry (IM-MS). The overall AGA process was greatly improved and led to the development of the first commercial Glyconeer 2.1® automated oligosaccharide synthesizer.

Depending on the molecular weight of the sugar, 10-50 mg of completely protected oligosaccharide is obtained from the resin in this batch size. After deprotection and purification of the final product, usually only 2-15 mg of the desired oligosaccharide product remain. These amounts are often sufficient for biochemical investigations and have been successfully used to produce glycan arrays. However, significantly more material is required for studies in other scientific areas. Carbohydrates in the form of cellulose and chitin are the two dominant biopolymers that require 100 mg or more of synthetic compounds to investigate their material properties.

It is therefore the objective of the present invention to provide an improved device and method for automated synthesis of oligo- and polysaccharides on a solid support. Especially desired is a device for easily scaling up the synthesis of oligo- and polysaccharides on a solid support, shortening the reaction time, increasing yield and decreasing side and decomposition products.

The objective of the present invention is solved by the teachings of the independent claims. Further advantageous features, aspects and details of the invention are evident from the dependent claims, the description, the figures, and the examples of the present application.

### Brief description of the invention

The present invention relates to a device for automated synthesis of oligo- and polysaccharides on a solid support, the device (100) comprising:
(a) a reaction vessel (400),
(b) a reagent storing component (660),
(c) a reagent delivery system (600),
(d) a cooling device (350) for cooling the reaction vessel (400),
(e) and a pre-cooling device (300) for pre-cooling the reagents to be supplied.

According to an aspect of the present invention the device may comprise a computing device (200) comprising at least one processor configured to control one or more components of the device for automated synthesis of oligo- and polysaccharides on a solid support.

According to an aspect of the present invention the reaction vessel (400) may be adapted for receiving the solid support.

According to an aspect of the present invention the device may be adapted for reactions under anhydrous and inert atmosphere.

According to an aspect of the present invention the device may further comprise an inert gas delivery system (800).

According to an aspect of the present invention the cooling device (350) may comprise a cooling jacket (307) or a cooling coil.

According to an aspect of the present invention the pre-cooling device (350) may be in thermal communication with the reagent delivery system (600).

According to an aspect of the present invention the pre-cooling device (300) may be adapted to cool the reagents to be supplied to a temperature not higher than +3°C of the temperature in the reaction vessel.

According to an aspect of the present invention the pre-cooling device (300) may be adapted to cool the reagents to be supplied in a temperature range of +3°C to -3°C of the temperature in the reaction vessel or more precisely of the temperature of the content in the reaction vessel.

According to an aspect of the present invention the pre-cooling device (300) may be a part or component of the cooling device (350) using the same cooling circuit together. Reworded, the pre-cooling device (300) and the cooling device (350) may form a single component.

According to an aspect of the present invention the reaction vessel (400) may be provided as an interchangeable reaction vessel.

According to an aspect of the present invention the reaction vessel (400) may be made of fluoropolymers such as perfluoroalkoxy alkanes (PFA).

According to an aspect of the present invention the device may comprise a reagent storing component (660) suitable for storing at least building blocks and/or building block solution, activators and/or activator solutions, washing solutions and deprotection solutions.

According to an aspect of the present invention the reagent delivery system (600) connects the reagent storing component (660) with the reaction vessel (400).

According to an aspect of the present invention the device may further comprise a microwave generator component (500).

According to an aspect of the present invention the reaction vessel may be a microwave transparent reaction vessel. According to an aspect of the present invention the cooling jacket or the cooling coil may be a microwave transparent cooling jacket or cooling coil.

In one embodiment of the present invention, the device comprises a first reagent delivery system (600) or top delivery system comprising a distribution component (651, 652, 653) or top distribution component for delivery of building blocks and/or building block solutions, activators and/or activator solutions and washing solution; and a second reagent delivery system (700) or bottom delivery system comprising a distribution component (719, 720) or bottom distribution component for delivery of deprotection solutions and optionally capping solutions. Preferably the device further comprises an exhaust gas exit (655).

According to an aspect of the present invention the device may further comprise a light source for cleaving photo-cleavable linkers.

### Description of the invention

Solid phase synthesis of oligo- or polysaccharides has been proven as suitable synthesis strategy for automation of the synthesis process. For solid phase synthesis of oligo- or polysaccharides the repetitive nature of glycosylation and deprotection can easily be framed into a coupling cycle. Excess reagents may be used to drive reactions to completion, while resin washes, for example through use of solvents, remove any soluble impurities. Only a single purification step is necessary after the oligo- or polysaccharide is liberated from the solid support.

The term **"glycosylation reaction"** or **"coupling reaction"** as used herein relates in the most general sense to a formation of an acetal connecting two sugar units. The anomeric substituent of a glycosyl donor acts as a leaving group thereby generating an electrophilic intermediate. Reaction of this species with a nucleophile, typically a hydroxyl group of a glycosyl acceptor, leads to the formation of a glycosidic linkage. Thus, the term "glycosylation reaction" or "coupling reaction" refers to reactions between a glycosyl donor and a glycosyl acceptor, wherein the reducing end of the donor reacts with a free hydroxy or amine group of the acceptor. Thus, O-glycosylation or N-glycosylation methods are preferably employed in the coupling reaction of the method according to the invention. More preferably, O-glycosylation methods are employed in the coupling reaction of the method according to the invention. These glycosylation methods are known from the state of the art. Generally, they require a leaving group at the reducing end of the donor, which is activated in the presence of a catalyst.

The glycosylation reactions take place upon treatment of a donor and an acceptor with a **"glycosylation reagent"** or **"activator"** which acts as an activator or an activating agent. Glycosylation reagents known to the skilled person include, but are not restricted to: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST).

The term **"coupling cycle"** or **"synthesis cycle"** or **"glycosylation cycle"** as used herein relates to an iterative or repetitive sequence of coupling reactions and deprotection steps in the synthesis of oligo- or polysaccharides on a solid support. In this synthesis strategy, the carbohydrate chain is elongated in a stepwise manner and thus gradually extended through each synthesis cycle until the desired oligo- or polysaccharide is obtained.

As used herein, the term **"saccharide"** refers to but is not restricted to monosaccharide, disaccharide, trisaccharide, tetrasaccharide, pentasaccharide, hexasaccharide, heptasaccharide, octasaccharide..., oligosaccharide, polysaccharide and glycan. The saccharide comprises preferably monosaccharide units selected from:
D-Arabinose, D-Lyxose, D-Ribose, D-Xylose, L-Arabinose, L-Lyxose, L-Ribose, L-Xylose, D-Ribulose, D-Xylulose, L-Ribulose, L-Xylulose, D-Deoxyribose, L-Deoxyribose, D-Erythrose, D-Threose, L-glycero-D-manno-Heptose, D-glycero-D-manno-Heptose, D-Allose, D-Altrose, D-Glucose, D-Mannose, D-Gulose, D-Idose, D-Galactose, D-Talose, D-psicose, D-fructose, D-sorbose, D-tagatose, 6-Deoxy-L-altrose, 6-Deoxy-D-talose, D-Fucose, L-Fucose, D-Rhamnose, L-Rhamnose, D-Quinovose, Olivose, Tyvelose, Ascarylose, Abequose, Paratose, Digitoxose, Colitose, D-Glucosamine, D-Galactosamine, D-Mannosamine, D-Allosamine, L-Altrosamine, D-Gulosamine, L-Idosamine, D-Talosamine, N-Acetyl-D-glucosamine, N-Acetyl-D-galactosamine, N-Acetyl-D-mannosamine, N-Acetyl-D-allosamine, N-Acetyl-L-altrosamine, N-Acetyl-D-gulosamine, N-Acetyl-L-idosamine, N-Acetyl-D-talosamine, N-Acetyl-D-fucosamine, N-Acetyl-L-fucosamine, N-Acetyl-L-rhamnosamine, N-Acetyl-D-quinovosamine, D-Glucuronic acid, D-Galacturonic acid, D-Mannuronic acid, D-Alluronic acid, L-Altruronic acid, D-Guluronic acid, L-Guluronic acid, L-Iduronic acid, D-Taluronic acid, Neuraminic acid, N-Acetylneuraminic acid, N-Glycolylneuraminic acid, Apiose, Bacillosamine, Thevetose, Acofriose, Cymarose, Muramic acid, N-Acetylmuramic acid, N-Glycolylmuramic acid, 3-Deoxy-lyxo-heptulosaric acid, Ketodeoxyoctonic acid, and Ketodeoxynononic acid. Preferably the monosaccharide units belong to the following group of α- and β-D/L-carbohydrates comprising or consisting of: α-D-ribopyranose, α-D-arabinopyranose, α-D-xylopyranose, α-D-lyxopyranose, α-D-allopyranose, α-D-altropyranose, α-D-glucopyranose, α-D-mannpyranose, α-D-glucopyranose, α-D-idopyranose, α-D-galactopyranose, α-D-talopyranose, α-D-psicopyranose, α-D-fructopyranose, α-D-sorbopyranose, α-D-tagatopyranose, α-D-ribofuranose, α-D-arabinofuranose, α-D-xylofuranose, α-D-lyxofuranose, α-D-Allofuranose, α-D-Altrofuranose, α-D-Glucofuranose, α-D-Mannofuranose, α-D-gulofuranose, α-D-idofuranose, α-D-galactofuranose, α-D-talofuranose, α-D-psicofuranose, α-D-fructofuranose, α-D-sorbofuranose, α-D-tagatofuranose, α-D-xylulofuranose, α-D-ribulofuranose, α-D-threofuranose, α-D-rhamnopyranose, α-D-erythrofuranose, α-D-glucosamine, α-D-glucopyranuronic acid, β-D-ribopyranose, β-D-arabinopyranose, β-D-xylopyranose, β-D-lyxopyranose, β-D-allopyranose, β-D-altropyranose, β-D-glucopyranose, β-D-mannpyranose, β-D-glucopyranose, β-D-idopyranose, β-D-galactopyranose, β-D-talopyranose, β-D-psicopyranose, β-D-fructopyranose, β-D-sorbopyranose, β-D-tagatopyranose, β-D-ribofuranose, β-D-arabinofuranose, β-D-xylofuranose, β-D-lyxofuranose, β-D-rhamnopyranose, β-D-allofuranose, β-D-altrofuranose, β-D-glucofuranose, β-D-mannofuranose, β-D-gulofuranose, β-D-idofuranose, β-D-galactofuranose, β-D-talofuranose, β-D-psicofuranose, β-D-fructofuranose, β-D-sorbofuranose, β-D-tagatofuranose, β-D-xylulofuranose, β-D-ribulofuranose, β-D-threofuranose, β-D-erythrofuranose, β-D-glucosamine, β-D-glucopyranuronic acid, α-L-ribopyranose, α-L-arabinopyranose, α-L-xylopyranose, α-L-lyxopyranose, α-L-allopyranose, α-L-altropyranose, α-L-glucopyranose, α-L-mannpyranose, α-L-glucopyranose, α-L-idopyranose, α-L-galactopyranose, α-L-talopyranose, α-L-psicopyranose, α-L-fructopyranose, α-L-sorbopyranose, α-L-tagatopyranose, α-L-rhamnopyranose, α-L-ribofuranose, α-L-arabinofuranose, α-L-xylofuranose, α-L-lyxofuranose, α-L-Allofuranose, α-L-Altrofuranose, α-L-Glucofuranose, α-L-Mannofuranose, α-L-gulofuranose, α-L-idofuranose, α-L-galactofuranose, α-L-talofuranose, α-L-psicofuranose, α-L-fructofuranose, α-L-sorbofuranose, α-L-tagatofuranose, α-L-xylulofuranose, α-L-ribulofuranose, α-L-threofuranose, α-L-erythrofuranose, α-L-glucosamine, α-L-glucopyranuronic acid, β-L-ribopyranose, β-L-arabinopyranose, β-L-xylopyranose, β-L-lyxopyranose, β-L-allopyranose, β-L-altropyranose, β-L-glucopyranose, β-L-mannpyranose, β-L-glucopyranose, β-L-idopyranose, β-L-galactopyranose, β-L-talopyranose, β-L-psicopyranose, β-L-fructopyranose, β-L-sorbopyranose, β-L-tagatopyranose, β-L-ribofuranose, β-L-arabinofuranose, β-L-xylofuranose, β-L-lyxofuranose, β-L-allofuranose, β-L-altrofuranose, β-L-glucofuranose, β-L-mannofuranose, β-L-gulofuranose, β-L-idofuranose, β-L-galactofuranose, β-L-talofuranose, β-L-psicofuranose, β-L-fructofuranose, β-L-sorbofuranose, β-L-tagatofuranose, β-L-xylulofuranose, β-L-ribulofuranose, β-L-threofuranose, β-L-erythrofuranose, β-L-glucosamine, β-L-glucopyranuronic acid, and β-L-rhamnopyranose.

The saccharides are further optionally modified to carry amide, carbonate, carbamate, carbonyl, thiocarbonyl, carboxy, thiocarboxy, ester, thioester, ether, epoxy, hydroxyalkyl, alkylenyl, phenylene, alkenyl, imino, imide, isourea, thiocarbamate, thiourea and/or urea moieties.

The term **"saccharide building block"** or **"building block"** as used herein refers to a saccharide acceptor or saccharide donor, i.e. to a saccharide that is capable of forming a glycosidic bond when being exposed to a glycosylating agent. The saccharide building block can be fully protected (i.e. each hydroxy or amino group is blocked by a protecting group), partially protected (i.e. at least one hydroxy or amino groups is blocked by a protecting group) or unprotected (i.e. none of hydroxy or amino groups is blocked by a protecting group). For forming an amide bond with an anchoring group present on the solid support the saccharide building block may also be modified at the reducing end with a suitable functional group such as a carboxylic acid (e.g. hydroxyacetate), azide, alkyne, thiol or an amine (e.g. aminopropyl or aminopentyl). Any known saccharide building block can be employed in the inventive methods described herein, including saccharides, glycopeptides or glycopeptoids as described in Beilstein J. Org. Chem. 2014, 10, 2453-2460. The building blocks for the glycosylation reaction may be provided as glycosyl donor or glycosyl acceptor molecules. Glycosyl donor building blocks relate preferably to carbohydrate molecules with a suitable leaving group at the anomeric position, which may be used as glycosyl donors for the glycosylation reaction. Glycosyl acceptor building blocks relate preferably to carbohydrate molecules with an unprotected nucleophilic hydroxyl group or a protected carbohydrate molecule which allows orthogonal deprotection of one protecting group, which may be used as glycosyl acceptors for the glycosylation reaction.

**"Alkyl"** refers to saturated hydrocarbon groups of from 1 to 18 carbon atoms, either straight chained or branched, more preferably from 1 to 8 carbon atoms, and most preferably 1 to 6 carbon atoms. An alkyl with a specified number of carbon atoms is denoted as C₁-C₈ alkyl and refers to a linear C₁-C₈ alkyl of -CH₃, -C₂H₅, -C₃H₇, -C₄H₉, -C₅H₁₁, -C₆H₁₃, -C₇H₁₅, -C₈H₁₇, -CH₂-Ph, -CH₂-CH₂-Ph or a branched C₁-C₈ alkyl or preferably branched C₃-C₈ alkyl of -CH(CH₃)₂, -CH₂-CH(CH₃)₂, -CH(CH₃)-C₂H₅, -C(CH₃)₃, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH(CH₃)₂, -C₃H₆-CH(CH₃)-C₂H₅, -C₃H₆-CH(CH₃)-C₂H₅, -C₂H₄-CH(CH₃)-C₃H₇, -CH₂-CH(CH₃)-C₄H₉, -CH(CH₃)-C₅H₁₁, -CH(C₂H₅)-C₄H₉, -C₂H₄-CH(CH₃)-C₃H₇, -CH₂-CH(C₂H₅)-C₃H₇, -CH₂-CH(CH₃)-C₄H₉, -CH₂-CH(CH₃)-CH₂-CH(CH₃)₂, -CH(C₂H₅)-CH₂-CH(CH₃)₂, -CH(CH₃)-C₂H₄-CH(CH₃)₂, -CH(CH₃)-CH₂-CH(CH₃)-C₂H₅, -CH₂-CH(CH₃)-CH(CH₃)-C₂H₅, -CH(CH₃)-CH₂-CH(CH₃)-C₂H₅, -CH(CH₃)-CH(C₂H₅)-C₂H₅, -CH(C₂H₅)-CH(CH₃)-C₂H₅, -CH(CH₃)-CH(CH₃)-C₃H₇, -C₂H₄-CH(CH₃)-CH(CH₃)₂, -CH₂-CH(C₂H₅)-CH(CH₃)₂, -CH₂-CH(CH₃)-CH₂-CH(CH₃)₂, -CH₂-CH(CH₃)-CH(CH₃)-C₂H₅, -C₂H₄-C(CH₃)₂-C₂H₅, -CH₂-C(CH₃)(C₂H₅)₂, -CH₂-C(CH₃)₂-C₃H₇, -CH₂-C(CH₃)₂-C₃H₇, -C(CH₃)(C₂H₅)-C₃H₇, -C(CH₃)₂-C₄H₉, -CH₂-C(CH₃)₂-CH(CH₃)₂, -C(CH₃)(C₂H₅)-CH(CH₃)₂, -C(CH₃)₂-CH₂-CH(CH₃)₂, -C(CH₃)₂-C(CH₃)₃, -C(CH₃)₂-CH(CH₃)-C₂H₅, -C₃H₆-C(CH₃)₃, -C₂H₄-C(CH₃)₂-C₂H₅, -CH₂-CH(CH₃)-C(CH₃)₃, -CH(C₂H₅)-C(CH₃)₃, -CH(CH₃)-CH₂-C(CH₃)₃, -CH(CH₃)-C(CH₃)₂-C₂H₅, -C₅H₁₀-CH(CH₃)₂, -C₄H₈-C(CH₃)₃, -C₄H₈-CH(CH₃)-C₂H₅, -C₄H₈-CH(CH₃)-C₂H₅, -C₃H₆-C(CH₃)₂-C₂H₅, -C₃H₆-CH(C₂H₅)-C₂H₅, -C₃H₆-CH(CH₃)-C₃H₇, -C₂H₄-C(CH₃)₂-C₃H_{7,} -C₂H₄-CH(C₂H₅)-C₃H₇, -C₂H₄-CH(CH₃)-C₄H₉, -CH₂-C(CH₃)₂-C₄H₉, -CH₂-CH(C₂H₅)-C₄H₉, -CH₂-CH(CH₃)-C₅H₁₁, -C(CH₃)₂-C₅H₁₁, -CH(CH₃)-C₆H₁₃, -CH(C₃H₇)-C₄H₉, -CH(C₂H₅)-C₅H₁₁, -CH₂-C(CH₃)(C₂H₅)-C₃H₇, -C₂H₄-CH(CH₃)-CH₂-CH(CH₃)₂, -CH₂-C(CH₃)₂-CH₂-CH(CH₃)₂, -CH₂-CH(C₂H₅)-CH₂-CH(CH₃)₂, -CH₂-CH(CH₃)-C₂H₄-CH(CH₃)₂, -CH₂-CH(CH₃)-CH₂-C(CH₃)₃, -CH₂-CH(CH₃)-CH₂-CH(CH₃)-C₂H₅, -C(CH₃)(C₂H₅)-CH₂-CH(CH₃)₂, -CH(C₃H₇)-CH₂-CH(CH₃)₂, -CH(C₂H₅)-C₂H₄-CH(CH₃)₂, -CH(C₂H₅)-CH₂-C(CH₃)₃, -CH(C₂H₅)-CH₂-CH(CH₃)-C₂H₅, -CH₂-CH(CH₃)-C₂H₄-CH(CH₃)₂, -C(CH₃)₂-C₂H₄-CH(CH₃)₂, -CH(C₂H₅)-C₂H₄-CH(CH₃)₂, -CH(CH₃)-C₃H₆-CH(CH₃)₂, -CH(CH₃)-C₂H₄-C(CH₃)₃, -CH(CH₃)-C₂H₄-CH(CH₃)-C₂H₅, -CH₂-CH(CH₃)-CH₂-CH(CH₃)-C₂H₅, -C(CH₃)₂-CH₂-CH(CH₃)-C₂H₅, -CH(CH₃)-C₂H₄-CH(CH₃)-C₂H₅, -CH(CH₃)-CH₂-C(CH₃)₂-C₂H₅, -CH(CH₃)-CH₂-CH(CH₃)-C₃H₇, -C₂H₄-CH(CH₃)-CH(CH₃)-C₂H₅, -CH₂-C(CH₃)₂-CH(CH₃)-C₂H₅, -CH₂-CH(C₂H₅)-CH(CH₃)-C₂H₅, -CH₂-CH(CH₃)-CH₂-CH(CH₃)-C₂H₅, -CH₂-CH(CH₃)-C(CH₃)₂-C₂H₅, -CH₂-CH(CH₃)-CH(C₂H₅)₂, -C₃H₆-CH(CH₃)-CH(CH₃)₂, -C₂H₄-C(CH₃)₂-CH(CH₃)₂, -C₂H₄-CH(C₂H₅)-CH(CH₃)₂, -C₂H₄-CH(CH₃)-C(CH₃)₃, -C₂H₄-CH(CH₃)-CH(CH₃)-C₂H₅, -C₃H₆-C(CH₃)₂-C₂H₅, -C₂H₄-C(CH₃)₂-C₃H₇, -CH₂-C(CH₃)(C₂H₅)₂, -C₂H₄-C(C₂H₅)₃, -C₂H₄-C(CH₃)₂-C₃H₇, -CH₂-C(CH₃)₂-C₄H₉, -C(C₂H₅)₂-C₃H₇, -C(CH₃)(C₃H₇)-C₃H₇, -C(CH₃)(C₂H₅)-C₄H₉, -C(CH₃)(-C₂H₅)-C₄H₉, -C(CH₃)₂-C₅H₁₁, -C₂H₄-C(CH₃)₂-CH(CH₃)₂, -CH₂-C(CH₃)₂-C(CH₃)₃, -C(C₂H₅)₂-CH(CH₃)₂, -C(CH₃)(C₃H₇)-CH(CH₃)₂, -C(CH₃)(C₂H₅)-C(CH₃)₃, -CH₂-C(CH₃)₂-CH₂-CH(CH₃)₂, -C(CH₃)₂-C₂H₄-CH(CH₃)₂, -C(CH₃)₂-CH₂-C(CH₃)₃, -CH₂-C(CH₃)₂-C(CH₃)₃, -C₄H₈-C(CH₃)₃, -C₃H₆-C(CH₃)₂-C₂H₅, -C₂H₄-C(CH₃)₂-C₃H₇, -C₂H₄-CH(CH₃)-C(CH₃)₃, -CH₂-C(CH₃)₂-C(CH₃)₃.

**"Aryl"** refers to an unsaturated aromatic carbocyclic group of from 6 to 12 carbon atoms inclusively having a single ring (*e.g*., phenyl) or multiple condensed rings (*e.g*., naphthyl or anthryl). Exemplary aryls include phenyl, pyridyl, naphthyl and the like.

The term **"glycosyl donor"** as used herein relates to a saccharide having a suitable leaving group at the anomeric position, which may be used as reactant for the coupling reaction. A donor or glycosyl donor refers to a saccharide that forms a glycal, or a saccharide comprising an epoxide or orthoester group or a saccharide that contains a leaving group at the reducing end. Suitable leaving groups include halogen, -O-C(=NH)-CCl₃, -O-C(=NPh)-CF₃, -OAc, -SR⁵, -SO-Ph, -SO₂-Ph, -O-(CH₂)₃-CH=CH₂, -O-P(OR⁵)₂, -O-PO(OR⁵)₂, -O-CO-OR⁵, 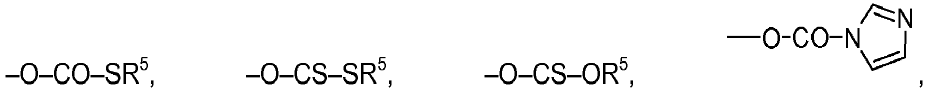 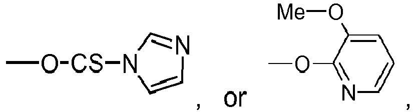 wherein R⁵ represents an alkyl or aryl group.

The term **"glycosyl acceptor"** as used herein relates to a carbohydrate molecule or a saccharide that contains at least one free hydroxy or amine function and is capable of forming a glycosidic bond with a glycosyl donor under suitable reaction conditions.

The term **"protecting group"** or "protective group" as used herein refers to commonly used groups in organic synthesis, preferably used for protection of amines, hydroxyl groups, thiols, imines, carbonyls, carboxyls or other common functional groups, and particularly preferred for amines and hydroxyl groups. The protecting groups are characterized in that they are stable under reaction conditions applied during the synthesis, i.e. they are not cleaved off or undergo undesired side reactions and prevent any reaction of the protected functional group they are bonded to. Additionally, the protecting groups are selected to not hinder or to not affect the performed reaction steps in terms of yield or stereoselectivity.

Preferred protecting groups for hydroxyl groups are acetyl, phenyl, benzyl, isopropylidene, benzylidene, benzoyl, *p*-methoxybenzyl, *p*-methoxybenzylidene, *p*-methoxyphenyl, *p*-bromobenzylidene, *p*-nitrophenyl, allyl, allyloxycarbonyl, monochloroacetyl, isopropyl, *p*-bromobenzyl, dimethoxytrityl, trityl, 2-naphthylmethyl, pivaloyl, triisopropylsilyl, *tert*-butyldimethylsilyl, *tert-*butyldiphenylsilyl, *tert*-butylmethoxyphenylsilyl, triethylsilyl, trimethylsilyl, 2-trimethylsilylethoxymethyl, 9-fluorenylmethoxycarbonyl, tert-butyloxycarbonyl, benzyloxymethyl, methyloxymethyl, *tert*-butyloxymethyl, methoxyethyloxymethyl, and levulinoyl.

The protecting groups can be differentiated in "permanent protecting groups" and "temporary protecting groups". Permanent protecting groups are protecting groups that are stable during the entire synthesis and that can be efficiently removed at the late stage of the synthesis. In this case, permanent protecting groups are masking the hydroxyl groups and amino groups, if present, during the entire synthesis. Preferably permanent protecting groups are benzyl, benzoyl, acetyl, allyloxycarbonyl (alloc) and benzyloxycarbonyl group (Cbz).

The term "temporary protecting group" as used herein relates to a protecting group of a protected saccharide which may be selectively removed from the protected saccharide, wherein the remaining further protecting groups or the "permanent protecting groups" are not removed under the same conditions. Preferably, removal of a temporary protecting group allows conversion of the protected saccharide to a saccharide having at least one unprotected hydroxyl group or amine function. Thus, a glycosyl acceptor for use in a subsequent coupling reaction may be provided by removing a temporary protecting group of the protected saccharide.

The temporary protecting groups are generally orthogonal protecting groups that can be selectively removed at different levels of the synthesis to free hydroxyl groups for subsequent introduction of different substituents, including monosaccharides, other protecting groups or other residues present on the molecule. Temporary protecting groups are preferably selected from, but are not restricted to: allyl, *p*-methoxybenzyl, 2-naphthylmethyl, tri-isopropylsilyl, *tert-*butyldimethylsilyl, *tert*-butylmethoxyphenylsilyl, triethylsilyl, trimethylsilyl, 2-trimethylsilylethoxymethyl, 9-fluorenylmethoxycarbonyl and levulinoyl.

The ingenious choice of protecting groups allows expedient access to a library of saccharides. It is apparent for a skilled person to choose the protecting groups in such a manner that they can be removed from the saccharide without cleaving the saccharide from the solid support.

The term **"deprotection"** as used herein relates to the removal of one or more protecting groups of a molecule functionalized with one or more protecting groups. The deprotection step of a coupling cycle as described herein preferably relates to the removal of a specific protecting group of a protected carbohydrate molecule. As a carbohydrate molecule typically exhibits several hydroxyl groups it is preferred that only one of said hydroxyl groups participates as nucleophile in the glycosylation reaction. Therefore, orthogonal protecting group strategy is typically applied to provide carbohydrate molecules with only one free hydroxyl group, wherein the protecting groups of the remaining hydroxyl groups are not removed. Orthogonal protecting group strategies are often the key step of a successful regioselective functionalization of a carbohydrate molecule. Suitable protecting groups and orthogonal protecting group strategies are therefore well known to the skilled person.

The term **"capping"** as used herein relates to blocking or capping of free hydroxyl groups by a highly reactive blocking group to avoid elongation of failure sequences of the oligo- or polysaccharide during the synthesis cycles. Deletion sequences missing just one sugar unit (n-1) are the most difficult to separate from the desired product and arise from incomplete coupling steps during any coupling cycle of the sequence. The oligosaccharide chains that fail to couple during one cycle may be successfully glycosylated during the following elongation steps. Therefore, a severe purification problem may exist at the end of the synthesis. To avoid the elongation of failure sequences, a capping step (i.e., a blocking step) may be included into the coupling cycle. After each completed coupling, a highly reactive blocking group can be used to cap any free hydroxyl group. For example, benzyl trichloroacetimidate can be employed as a capping reagent (activated with TMSOTf) to yield benzyl ethers in positions that were not glycosylated and render them unreactive throughout the synthesis. Using this straightforward capping step, the purification of the finished oligosaccharide products may be greatly simplified, since the presence of (n-1)-deletion sequences will be minimized.

The term **"solid support"** as used herein refers to an insoluble, functionalized, polymeric material to which saccharides or other reagents may be attached or immobilized, directly or via a linker bearing an anchoring group, allowing saccharides to be readily separated (by washing, filtration, centrifugation, etc.) from excess reagents, soluble reaction by-products, or solvents. The solid support has preferably the form of a resin bead or CPG (Controlled Pore Glass) bead.

The inventors of the present invention considered the following key issues for the development and improvement of an automated oligo- and polysaccharide synthesizer: design of an overall synthesis strategy with either the reducing or the non-reducing end of the growing carbohydrate chain attached to the support, selection of a polymer and linker that are inert to all reaction conditions during the synthesis but cleaved efficiently when desired, a protecting group strategy consistent with the complexity of the target oligo- or polysaccharide, stereospecific and high yielding glycosylation reactions, and a device capable of performing repetitive chemical manipulations at variable temperatures.

Surprisingly, it has been found that a device for automated synthesis of oligo- and polysaccharides on a solid support comprising a reaction vessel, a reagent storing component, a reagent delivery system, a cooling device for cooling the reaction vessel, and a pre-cooling device for pre-cooling the reagents to be supplied, resolves the above objective.

Thus, the present invention relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied.

According to the present invention, the device for automated synthesis of oligo- or polysaccharides on a solid support comprises an improved cooling system to prevent fluctuations in temperature of a reaction mixture which may otherwise result in loss of sensitive intermediates and reagents, in particular in the coupling steps of the synthesis cycles. Thus, with the cooling system of the device of the present invention unfavorable temperature spikes may be effectively prevented during the transfer of building blocks and activators to the reaction vessel. Furthermore, the cooling system of the present invention allows for advantageous temperature adjustment of the reaction vessel and further of the reaction mixture to prevent decomposition of the reactants, sensitive intermediates and desired products at different thermal stages of the synthesis cycles. According to the present invention the improved cooling system consists of a cooling device and a pre-cooling device. The device of the present invention for automated synthesis of oligo- or polysaccharides on a solid support therefore comprises a cooling device for cooling the reaction vessel and a pre-cooling device for pre-cooling the reagents to be supplied for control of the temperature of one or more reagents and/or the reaction mixture during one or more synthesis steps of the synthesis cycles to prevent decomposition of the sensitive intermediates and reagents. Of course, the cooling device for cooling the reaction vessel and the pre-cooling device for pre-cooling the reagents can be combined to a single cooling device for cooling both, the reaction vessel and the reagents to be supplied. Most preferably the one cooling device for both is a Peltier cooling element.

As used herein, the term >> **pre-cooling the reagents** << refers to the cooling of the reagent storing component and/or the cooling of the reagent delivery system to the reaction vessel and preferably to the cooling of the reagent delivery system and more preferably to the cooling of the reagent storing component and the cooling of the reagent delivery system.

According to a preferred embodiment of the present invention, the device for automated synthesis of oligo- and polysaccharides on a solid support may further comprise a microwave generator component to enable rapid microwave-assisted blocking/deblocking synthesis steps, such as deprotection or capping steps that allow for efficient and rapid syntheses in different batch sizes. Use of microwave radiation during part of the synthesis cycles may significantly shorten the time to achieve complete conversion, which is important for scale-up.

According to the present invention the device for automated synthesis of oligo- and polysaccharides on a solid support comprises a **reaction vessel.** The reaction vessel may be made of any material known in the art which is chemically and physically compatible with the reagents used and reaction conditions applied in the automated synthesis of oligo- and polysaccharides on a solid support. Examples of reaction vessel materials include, but are not limited to, glass, quartz, PTFE (Teflon), polypropylene. In preferred embodiments the reaction vessel is interchangeable. In preferred embodiments the reaction vessel is made of fluoropolymers such as PFA for improved chemical resistance and ease of swapping reactions vessels of different sizes.

The reaction vessel of the present invention is adapted for holding a reaction mixture. The reaction vessel therefore comprises an inner cavity or effective loading space for performing one or more synthesis steps of the synthesis cycles to obtain the desired oligo- or polysaccharide. The reaction vessel is adapted to enable performance of one or more synthesis steps or even all of the synthesis steps of the synthesis cycles in an isolated, anhydrous and inert atmosphere. These steps may include, but are not limited to, coupling steps, washing steps, deprotection steps, capping steps and decoupling from the solid phase resin. The solid support or solid phase resin may be initially loaded into the reaction vessel. With other words the solid support or solid phase resin may be loaded into the inner cavity or effective loading space of the reaction vessel. The loading of the reaction vessel with the solid phase resin may be performed as part of a pre-automation process but may also be part of the automation process. Thus, the loading with the solid support may be also performed in an automated manner. The solid support may be provided in form of insoluble resin bead(s) and is preferably a functionalized resin suitable for oligo- and polysaccharide synthesis on a solid support, such as a resin equipped with an appropriate linker or in particular a polystyrene-based resin functionalized with an appropriate linker. Several suitable functionalized solid phase resins for oligo- or polysaccharide synthesis on a solid support have been described in the prior art. Examples of functionalized solid phase resins suitable for use with the device of the present invention are described further below.

Thus, it is preferred that the reaction vessel is adapted for receiving a solid support. Therefore, the reaction vessel preferably comprises an inner chamber or effective loading space for performing one or more synthesis steps of the synthesis of oligo- and polysaccharides on a solid support, preferably in an isolated, anhydrous and inert atmosphere. It is preferred that the inner cavity or effective loading space holds between 1 mL and 100 mL solvent, more preferably 5 - 20 mL solvent. It is preferred that the inner cavity or the effective loading space is sized to accommodate the solid support, reagents and solvent.

Therefore the present invention further relates to a device for automated synthesis of oligo- and polysaccharides on a solid support, the device comprising:
(a) a reaction vessel adapted for receiving the solid support,
(b) a reagent storing component,
(c) a reagent delivery system,
(d) a cooling device for cooling the reaction vessel,
(e) and a pre-cooling device for pre-cooling the reagents to be supplied.

For performing the coupling reactions appropriate building blocks and activators are supplied or added to the reaction vessel. Preferably, the building blocks and activators are supplied or added to the reaction vessel already containing the solid support or solid phase resin. The coupling reactions preferably take place inside the reaction vessel followed by appropriate deprotection, capping and/or washing steps. All of these synthesis steps and treatments in the synthesis cycles are preferably performed inside of the reaction vessel of the device of the present invention. The synthesis cycles are repeated as often as necessary to achieve the desired length of the desired oligo- or polysaccharide. The synthesis cycle is preferably repeated at least three times, more preferably at least four times or more preferably more than four times for obtaining an oligo- or polysaccharide.

For supplying liquids, solutions and/or gases the reaction vessel of the device of the present invention may further comprise one or more inlets, for example, for supplying washing solvents or washing solutions, building blocks or building block solutions, activators or activator solutions, capping solutions, deprotection solutions and inert gas, and may further comprise one or more outlets, for example, for discharging the liquids or solutions after each chemical reaction and/or also ventilation exits for inert gas or other gases from the reaction mixture.

In preferred embodiments of the present invention the one or more inlets may also function as one or more outlets, that means one or more inlets for supplying reagents, solvents, reaction mixtures, inert gas and the like can also be used to remove a solution, inert gas, reaction components and the like from the device for automated synthesis. In such embodiments one or more technical means such as one or more valves, valve assemblies, one or more vents, one or more manifolds and/or similar technical means may be located and mounted upstream to the one or more inlets of the reaction vessel. For example, by implementing a valve assembly or one or more valves in the device of the present invention the delivery of liquids, solutions and/or gases and also the discharging of liquids, solutions and/or gases after or during each chemical reaction or treatment may be realized through one inlet of the reaction vessel only or through two, three or more inlets of the reaction vessel. By implementation of technical means such as valves, valve assemblies, vents, manifolds or similar technical means, the total number of in- and outlets of the reaction vessel may be reduced.

Furthermore, technical means such as valves, valve assemblies, vents and manifolds and similar technical means may be electronically coupled to a **computing device** comprising at least one processor and therefore may be under control of a computing device comprising at least one processor. Thus, the delivery of the liquids, solutions and/or gases and also the discharging of liquids and solutions may be under control of the computing device comprising the at least one processor. The computing device comprising the at least one processor may be configured to control said technical means in order to deliver liquids, solutions and/or gases to the reaction vessel and further to discharge liquids and solutions from the reaction vessel in a controlled and automated manner to allow automation of the synthesis of oligo- or polysaccharide on a solid support with the device of the present invention.

The reaction vessel may further comprise one or more openings or apertures for insertion of various other components or technical means. For example, the reaction vessel may further comprise an opening or aperture for insertion of a temperature probe or a temperature sensor for monitoring and measuring the temperature inside of the reaction vessel. Monitoring and measuring the temperature inside of the reaction vessel thereby also means monitoring and measuring the temperature of the reaction mixture during one or more or even all synthesis steps of the synthesis cycles. Thus, it is preferred that the reaction vessel further comprises at least one opening or aperture for insertion of a temperature sensor for monitoring and measuring the temperature inside of the reaction vessel.

The one or more inlets of the reaction vessel may be located at various positions of the reaction vessel body. In preferred embodiments of the present invention the reaction vessel may comprise one or more inlets at the top of the reaction vessel, for example, for delivery of washing solvents or washing solutions, building blocks or building block solutions, and activators or activator solutions and may further comprise one or more outlets at the top of the reaction vessel, for example, for exits for inert gas or other gases and may further comprise one or more inlets at the bottom of the reaction vessel, for example, for delivery of capping solutions, deprotection solutions and inert gas and may further comprise one or more outlets for discharging the liquids or solutions after each chemical reaction or after each washing step. As mentioned above, by implementing further technical means such as one or more valves, valve assemblies or similar technical means, these one or more inlets may also function as outlets for the liquids, solution and/or gases.

Thus, in preferred embodiments of the present invention the reaction vessel comprises one or more inlets at the top of the reaction vessel and one or more inlets at the bottom of the reaction vessel to enable two delivery methods for solvents and reagents, one from the top and another from the bottom of the reaction vessel. The usage of two different ports allows for the separation of incompatible reagents such as acids and bases, which may produce salts when combined. Over time these salts may build up and block tubing that may corrode components of the device of the present invention, which may lead to costly repairs. It is therefore preferred that the solutions to remove the temporary protecting groups (for example protecting groups such as Fmoc, Lev, ClAc, and NAP) and acetyl capping solutions are delivered via the one or more bottom inlets or bottom port of the reaction vessel. These reactions are desired to be performed rapidly and solutions are provided in excess, so these solutions will be pushed into the reaction vessel for example by pressurized inert gas such as argon. The inert gas inlet may serve for a dual purpose to not only deliver the reagents, but to efficiently mix the reaction. In addition, the one or more bottom inlets may serve to remove all the reagents and solutions from the reaction vessel. Oppositely, the activators or activator solutions and the building blocks or building block solutions are preferably delivered through the one or more top inlets or top port of the reaction vessel. These reagents are more valuable and are required to be delivered in a controlled stoichiometry, so these are delivered preferably dropwise, for example, via a syringe pump system.

In preferred embodiments of the present invention the reaction vessel may comprise only one inlet at the bottom of the reaction vessel, for example, for delivery of capping solutions, deprotection solutions and inert gas and for discharging the liquids or solutions after each chemical reaction or after each washing step and one or more inlets at the top of the reaction vessel, for example, for delivery of washing solvents or washing solutions, building blocks or building block solutions, and activators or activator solutions. The reaction vessel may further comprise openings or apertures at the top of the reaction vessel for insertion of, for example, a temperature probe or a temperature sensor. The reaction may further comprise an exhaust gases exit at the top of the reaction vessel.

In preferred embodiments of the present invention the reaction vessel may comprise at least three inlets/outlets, preferably four inlets/outlets (601, 602, 645, 655) at the top of the reaction vessel, for example, one inlet for delivery of washing solvents or washing solutions, one inlet for delivery of building blocks or building block solutions and one inlet for delivery of activators or activator solutions and preferably a further outlet for the exhaust of gases and/or mixing gas (655). The separate and distinct delivery of the building blocks and activators to the microwave transparent reaction vessel is advantageous for preventing a contacting of the building blocks and activators before supplying the building blocks and activators to the reaction mixture inside of the reaction vessel. It is particular preferred that the building blocks or building block solutions and the activators or activator solutions are not mixed or merged with each other before delivery to the reaction vessel. It is further advantageous that the washing solvents or washing solutions are separately supplied to the reaction vessel to allow rapid delivery of washing solvents or washing solution in a larger amount and to ensure well distribution of the washing solvents or washing solutions inside of the reaction vessel. For example, a channel for supplying the washing solvents or washing solution may be used to supply the washing solvents or washing solution to the reaction vessel. Such a channel may be adapted for spraying the washing solvents or washing solutions by splitting the liquid among a series of holes at the tip of the channel. The channel may also comprise a conical end to assure complete dropping of the washing solvents or washing solutions. Such a channel for delivery of washing solvents or washing solution allows to effectively wash-off other reagent solutions, reagents or adhered solid support from the inner walls of the reaction vessel. In contrary, the building blocks and activators are preferably supplied directly into the reaction mixture in a dropwise manner.

In embodiments of the present invention wherein the reaction vessel comprises one or more inlets at the bottom of the reaction vessel or only one inlet at the bottom of the reaction vessel it is preferred that the effective loading space or inner chamber of the reaction vessel is fenced by the bottom inlet. The bottom compartment may prevent canalization of fluid through a frit. A frit allows for dispersion of inert gas bubbling for mixing purposes. The frit may also ensure that the solid support remains inside of the reaction vessel. Thus, in preferred embodiments of the present invention the reaction vessel may further comprise a frit. It is preferred that the frit is located in the bottom compartment of the reaction vessel. Thus, to prevent solid support from being drawn from the bottom inlet, the end in the reaction vessel may be fitted with a frit or a filter.

Preferably the reaction vessel is made of a material such as glass, quartz, PTFE (Teflon), polypropylene or fluoropolymers. A reaction vessel made of glass may be provided in form of a triple-jacketed glass reaction vessel. The reaction vessel may be provided as a glass reaction vessel surrounded by a second cavity that allows for circulation of coolant fluid to control the temperature of the reaction mixture. Thus, the reaction vessel may be provided with a cooling jacket for cooling the microwave transparent reaction vessel.

In preferred embodiments of the present invention the reaction vessel may be provided as interchangeable reaction vessel. In such embodiments it is preferred that the reaction vessel may be removed from a cooling means such as a cooling jacket without interrupting the flow of a circulating coolant fluid.

In such embodiments the reaction vessel may be removed for example for loading with solid support or for discharging the solid support. Furthermore such an interchangeable reaction vessel allows use of various reaction vessels of different sizes for different batch sizes.

Therefore the present invention preferably relates to a device for automated synthesis of oligo- and polysaccharides on a solid support, the device comprising:
(a) an interchangeable reaction vessel,
(b) a reagent storing component,
(c) a reagent delivery system,
(d) a cooling device for cooling the reaction vessel,
(e) and a pre-cooling device for pre-cooling the reagents to be supplied.

Therefore the present invention preferably relates to a device for automated synthesis of oligo- and polysaccharides on a solid support, the device comprising:
(a) an interchangeable reaction vessel adapted for receiving the solid support,
(b) a reagent storing component,
(c) a reagent delivery system,
(d) a cooling device for cooling the reaction vessel,
(e) and a pre-cooling device for pre-cooling the reagents to be supplied.

The inventors have surprisingly found that a reaction vessel made of fluoropolymers such as polytetrafluoroethylene (PTFE), perfluoroalkoxy alkanes (PFA), fluorinated ethylene propylene (FEP), ethylene tetrafluoroethylene (ETFE) or a reaction vessel comprising a coating of polytetrafluoroethylene (PTFE), perfluoroalkoxy alkanes (PFA), fluorinated ethylene propylene (FEP), ethylene tetrafluoroethylene (ETFE) is particularly suitable for the device for the synthesis of oligo- and polysaccharides on a solid support of the present invention.

The inventors of the present invention have found that the change in the manufacturing of the reaction vessels from silicate glass to fluoropolymer allows one the one hand integration of a microwave device into the synthesis cycle, and on the other hand improves chemical resistance, as well as eases of swapping reaction vessels of different sizes. Thus, it is advantageous to switch from a glass reaction vessel to one made of fluoropolymers such as perfluoroalkoxy alkanes (PFA). Microwave transparency, improved flow, minimal resin adherence, thermal resistance, and chemical resistance are among the many properties that PFA excels over glass. Typical silicate glass contains hydroxyl groups, which creates a hydrophilic surface. During the synthesis cycles, the resin often adheres to glass walls above the reaction solution causing deletion sequences and mixtures in the final product. To prevent adhesion problems, regular silanization of the glass reactor is necessary. On the other hand, PFA reactors have better hydrophobic properties and are non-adherent to the resin. This allows for reaction vessels made from PFA materials to tolerate a wider range of chemical reactions that are incompatible with silane coating on glass. An additional advantage is the physical durability of the PFA reactor compared to glass. Typically, AGA synthesizers are utilized for high throughput synthesis and are in constant use, so durable components are preferred.

In preferred embodiments of the present invention the reaction vessel is made of perfluoroalkoxy alkanes (PFA). In further preferred embodiments of the present invention the reaction vessel comprises a coating of perfluoroalkoxy alkanes (PFA). Perfluoroalkoxy alkanes are copolymers of tetrafluoroethylene (C₂F₄) and perfluoroethers (C₂F₃OR^{f}, where R^{f} is a perfluorinated group such as trifluoromethyl (CF₃).

Therefore the present invention further relates to a device for automated synthesis of oligo- and polysaccharides on a solid support, the device comprising:
(a) a reaction vessel made of perfluoroalkoxy alkanes (PFA),
(b) a reagent storing component,
(c) a reagent delivery system,
(d) a cooling device for cooling the reaction vessel,
(e) and a pre-cooling device for pre-cooling the reagents to be supplied.

Therefore the present invention further relates to a device for automated synthesis of oligo- and polysaccharides on a solid support, the device comprising:
(a) an interchangeable reaction vessel made of perfluoroalkoxy alkanes (PFA) adapted for receiving the solid support,
(b) a reagent storing component,
(c) a reagent delivery system,
(d) a cooling device for cooling the reaction vessel,
(e) and a pre-cooling device for pre-cooling the reagents to be supplied.

In preferred embodiments of the present invention the reaction vessel may further comprise a cap adapted for closing the reactor vessel for protecting the inner chamber or effective loading space from the external atmosphere. Thus, a cap may be provided for closing the reaction vessel in order to provide an isolated, anhydrous and inert atmosphere inside of the reaction vessel.

According to the present invention the device for automated synthesis of oligo- or polysaccharides on a solid support comprises a **reagent storing component.** The device for automated synthesis of oligo- or polysaccharides on a solid support of the present invention may comprise one or more reagent storing components. The device for automated synthesis of oligo- or polysaccharides on a solid support of the present invention may comprise at least one reagent storing component. The reagent storing component or the one or more reagent storing components are preferably adapted for storing the building blocks and/or building block solutions, activators and/or activator solutions, washing solvents and/or washing solutions, deprotection solutions and/or capping solutions. The reagent storing component or the one or more reagent storing components may comprise one or more reagent containers or may comprise a plurality of reagent containers. The reagent storing component or the one or more reagent storing components may comprise one or more reagent containers for storing building blocks, one or more reagent containers for storing building block solutions, one or more reagent containers for storing activators, one or more reagent containers for storing activator solutions, one or more reagent containers for storing washing solvents and/or washing solutions, one or more reagent containers for storing deprotection solutions and one or more reagent containers for storing capping solutions. The reagent storing component or the one or more reagent storing components are preferably adapted for storing reagents and solvents in an anhydrous and inert atmosphere. In preferred embodiments of the present invention the reagents and solvents are stored under argon pressure. In preferred embodiments the reagent storing component or the one or more reagent storing components are connected to an inert gas delivery system or gas distribution system. The gas delivery system or gas distribution system may provide inert gas, such as argon, to the reagent storing component or the one or more reagent storing components. The inert gas delivery system or gas distribution system may provide inert gas, such as argon, to each of the containers of the reagent storing component or the one or more reagent storing components. Thus, in preferred embodiments of the present invention each of the containers of the reagent storing component or the one or more reagent storing components may be connected to an inert gas delivery system or gas distribution system.

The containers may be made of any suitable chemical resistant material known in the art which is suitable for storing chemical reagents and/or solutions and furthermore suitable for storing the building blocks and/or building block solutions, activators and/or activator solutions, washing solvents or washing solutions, deprotection solutions and also capping solutions. The containers may preferably be adapted for storing the reagents in an anhydrous and inert atmosphere, for example, under argon pressure. For example, synthesis-ready building blocks may be pre-weighed and either dissolved in the appropriate anhydrous solvent or kept as solids and placed in sealed vials, for example, on a 64-position carousel or a 4-position means (e.g. test tube type containers) were a solution of building block (e.g. up to 8 mL of the appropriate concentration) is coupled to the system by a multiport valves or similar technical means. Preferably a cap seals the reaction vessel which has an inlet for inert gas, such as argon, providing an anhydrous atmosphere and an outlet for the building block solution extraction. The extraction tube may reach the bottom container and may be allowed to take out the required volume of building block solutions. The building blocks are preferably those that can be synthesized in large quantities, are stable upon storage for extended periods of time, and upon activation result in very high coupling yields with excellent stereo-control. Solvents and reagents may for example be placed in various sizes of glass bottles and kept under inert gas, for example, under argon pressure.

Preferably the device for automated synthesis of oligo- and polysaccharides on a solid support comprises a reagent storing component for storing building blocks and/or building block solutions, activators and/or activator solutions, washing solutions and/or washing solvents and/or deprotection solutions. Preferably the device for automated synthesis of oligo- and polysaccharides on a solid support comprises a reagent storing component for storing one or more reagents and/or reagent solution and/or solvents. The reagents and/or reagent solutions preferably comprise building blocks, building block solutions, activator, activators solutions, washing solutions, washing solvents and deprotection solutions. Preferably the device for automated synthesis of oligo- and polysaccharides on a solid support comprises a reagent storing component for storing building blocks or building block solutions, activators or activator solutions, washing solutions or washing solvents, deprotection solutions and capping solutions.

Therefore, the present invention preferably relates to a device for automated synthesis of oligo- and polysaccharides on a solid support, the device comprising:
(a) a reaction vessel,
(b) a reagent storing component for storing building blocks and/or building block solutions, activators and/or activator solutions, washing solutions and/or washing solvents and deprotection solutions,
(c) a reagent delivery system,
(d) a cooling device for cooling the reaction vessel,
(e) and a pre-cooling device for pre-cooling the reagents to be supplied.

The device of the present invention may comprise one or more liquid, solution or inert gas **delivery systems** for driving the flow of liquids or gases to the reaction vessel.

According to the present invention the device for automated synthesis of oligo- or polysaccharides on a solid support comprises a **reagent delivery system.** The device of the present invention may comprise at least one reagent delivery system. Preferably the device of the present invention comprise a reagent delivery system adapted for delivery of building blocks or building block solutions, activators or activator solutions, washing solvents or washing solutions, deprotection solutions, capping solutions and inert gas to the reaction vessel. Preferably the device of the present invention comprises a reagent delivery system adapted for receiving building blocks or building block solutions, activators or activator solutions, washing solvents or washing solutions, deprotection solutions and capping solutions from the reagent storing component. The device of the present invention may also comprise one or more reagent delivery systems. The reagent delivery system may comprise one or more reagent delivery sub-systems or reagent delivery sub-components for delivery of one or more of the one or more reagents, solutions and/or reagent solution to the reaction vessel.

Therefore, the present invention further relates to a device for automated synthesis of oligo- and polysaccharides on a solid support, the device comprising:
(a) a reaction vessel,
(b) a reagent storing component,
(c) a reagent delivery system for delivery of buildings blocks and/or building block solutions, activators and/or activator solutions, washing solvents and/or washing solutions and deprotection solutions,
(d) a cooling device for cooling the reaction vessel,
(e) and a pre-cooling device for pre-cooling the reagents to be supplied.

Preferably, the present invention relates to a device for automated synthesis of oligo- and polysaccharides on a solid support, the device comprising:
(a) a reaction vessel,
(b) a reagent storing component,
(c) a reagent delivery system for delivery of building block solutions, activator solutions, washing solvents and/or washing solutions and deprotection solutions,
(d) a cooling device for cooling the reaction vessel,
(e) and a pre-cooling device for pre-cooling the reagents to be supplied.

Therefore, the present invention further relates to a device for automated synthesis of oligo- and polysaccharides on a solid support, the device comprising:
(a) a reaction vessel,
(b) a reagent storing component for storing building blocks and/or building block solutions, activators and/or activator solutions, washing solutions and/or washing solvents and deprotection solutions,
(c) a reagent delivery system for delivery of buildings blocks and/or building block solutions, activators and/or activator solutions, washing solvents and/or washing solutions and deprotection solutions,
(d) a cooling device for cooling the reaction vessel,
(e) and a pre-cooling device for pre-cooling the reagents to be supplied.

Preferably the reagent delivery system connects the reagent storing component with the reaction vessel. Preferably the reagent delivery system is connected to the reagent storing component and is further connected to the reaction vessel. It is preferred that the reagent storing component and the reaction vessel are not directly connected to each other, in other words it is preferred that the reagent storing component and the reaction vessel are not in direct fluid communication with each other. Preferably the reagent delivery system is interposed between the reaction vessel and the reagent storing component. Preferably the reagent delivery system is in fluid communication with the reagent storing component and is further in fluid communication with the reaction vessel. It is preferred that the reagent delivery system receives the reagents from the reagent storing component and after receiving the reagents from the reagent storing component the reagents delivery system may deliver the reagents to the reaction vessel in order supply the reagents to the reaction mixture inside the reaction vessel.

Therefore, the present invention preferably relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied, and
wherein the reagent delivery system connects the reagent storing component with the reaction vessel.

The reagent delivery system may be connected to the reaction vessel through one or more inlets of the reaction vessel for delivery of the reagents and/or reagents solution and/or solvents to the reaction vessel through the one or more inlets of the reaction vessel. The reagent delivery system may be connected to the reaction vessel through one or more reagent delivery lines. Thus, the reagent delivery system may be adapted to deliver reagents and/or reagent solutions and/or solutions and/or solvents to the reaction vessel and may be further adapted to supply the reagents and/or reagent solution and/or solutions or solvents through the one or more inlets of the reaction vessel. Preferably the reagent delivery system may be adapted to deliver one or more reagents and/or one or more reagent solutions and/or one or more solutions in different amounts, at different points in time and in a specific sequence or specific order, and further through specific inlets of the one or more inlets of the reaction vessel during the synthesis cycles of the automated synthesis of oligo- and polysaccharides on a solid support. It is therefore preferred that the reagent delivery system comprises suitable technical means, preferably suitable technical means electronically coupled to a computing device comprising at least one processor, for delivery of one or more reagents and/or one or more reagent solutions and/or one or more solutions in different amounts, at different points in time and in a specific sequence or specific order during the synthesis cycles of the automated synthesis of oligo- and polysaccharides on solid support. The reagent delivery system may comprise a pump system or one or more pumps such as syringe pumps, peristaltic pumps or other suitable pumps and may further comprise one or more valves or one or more valve assemblies, one or more manifolds, one or more distributing components and similar suitable technical means. It is preferred that the pump system or the one or more pumps, the one or more valves or the one or more valve assemblies, the one or more manifolds and/or the one or more distributing components and similar technical means are under control of a computing device comprising at least one processor.

The device of the present invention may therefore comprise one or more valves or valve assemblies for regulating, directing or controlling the flow of fluids like gases or liquids. Valves or valve assemblies may be adapted for opening, closing or partially obstructing various passageways. Valves may be operated in gradual change between two or more positions, such as in two-port, three-port, four-port or in multiport valves. The device of the present invention may comprise one or more fluidic valves operably connected to one or more components of the device for automated synthesis of oligo- and polysaccharides on a solid support. Each fluidic valve of the device of the present invention may be a rotary valve, solenoid valve block or other multi-port valve or valve system or other suitable valves known to those skilled in the art. The device of the present invention may further comprise one or more pumps such as syringe pumps, peristaltic pumps or other pumps known to those skilled in the art which may be operably connected to one or more fluidic valves or valve assemblies of the device of the present invention.

In preferred embodiments of the device of the present invention the reagent delivery system may comprise one or more valves, preferably one or more fluidic valves, more preferably one or more rotary valves, and even more preferably one or more rotary valves with 3, 4, 5, 6, 7, 8 , 9 or 10 ports. Preferably the reagent delivery system may comprise one or more valves such as one or more rotary valves, one or more solenoid valve blocks or other multiport valves. The reagent delivery system may further comprise one or more pumps or a pump system. Preferably, the reagent delivery system comprises at least one syringe pump. Preferably, the reagents may be delivered via a syringe pump system. Preferably the reagents may be delivered via a syringe pump system as a component of the reagent delivery system. Preferably, the syringe pump system may be a component of the reagent delivery system.

In preferred embodiments of the present invention a syringe pump may drive the fluids within the reagent delivery system. The syringe pump and the reagent delivery system may be connected through one or more loop lines. Loop lines allow for careful delivery of sensitive and/or corrosive chemical solutions. The resting volume of the loops is defined as a function of the tubing length. A driving solvent may be taken to fill up the loops ahead the withdrawing of chemical solution from the reagent delivery system. This avoids the direct contact of the reagents with the syringe pump, which prevents pump deterioration and cross-contamination. The loops may be made from an inert material such as, for example, Teflon, poly-(tetrafluoroethylene) (PTFE) and the like. The size of the loops may be varied. The exact size will depend on the capacity of the syringe pump and the amount of reagent to be delivered to the reaction vessel. The size of each loop will also depend on the nature of the reagent to which it is associated. For example, if the reaction vessel is 20 mL, then a loop sized from about 1 to 5 mL may be used, preferably from about 2 to 4 mL. Each loop may be sized the same or different. For example, loops attached to building blocks may be smaller than those attached to basic reagents such as the deprotection or capping reagents as the quantity of the former used during any synthesis step is relatively small compared to the amount of such basic reagents.

In preferred embodiments of the present invention the reagent delivery system may be connected to a pre-cooling device. With other words, in preferred embodiments of the present invention the reagent delivery system may be connected to a pre-cooling device for pre-cooling the reagents to be supplied. The reagent delivery system device may be adapted for receiving reagents from the reagent storing device and may be further adapted for delivery of the received reagents to a pre-cooling device for pre-cooling the reagents to be supplied and may be further adapted for delivery of the pre-cooled reagents to the reaction vessel. Thus, in preferred embodiments of the present invention the reagent delivery system may be adapted for receiving one or more reagents such as building blocks and activators and/or one or more reagent solutions such as building block solutions and activator solutions and/or one or more washing solutions and/or one or more deprotection solutions from a reagent storing component or one or more reagent storing components and may be further adapted for delivery of the received one or more reagents and/or one or more reagent solutions and/or one or more washing solution and/or one or more deprotection solutions to a pre-cooling device for pre-cooling the reagents to be supplied and may be further adapted to deliver the pre-cooled one or more reagents and/or one or more reagent solutions and/or one or more washing solutions and/or one or more deprotection solutions to the reaction vessel.

In preferred embodiment of the present invention the reagent delivery system may be adapted for delivery of one or more of the reagents and/or reagents solutions and/or washing solutions and/or deprotection solution to the pre-cooling device for pre-cooling the reagents to be supplied. In such embodiments it is particularly preferred that at least the building blocks and the activators are delivered to the pre-cooling device for pre-cooling the reagents to be supplied. In further preferred embodiments the reagent delivery system may also be adapted for delivery of the washing solutions or washing solvents to the pre-cooling device for pre-cooling the reagents to be supplied.

In preferred embodiments of the present invention the reagent delivery system may be in thermal communication with a pre-cooling device for pre-cooling the reagents to be supplied. In further preferred embodiments of the present invention one or more delivery lines connecting the reagent delivery system and the reaction vessel may be in thermal communication with a pre-cooling device for pre-cooling the reagents to be supplied. Thus, in preferred embodiments of the present invention the reagent delivery system may be adapted for delivery of one or more reagents to the reaction vessel, the reagent delivery system in thermal communication with a pre-cooling device for pre-cooling the reagents to be supplied, wherein the one or more reagents are pre-cooled by the pre-cooling device before entering the reaction vessel, and preferably wherein the one or more reagents are pre-cooled through contact cooling by the pre-cooling device for pre-cooling the reagents to be supplied.

In preferred embodiments of the present invention the reagent delivery system may be interposed between a pre-cooling device and the reagent storing component. In preferred embodiments of the present invention the reagent delivery system is connected to the reagent storing component and is further connected to a pre-cooling device. It is particularly preferred that the reagent storing component and the pre-cooling device are not directly connected to each other. In other words it is preferred that the reagent storing component and the pre-cooling device are connected through the reagent delivery system. It is further preferred that the pre-cooling device for pre-cooling the reagents to be supplied connects the reagent delivery system with the reaction vessel. Thus, in particularly preferred embodiments of the present invention the reaction vessel, the pre-cooling device, the reagent delivery system and the reagent storing device are successively connected in the following sequence: reaction vessel - pre-cooling device - reagent delivery system - reagent storing device.

As already described above in preferred embodiments of the present invention the reaction vessel may comprise one or more inlets at the top of the reaction vessel and may comprise one or more inlets at the bottom of the reaction vessel. In further preferred embodiments the reaction vessel may comprise one or more inlets at the top of the reaction vessel and may comprise only one inlet at the bottom of the reaction vessel. The reagent delivery system may be connected to the reaction vessel through the one or more inlets at the top of the reaction vessel and/or through the one or more inlets at the bottom of the reaction vessel. In preferred embodiments of the present invention the reagent delivery system may be adapted to deliver building blocks or building block solutions, activators or activator solutions, and washing solvents or washing solutions through the one or more inlets at the top of the reaction vessel. The reagent delivery system may be further adapted to deliver deprotection solutions and capping solutions through the one or more inlets at the bottom of the reaction vessel. The reagent delivery system may be further adapted to deliver inert gas, such as argon, to the reaction vessel.

The reagent delivery system may comprise one or more reagent distribution components. The reagent delivery system may comprise one or more reagent distribution components for delivery of reagents and/or solutions and/or reagent solution to the reaction vessel. Each of the reagent distribution components may be connected to the reagent storing component or one or more reagent storing components. In preferred embodiments of the present invention the reagent delivery system comprises a first and a second reagent distribution component for driving the flow of fluids or gases to the reaction vessel. In preferred embodiments of the present invention the device comprises a first reagent distribution component and a second reagent distribution component.

Therefore, the present invention preferably relates to a device for automated synthesis of oligo- and polysaccharides on a solid support, the device comprising:
(a) a reaction vessel,
(b) a reagent storing component,
(c) a reagent delivery system comprising a first reagent distribution component and a second reagent distribution component,
(d) a cooling device for cooling the reaction vessel,
(e) and a pre-cooling device for pre-cooling the reagents to be supplied.

The first reagent distribution component may be connected to the reaction vessel through the one or more inlets on the top of the reaction vessel. Thus, the first distribution component or the top distribution component may be in fluid communication with the reaction vessel through the one or more inlets at the top of the reaction vessel. The second distribution component may be connected to the one or more inlets at the bottom of the reaction vessel. Thus, the second distribution component or bottom distribution component may be in fluid communication with the reaction vessel through the one or more inlets at the bottom of the reaction vessel.

Preferably the first distribution component or the top distribution component supplies the washing solvents, building blocks or building block solutions and activators or activator solutions to the reaction vessel. It is particularly preferred that the first distribution component or the top distribution component is connected to a pre-cooling device for pre-cooling the reagents to be supplied for delivery of pre-cooled building blocks or building block solutions and activators or activator solutions to the reaction vessel. In preferred embodiments of the present invention the first distribution component or the top distribution component may also deliver the washing solvents or washing solution to the pre-cooling device and thereafter may supply pre-cooled washing solvents or washing solution to the reaction vessel. The top distribution component may be also adapted to provide a ventilation exit for gases.

Preferably the second distribution component or the bottom distribution component supplies the deprotection solutions, capping solutions and supplies inert gas (bubbling gas) to the reaction vessel. The inert gas may be provided as bubbling gas which may be used for mixing and creating an inert and anhydrous atmosphere inside the reaction vessel. In preferred embodiments the bottom distribution component is also adapted for discharging the liquids or solution after one or more synthesis or washing steps.

Therefore, the present invention preferably relates to a device for automated synthesis of oligo- and polysaccharides on a solid support, the device comprising:
(a) a reaction vessel,
(b) a reagent storing component,
(c) a reagent delivery system comprising a first reagent distribution component for delivery of washing solvents, building blocks and/or building block solutions and activators and/or activator solutions and a second reagent distribution component for delivery of deprotection solutions,
(d) a cooling device for cooling the reaction vessel,
(e) and a pre-cooling device for pre-cooling the reagents to be supplied.

In preferred embodiments of the present invention the reagent delivery system or the one or more reagent distribution components may further comprise one or more reagent distribution sub-components. In preferred embodiments of the present invention the reagent delivery system may comprise a building block distribution component for delivery of the building blocks and/or building block solutions, an activator distribution component for delivery of the activators and/or the activator solutions and/or a washing solvent distribution component for delivery of washing solvents or washing solution, a deprotection distribution component for delivery of deprotection solution and may further comprise a capping distribution component for delivery of capping solutions to the reaction vessel.

In preferred embodiments of the present invention the **top distribution component** may comprise a building block distribution component, an activator distribution component and a washing solvents or washing solutions distribution component. The top distribution component may further comprise a syringe pump for driving the fluids within the top distribution component. The lines between the syringe pump and the top distribution component may be provided in form of loop lines to allow for careful delivery of sensitive and/or corrosive chemical solutions. The resting volume of the loops is defined as a function of the tubing length. The top distribution component may further comprise a washing solvents component, wherein a driving solvent may be taken to fill up the loops ahead the withdrawing of chemical solutions from the building blocks distribution component and the activator distribution component. This avoids the direct contact of the reagents with the syringe pump, which prevents the pump deterioration and cross-contamination.

The **building block distribution component** may comprise one or more valves or a valve assembly or similar technical means, for example, a rotary valve that distributes the fluids within the building block distribution component. The building block distribution component is connected to the reagent storing component, preferably to a building blocks storing component which may comprise one or more building block containers or building block solution containers. Each of the building block containers or building block solution containers may be connected to the one or more valves or valve assembly or similar technical means. The building block containers of the building block storing component may be connected to a gas distribution system to provide inert gas to each of the building block containers. Thus, each of the building block containers may be stored under anhydrous and inert atmosphere. Each of the building block containers or building block solutions containers may be in fluid communication with the building block distribution component through the one or more valves or valve assembly or similar technical means. The one or more valves or the valve assembly may be further connected to the reaction vessel. Thus, the reaction vessel may be in fluid communication with the building block distribution component through the one or more valves or valve assembly or similar technical means. The reaction vessel may be in fluid communication with the building block distribution component through, for example, one or more valves or valve assembly and may be further connected through a building blocks delivery line. The building block distribution component may be adapted to deliver the building blocks to a pre-cooling device for pre-cooling the reagents to be supplied and may be further adapted to deliver pre-cooled building blocks to the reaction vessel. The building block distribution component may be also connected to a waste container and/or gas distribution system for receiving inert gas through one or more further delivery lines. The one or more valves or valve assembly or other suitable technical means for distribution of the building blocks within the building block distribution component may be under control of a processor of a computing device configured to control the distribution of the building blocks and preferably configured to control the one or more valves or valve assembly such as a rotary valve distributor. The building block distribution component may be connected to a syringe pump system, for example, through one or more valves or valve assembly and may be further connected to the syringe pump via a loop line.

The **activator distribution component** may comprise one or more valves or a valve assembly or similar technical means for example a rotary valve that distributes the fluids within the activator distribution component. The activator distribution component is connected to a reagent storing component, preferably an activator or activator solution storing component which may comprise one or more activator containers or activator solution containers. The activator containers of the activator storing component may be connected to a gas distribution system to provide inert gas to each of the activator containers. Thus, each of the activator containers may be stored under anhydrous and inert atmosphere. Each of the activator containers or activator solution containers may be connected to the one or more valves or valve assembly or similar technical means of the activator distribution component. Thus, each of the activator containers or activator solutions container may be in fluid communication with the one or more valves or valve assembly of the activator distribution component. The activator distribution component may be connected to the reaction vessel through the one or more valves or valve assembly or similar technical means. Thus, the reaction vessel may be in fluid communication with the activator distribution component through the one or more valves or valve assembly or similar technical means. The reaction vessel may be in fluid communication with the activator distribution component through one or more valves or valve assembly or similar technical means and may be further connected through an activator delivery line. The activator distribution component may be adapted to deliver the activators to a pre-cooling device for pre-cooling the reagents to be supplied and may be further adapted to deliver pre-cooled activators to the reaction vessel. The activator distribution component may be also connected to a waste container and/or to a gas distribution system for receiving inert gas through one or more delivery lines. The one or more valves or valve assembly or similar technical means may be under control of a processor of a computing device configured to control the distribution of the activators within the activator distribution component and preferably configured to control the one or more valves or valve assembly and similar technical means such as a rotary valve distributor. The activator distribution component may be connected to a syringe pump system, for example, through one or more valves or valve assembly and may be further connected to the syringe pump via a loop line.

The **washing solvent distribution** component may provide the reaction vessel with one or more washing solvents or washing solutions. Exemplary washing solvents suitable for automated synthesis of oligo- and polysaccharides on a solid support include, but are not limited to dichloromethane (DCM), tetrahydrofuran (THF) and dimethylformamide (DMF). The washing solvent distribution component is connected to a reagent storing component, preferably a washing solvent storing component. Each of the washing solvents may be stored in a respective solvent container of the washing solvent storing component. Thus, the washing solvent distribution component may be connected to a washing solvent storing component comprising one or more solvent containers. The washing solvent distribution component may be further connected to a syringe pump. One or more of the solvent containers may be connected to the syringe pump through the washing solvent distribution component. The washing solvent distribution component may comprise one or more valves or valve assembly or similar technical means which may be connected to each of the solvent containers of the washing solvents storing component. As an example, the washing solvent distribution component may comprise a multi-port valve, such as a four way magnetic valve. The washing solvents may be delivered to the reaction vessel by the washing solvent distribution component. The washing solvents may be delivered to the reaction vessel through a washing solvent delivery line. The washing solvent distribution component may be adapted to deliver the washing solvents to a pre-cooling device and may be further adapted to deliver pre-cooled washing solvents to the reaction vessel. The washing solvent containers of the washing solvent storing component may be connected to a gas distribution system to provide inert gas to the each washing solvent container. Thus, each of the washing solvent containers may be stored under anhydrous and inert atmosphere, such as under argon atmosphere. The magnetic valve may be further connected to the gas distribution system to receive inert gas and to provide and deliver inert gas to the microwave transparent reaction vessel. The one or more valves or valve assembly or similar technical means suitable for distribution of the washing solvents within the washing solvent distribution component may be under control of a processor of a computing device configured to control the distribution of the washing solvents and washing solution and preferably configured to control the one or more valves or valve assembly and similar technical means such as a magnetic valve distributor.

The **bottom distribution component** may comprise one or more valves such as one or more multi-port valves, or valve assemblies, or similar technical means. The bottom distribution component may comprise a deprotection distribution component for delivery of deprotection solutions to the reaction vessel and may further comprise a capping distribution component for delivery of capping solution to the reaction vessel. The bottom distribution component may be further connected to a waste container. The bottom distribution component may be further connected with a gas distribution component for delivery of inert gas, such as argon, to the reaction vessel. One or more of the components of the bottom distribution component may be connected to the reaction vessel through a loop line which allows for delivery of chemical solutions from the deprotection distribution component, the capping distribution component and delivery of inert gas from the gas distribution component. The loop lines may be also adapted for discharge of waste liquids from the reaction vessel. The bottom distribution component may comprise for example a multiple ways magnetic valve which may be electronically coupled to a computing device comprising at least one processor configured to control the delivery of deprotection solutions, capping solutions and inert gas to the reaction vessel and further configured to control the discharging of liquids and solutions of the reaction vessel after each reaction or washing step. The deprotection distribution component is connected to a reagent storing component, preferably a deprotection solution storing component. The deprotection storing component may comprise one or more containers for storing deprotection reagents. The deprotection storing component may be connected to a gas distribution system to provide inert gas to the each deprotection reagent container. Thus, each of the deprotection reagent containers may be stored under anhydrous and inert atmosphere, such as under argon atmosphere. The capping distribution component is connected to a reagent storing component, preferably a capping solution storing component. The capping storing component may comprise one or more containers for storing capping reagents. The capping storing component may be connected to a gas distribution system to provide inert gas to the each capping reagent container. Thus, each of the capping reagent containers may be stored under anhydrous and inert atmosphere, such as under argon pressure.

The device of the present invention may further comprise a **gas distribution system** to provide anhydrous and inert gas to one or more components of the device of the present invention for mixing, atmosphere conditioning and driving fluid purposes. Preferably the device for automated synthesis of oligo- and polysaccharides on a solid support is adapted for performing reactions under anhydrous and inert atmosphere. The gas distribution system comprises at least one gas container, such as an argon container, to provide inert gas to the one or more components of the device of the present invention. The gas distribution system may comprise one or more valves, valve assemblies, one or more vents and/or one or more manifolds or similar technical means to allow the distribution and delivery of inert gas to the one or more components of the device of the present invention. The one or more valves, valve assemblies, one or more vents and/or one or more manifolds or similar technical means allow control of the pressure within the one or more lines which connect the one or more components of the device of the present invention. The one or more valves, valve assemblies, one or more vents and/or one or more manifolds or similar technical means may be under control of a computing device comprising at least one processor. The processor may be configured to control the delivery and distribution of the inert gas within the device of the present invention and is configured to control gas pressure within the one or more lines and one or more components of the device of the present invention.

According to the present invention the device for automated synthesis of oligo- and polysaccharides on a solid support comprises a cooling system. According to the present invention the device for automated synthesis of oligo- and polysaccharides on a solid support comprises at least one **cooling device** for cooling the reaction vessel or more precisely for cooling the content of the reaction vessel and at least one **pre-cooling device** for pre-cooling the reagents to be supplied to the reaction vessel. The cooling device provides at least active cooling of the reaction vessel. The pre-cooling device provides at least active cooling of one or more of the reagents to be supplied to the reaction vessel.

Currently, the automated glycosylation process requires expensive and large cooling systems. Thus, the devices for automated synthesis of oligo- and polysaccharides on a solid support known in the art require expensive and large cooling systems. However, the devices known in the art have shown several disadvantages in performing coupling reactions in connection with the use of common reagents especially during scale-up of the synthesis of oligo- and polysaccharides on the solid support. Even if the devices known in the art allow for active cooling of the reaction vessel during the coupling reactions, not sufficiently high yields were obtained, decomposition of reagents and formation of side and decomposition products were detected.

Next the cooling of the reaction vessel was adjusted to even lower temperatures which did actually not result in the desired improvement of yield and reduction of decomposition and decrease of formation of side and decomposition products. Also a very slow addition and careful delivery of the reagents to the reaction mixture under simultaneous monitoring of the temperature of the reaction mixture in the reaction vessel did also not bring the expected improvement in yield and reduction of side and decomposition products. Moreover, due to the very slow addition and careful delivery of the reagents, the total reaction time increased to an inacceptable degree while simultaneously also the side and decomposition products increased without the desired enhancement of the yield. In addition, up-scaling of this way running the reaction was almost impossible.

Thus, also with the devices known in the art allowing slow and careful delivery of the reagents to the reaction mixture under simultaneous monitoring of the temperature of the reaction mixture have shown a disadvantageous impact on the resulting yield of the desired products, in particular with regard to larger batch sizes. The delayed addition of the reagents, particularly in larger batch sizes, resulted in still unsatisfying overall yields of the desired oligo- and polysaccharides.

However, after several attempts failed to increase yield and reduce side and decomposition products especially by up-scaled reactions, the objective could be solved by the pre-cooling device and by pre-cooling the reagents to be supplied to a temperature close to the temperature of the reaction mixture in the reaction vessel. Thus, the device of the present invention comprises an improved cooling system, in particular comprises a cooling device for cooling the reaction vessel and a pre-cooling device for pre-cooling the reagents to be supplied and has been shown to be particularly advantageous for automated synthesis of oligo- and polysaccharides on a solid support especially in connection with larger batch sizes and therefore overcomes the disadvantages of the devices known in the art.

Thus, the inventors of the present patent application have surprisingly found that a device for automated synthesis of oligo- and polysaccharides on a solid support comprising a reaction vessel, a reagent storing component, a reagent delivery system, a cooling device for cooling the reaction vessel, and a pre-cooling device for pre-cooling the reagents to be supplied to a temperature close to the temperature of the mixture in the reaction vessel, overcomes the disadvantages of the devices known in the art for automated synthesis of oligo- and polysaccharides on a solid support.

The inventors have found that pre-cooling of the reagents prevents and avoids decomposition of the reagents during the synthesis cycles which otherwise could result in undesired side-products and decomposition products. The inventors have found that with a device for automated synthesis of oligo- and polysaccharides on a solid support comprising at least a cooling device for cooling the reaction vessel and a pre-cooling device for pre-cooling the reagents to be supplied, conversion and yield could be increased, decomposition of reagents could be decreased and formation of side and decomposition products could effectively be prevented as the device allows for active cooling of the reaction vessel and additionally allows for active cooling of the reagents to be supplied to the reaction vessel.

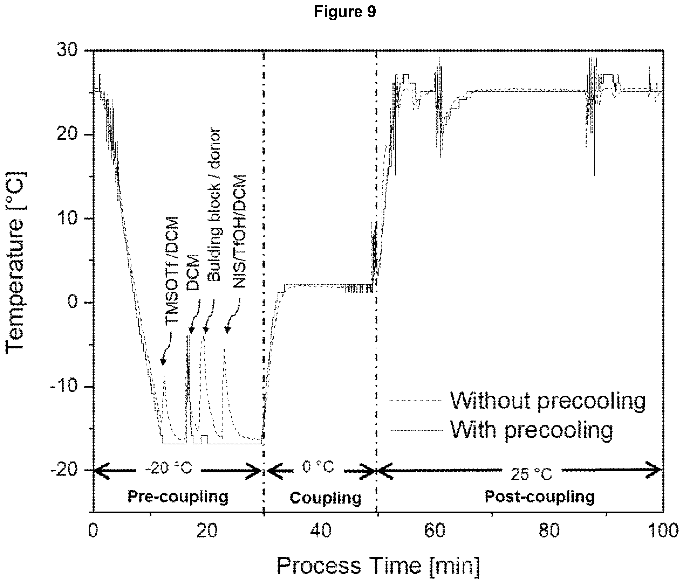

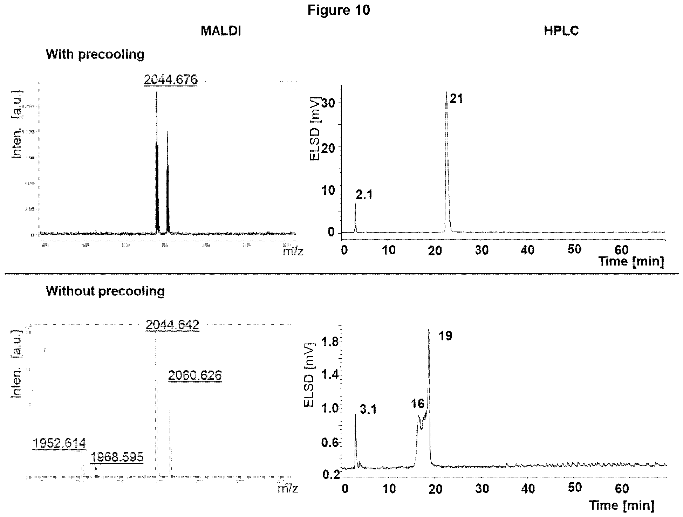

For example, Fig. 9 shows the thermal profile during the synthesis of a mannose-α-1,6-tetramer "standard test" in the presence and absence of pre-cooling. Here, the reactor is made of glass and a thioglycoside donor is used as building block, and active cooling of the reaction vessel by a cooling device is ON in both experiments. By using a pre-cooling device according to the present invention, the thermal perturbation due to the delivery of reagents is suppressed (solid line), specifically the delivery of activator reagents and building block solution / donor solution. In this example, the washing solvent was not pre-cooled to highlight the strong contrast in the thermal spike. Fig. 10 shows the analytical results of the experiments shown in Fig 9. Mass spectrometry (MALDI) and HPLC chromatograms are provided. The experiment performed in presence of a pre-cooling device for cooling the reaction vessel shows superior results: only the desired product was obtained. An overall yield of 60% was achieved. The reaction without the pre-cooling delivers a mixture of tetra-mannose and some partially deprotected tetra-mannose. This further complicates the purification process and results in lower overall yield.

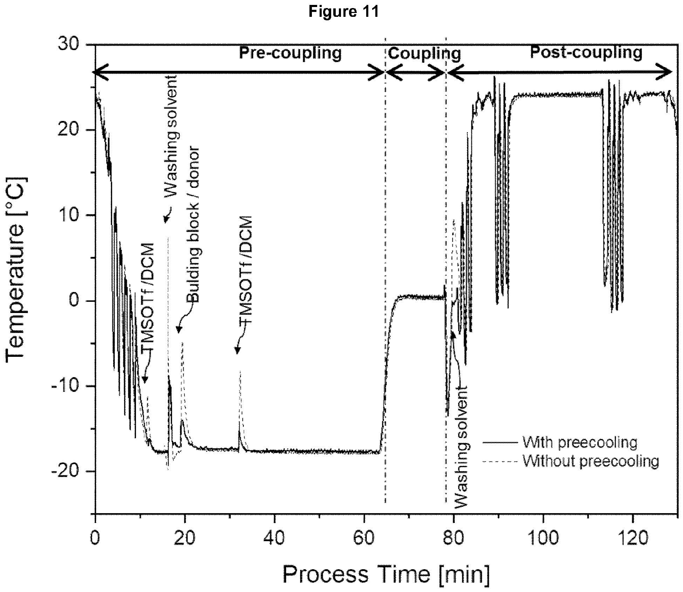

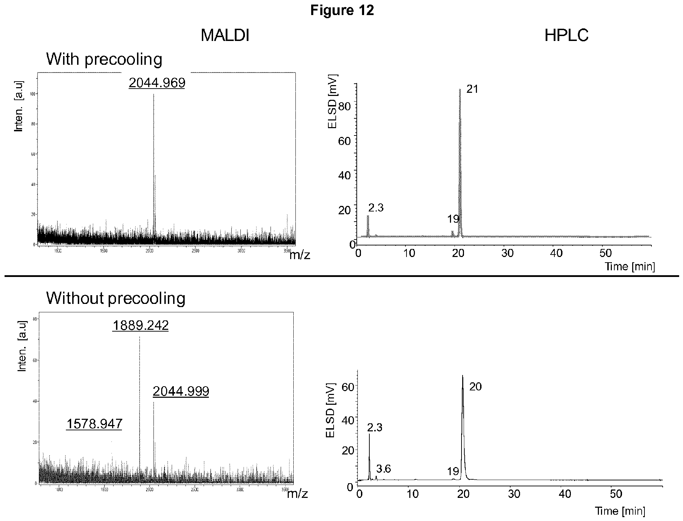

As a further example, Fig. 11 demonstrates the thermal profile during the synthesis of a mannose-α-1,6-tetramer "standard test" in the presence and absence of pre-cooling. The reaction vessel is made of glass, and a glycosyl phosphate donor is used as building block, and active cooling of the reaction vessel by a cooling device is ON in both experiments. By using a pre-cooling device according to the present invention, the thermal perturbation due to the delivery of reagents is suppressed (solid line), specifically the delivery of activator reagents and building block / donor solution. The washing solvent was not pre-cooled to highlight the strong contrast in the thermal spike. Fig. 12 shows the analytical results of the experiments as shown in Fig.11. Mass spectrometry (MALDI) and HPLC chromatograms are provided. The experiment performed in presence of a pre-cooling device according to the invention shows superior results: only the desired product was obtained. An overall yield of 60% was achieved. The reaction without the pre-cooling shows a HPLC chromatogram with more side product traces (peaks at 2.3 min and 3.6 min).

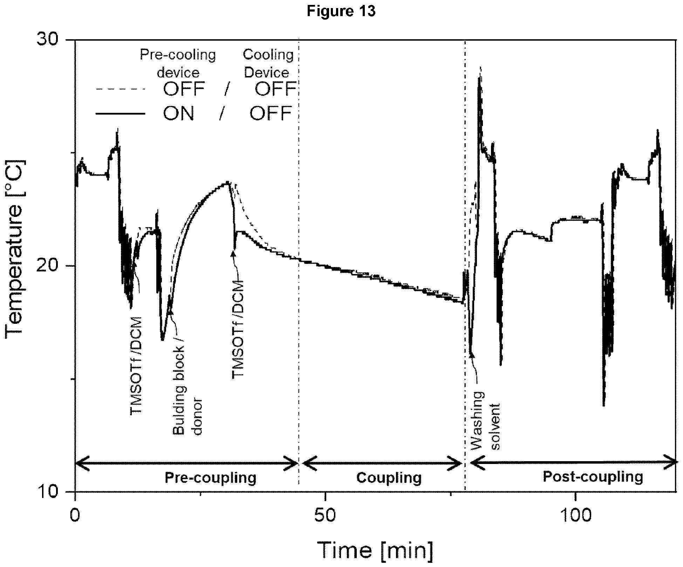

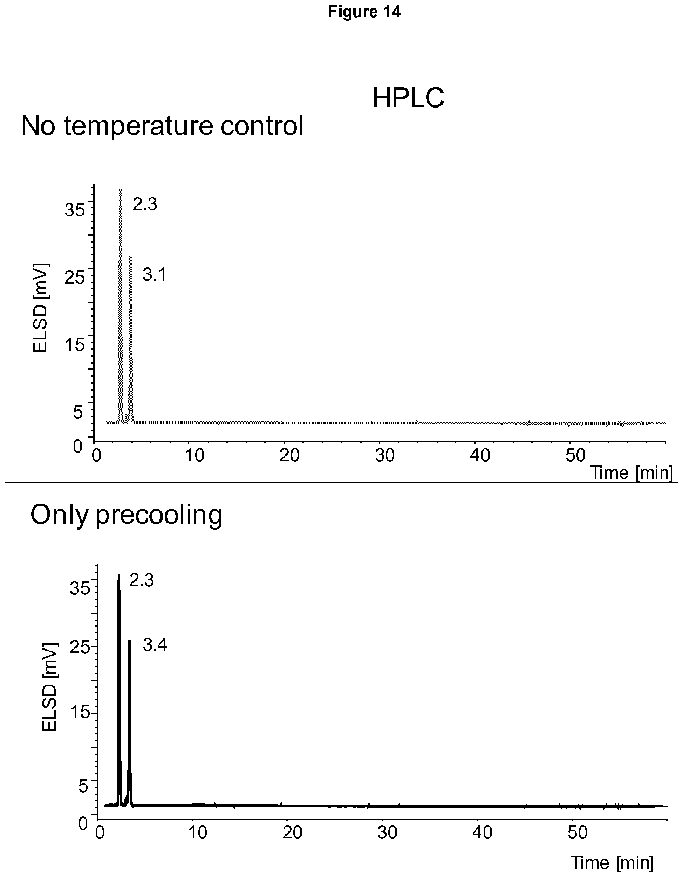

As a further example, Fig. 13 demonstrates the thermal profile during the synthesis of a mannose-α-1,6-tetramer "standard test" in the presence and absence of pre-cooling. The reaction vessel is made of PFA, and a glycosyl phosphate donor is used as building block, and active cooling of the reaction vessel by a cooling device is OFF in both experiments. By using a pre-cooling device according to the present invention, there is a brief drop in temperature when the pre-cooled reagents/building blocks are delivered (solid line). In both cases the temperature oscillates around 20 °C. Fig. 14 shows the analytical results of the experiments as described in Fig 13. HPLC chromatograms are provided. In both experiments no desired product was observed. Only side products are visible in HPLC chromatogram (peaks at 2.3 min and 3.4 min). Precise control of the temperature is therefore instrumental during the automated synthesis of oligosaccharides.

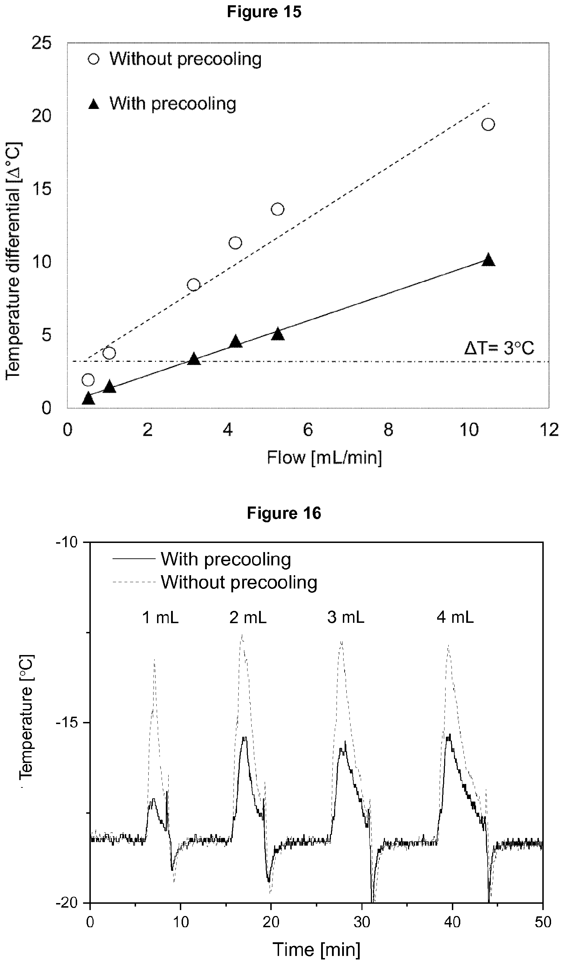

As a further example, Fig. 15 demonstrates the temperature differential (maximum temperate reached minus the actual reaction vessel temperature) within a glass reaction vessel when 1 mL of Dichloromethane is delivered at different flow rates to a reactor vessel at -18 °C. The white circle series refer to the solvent delivered without pre-cooling; the black triangle series refers to pre-cooled solvent. By using the pre-cooling device, the reagent can be delivered 3 times faster than the current state of the art devices with a thermal perturbation lesser than ±3 °C (highlighted in box).

As a further example, Fig. 16 demonstrates the thermal perturbation in the reaction vessel when different volumes of dichloromethane are delivered at 1.05 mL/min in a reaction vessel at -18 °C in the presence or absence of pre-cooling. The solid line refers to the thermal perturbation with pre-cooling. The dash line refers to the thermal perturbation without pre-cooling. The pre-cooling reduces the thermal perturbation even when 4 mL are delivered which corresponds 4 times the current scale of the current state of the art synthesizers. This presents a major advantage of the present invention in the up-scaling the synthesis of oligosaccharides.

The advantages and improvement of the automated glycosylation via pre-cooling of the reagents are most apparent on Figure 10 and 12. Furthermore, it has been found that by pre-cooling the reagents, the overall duration of a synthesis cycle can be reduced as the pre-cooling device allows rapid delivery of pre-cooled reagents. By using the pre-cooling device of the present invention, the building block/ donor could be delivered 3 times faster than the current state of the art devices; this was achieved without a thermal perturbation larger than 3 °C compared to the actual reaction vessel temperatures. In addition, the pre-cooling reduces the thermal perturbation even when 4 mL are delivered which is 4 times the current scale of the current state of the art devices. This presents a major advantage of the present invention in the up-scaling the synthesis of oligosaccharides.

As one synthesis cycle includes different thermal stages the device of the present invention additionally allows advantageous rapid and dynamic temperature adjustment for the different thermal stages of the synthesis cycles. Therefore, the device of the present invention allows dynamic cooling of the reaction mixture and simple and convenient adjustment of the temperature of the reaction vessel during the synthesis cycles of the synthesis of oligo- and polysaccharides on a solid support.

The synthesis cycle or glycosylation cycle or coupling cycle includes three stages. The first stage relates to providing and supplying of the reagents to the reaction vessel, the second stage relates to the coupling reaction which takes place inside of the reaction vessel followed by capping, deprotection and/or washing steps as third stage. Following the deprotection steps and optional capping steps the next synthesis cycle or glycosylation cycle may follow for introducing the next building block for building up the desired oligo- or polysaccharide in a step-by-step procedure. Therefore, three thermal stages are present in a synthesis cycle or glycosylation cycle. Thus, a synthesis cycle or glycosylation cycle may be classified into three temperature regimes or stages as shown in Fig.9 or Fig. 11.

The first stage relates to the pre-coupling regime in the range of -40°C to room temperature, in other words in the range of -40°C to 25°C and preferably in the range of -40 °C to -10 °C. In this pre-coupling regime the building block and activator are supplied or added and are allowed to impregnate the resin and to diffuse through the porous solid. The low temperature in the range of preferably -40°C to -10°C prevents the early decomposition of the intermediates before the actual coupling reaction.

The second stage relates to the coupling regime at a temperature around 0°C or in the range of -10°C to room temperature, in other words in the range of -10° to 25°C, preferably -10°C to 5°C, more preferably -10°C ± 3°C to 0°C ± 3°C.The increase in the temperature to preferably around 0°C allows the initiation of the coupling reaction by promoting the formation of the intermediates.

The third stage relates to the post-coupling regime at a temperature around 25°C (room temperature or standard temperature) or above. The capping and deprotection reactions take place at higher temperatures than the coupling reaction. These reactions complete the glycosylation cycle. Then, the next coupling cycle or termination of the process may take place.

According to the present invention the device for automated synthesis for oligo- and polysaccharides on a solid support comprises **a cooling device** for cooling the reaction vessel. The cooling device therefore provides active cooling to the reaction vessel and thus provides active cooling of the reaction mixture inside of the reaction vessel. The cooling device is therefore preferably in thermal communication with the reaction vessel. The cooling device is preferably adapted for cooling the reaction vessel to temperatures of -80°C to +60 °C, preferably -40°C to 40°C. Thus, the device of the present invention may comprise a cooling device for regulating the temperature of the reaction vessel. The cooling device may be under control of a computing device comprising at least one processor. The cooling device may be further adapted to maintain the temperature within the reaction vessel. The cooling device may be adapted to adjust the temperature within +1°C and -1°C of the reaction temperature. The cooling device may be a heating and/or cooling device equipped with a temperature sensor or thermometer, wherein the cooling device temperature may be adjusted either manually or preferably by a computing device. For example, the cooling device could be a heating bath, an external refrigerated circulator or a heating/cooling block. The heating/cooling block may be made of any heat transfer material. The block may have channels running through to pass coolant fluid through. The reaction vessel may be placed in a cavity of the heating/cooling block. In a preferred embodiment of the present invention the coolant fluid may be circulated around the reaction vessel via a sleeve surrounding the reaction vessel. In preferred embodiments of the present invention the cooling device may comprise a cooling jacket. The cooling jacket may be in form of a cooling coil surrounding the reaction vessel. Preferably, the cooling jacket is in thermal communication with the reaction vessel. The cooling coil or cooling jacket may be further provided with a thermal isolation cover to increase the heat exchange efficiency of the cooling jacket or cooling coil.

The reaction vessel may be constructed as a double-wall structure which forms two cavities, wherein the first cavity accommodates the synthesis of oligo- and polysaccharides and wherein the second cavity accommodates a coolant fluid. The double-wall structure of the reaction vessel may be made of glass. Thus, the reaction vessel may be provided with a cooling jacket for cooling the reaction vessel. Preferably, the cooling jacket may be made of a material such as glass, quartz, PTFE (Teflon), polypropylene or fluoropolymers. In preferred embodiments of the present invention the reaction vessel may be provided as an interchangeable reaction vessel. In such embodiments it is preferred that the reaction vessel may be removed from a cooling means such as a cooling jacket without interrupting the flow of a circulating coolant fluid. In such embodiments the reaction vessel may be removed for example for loading with solid support or for discharging the solid support. Furthermore such an interchangeable reaction vessel allows use of various reaction vessels with different sizes for different batch sizes.

The cooling device may be connected to a coolant fluid reservoir. The cooling device may be connected to a cooling circuit pump. In preferred embodiments of the invention the cooling jacket surrounding the reaction vessel may be connected to a coolant fluid reservoir and a coolant circuit pump. The coolant fluid reservoir, the coolant circuit pump and the cooling jacket surrounding the reaction vessel may form a closed cooling circuit. The cooling jacket may be provided in form of a cooling coil surrounding the reaction vessel. The coolant circuit pump may be under control of a computer comprising at least one processor. The cooling device may comprise a cooling unit configured to control the temperature of the cooling device. The cooling unit may be electronically coupled to a computing device comprising at least one processor. The cooling circuit pump may be electronically coupled to a computing device comprising at least one processor to control the flow of the coolant fluid through the cooling circuit.

In general, the incoming solutions are delivered at room temperature from the reagent delivery system to the reaction vessel. This supposes an increase of the temperature within the reactor vessel. Such a temperature increase is proportional to the incoming mass. This results in a detriment for the reagents and sensitive intermediates before the coupling. A pre-cooling component is introduced as a solution to this issue.

Since many glycosylation reactions require low temperatures and the formation of the intermediate reactive species could take place rather fast, the device of the present invention comprises a pre-cooling device to assure a minimum temperature difference between the inside of the reaction vessel and the incoming reagents, in particular the building blocks and the activators. A cooling device provides a better control of the temperature at which the solid phase support and reagents (such as the building block and activators) get mixed. At the same time a pre-cooling device provides active cooling that allows reaching temperatures until -30 °C.

Thus, according to the present invention the device for automated synthesis of oligo- and polysaccharides comprises a **pre-cooling device** for pre-cooling the reagents to be supplied.

Preferably the pre-cooling device is in thermal communication with the reagent delivery system. The pre-cooling device is preferably interposed between the reaction vessel and the reagent delivery device. The pre-cooling device is preferable positioned in close proximity to the one or more inlets of the reaction vessel. Thus, the pre-cooling device may be adapted to function as a pre-cooling inlet for pre-cooling the reagents to be supplied. The pre-cooling component may be adapted for active cooling of the reagents to be supplied. In other words the incoming solutions at room temperature from the reagent delivery system are actively cooled by the pre-cooling device to a temperature around the preset temperature of the reaction mixture in the reaction vessel. By pre-cooling the at room temperature incoming solutions to around the temperature of the reaction vessel the reagents to be supplied are added to the reaction mixture at a temperature that preferably corresponds to the temperature of the reaction mixture in the reaction vessel.

In one embodiment, all reagents and solvents, including, building blocks, activators and washing solvents, are pre-cooled before addition to the reaction mixture in the reaction vessel. In one embodiment, only the activators or activator solutions are pre-cooled before addition to the reaction mixture in the reaction vessel. Preferably at least the building blocks and activators (or a solution thereof) are pre-cooled before addition to the reaction mixture in the reaction vessel and preferably all reagents and solutions added to perform the coupling reaction are pre-cooled close to the temperature of the reaction mixture in the reaction vessel. Furthermore, the resulting intermediates are often temperature sensitive and may decompose at higher temperatures then the desired temperature of the reaction mixture. It should be clear that already formed temperature sensitive intermediates in the reaction mixture may decompose in case further warmer reagents or solvents are added to the reaction mixture, such as reagents or solvents at room temperature. In such a case it may be suitable to supply these reagents or solvents in a slow and careful manner, for example dropwise manner, by monitoring the temperature of the reaction mixture at the same time. However, in larger batch sizes a larger amount of the reagents to be supplied are added to the reaction mixture. The delayed addition, for example dropwise addition, and the period of time until the reaction mixture is adjusted to the desired and preset temperature may also result in decomposition of the reagents and in particular the sensitive intermediates. The inventors of the present invention have found that on the one hand the rapid addition of the reagents to the reaction mixture in the pre-coupling stage and impregnating of the resin to allow these reagents to diffuse through the porous solid for a period of time of 1 minute to 20 minutes, preferably 5 minutes has an advantageous effect on the overall yield of the desired oligo- and polysaccharide. On the other hand the inventors have found that the monitoring of the reaction mixture temperature during the addition of reagents and solvents and the careful and slow addition of these reagents such that the reaction mixtures maintains the desired reaction mixture has also an advantageous effect on the overall yield of the desired oligo- and polysaccharide. The device of the present invention comprising a reaction vessel, a reagent storing component, a reagent delivery system, a cooling device for cooling the reaction vessel, and a pre-cooling device for pre-cooling the reagents to be supplied has the advantage that the pre-cooled reagents to be supplied may be added in a faster and rapid manner to the reaction mixture without unfavorable temperature fluctuations in the reaction mixture in the pre-coupling stage at preferably -40°C to -10°C and has the advantages that the added pre-cooled reagents may be allowed to impregnate the resin to diffuse through the porous solid for a period of time of 1 minute to 20 minutes, preferably 5 minutes such that the overall time of the pre-coupling stage including the adding and impregnating of the reagents may be reduced such that decompositions of the reagents and the resulting intermediates may be effectively prevented in comparison to a slow and careful addition of the reagents to be supplied which may result in an overall time of the pre-coupling stage of over 25 minutes or 30 minutes or more.

The rapid addition of the pre-cooled reagents in the pre-coupling stage to allow effective impregnation of the resin and the prevention of the temperature fluctuation during the addition of the pre-cooled reagents has been shown to be particularly advantageous with regard to the glycosylation reactions in the synthesis of oligo- and polysaccharides.

The pre-cooling device is preferably adapted for pre-cooling of the reagents to be supplied to the corresponding temperature of the reaction mixture in the reaction vessel. For example, if the reaction vessel is actively cooled to a temperature of -30° in the pre-coupling stage of a synthesis cycle the reagents to be supplied may preferably be pre-cooled also to a temperature of -30°C by the pre-cooling device or preferably to -31°C or -32°C or -33°C or to a lower temperature. In such a case the pre-cooled reagents at a temperature of -30°C are added to the reaction mixture having a temperature of -30°C, wherein temperature fluctuations in the reaction mixture are prevented during addition of the reagents. However, the reagents to be supplied to the reaction vessel may be also added at a slightly higher temperature then the temperature of the reaction mixture in the reaction vessel.

Preferably the reagents are pre-cooled by the pre-cooling device to a temperature in the temperature range corresponding to the temperature range of -40°C to -8°C, preferably of -30°C to -9°C, and more preferably to the temperature range of -20°C to -10°C of the pre-coupling stage. In preferred embodiments of the present invention the pre-cooling device may be adapted for pre-cooling the reagents to be supplied at least to a temperature which is not higher than -3°C, preferably -6°C, more preferably -8°C or -10°C of the temperature of the reaction mixture in the transparent reaction vessel so that the temperature of the supplied reagents is not higher than 3°C, preferably 6°C, more preferably 8°C or 10°C below the temperature of the reaction mixture in the reaction vessel. Preferably the pre-cooling device may be adapted to cool the reagents to be supplied to a temperature in the range of 3°C to 6°C below the temperature of the reaction mixture in the reaction vessel. Thus, it is preferred that the reagents to be supplied are pre-cooled to the corresponding temperature of the reaction vessel with a maximum variance of 0°C to 10°C below the temperature of the reaction mixture in the reaction vessel. Most preferably the temperature of the reaction mixture in the reaction vessel does not exceed a temperature of -10°C in the pre-coupling stage of the synthesis cycle.

Thus, it is preferred that the reagents to be supplied are pre-cooled to the corresponding temperature of the reaction vessel with a maximum variance of 10°C or maximum deviation of 10°C, preferably with a maximum variance of 5°C or maximum deviation of 5°C, more preferably with a maximum variance of 3°C or maximum deviation of 3°C. In preferred embodiments it is preferred that the temperature of the reaction mixture in the reaction vessel does not exceed a temperature of -10°C in the pre-coupling stage of the synthesis cycle.

Most preferably, the temperature of the reagents to be supplied to the reaction vessel is in the range of -18°C to -8°C, preferably in the range of -16°C to -9°C, more preferably in the range of -14°C to -10°C and still more preferably in the range of -12°C to -10°C.

In preferred embodiments of the present invention various liquid lines such as washing solvents lines, building block lines, and activator solution lines may be pre-cooled before feeding the reaction vessel. In other words the pre-cooling device may be located upstream to the reaction vessel. The pre-cooling device may be interposed between the reaction vessel and the reagent delivery system.

Thus, the reaction vessel may be connected to a pre-cooling device which allows active pre-cooling of various liquid lines such as washing solvents, building blocks, activator solution before said washing solvents, building blocks, activator solution are delivered and supplied to the reaction vessel. In such embodiments the flow of liquid to the reaction vessel from the reagent delivery system is not interrupted as the reagents to be supplied to the reaction vessel are pre-cooled during the delivery to the reaction vessel. Preferably the flow speed of the liquids through the various liquid lines are taken into account as by lower flow speeds the reagents may be pre-cooled for a longer period of time to enable effective cooling to the desired temperatures. In one embodiment, all liquid lines between the reagent delivery system and the reaction vessel are pre-cooled by the pre-cooling device located between the reagent delivery system and the reaction vessel. In one embodiment, only one liquid line between the reagent delivery system and the reaction vessel is pre-cooled by the pre-cooling device located between the reagent delivery system and the reaction vessel. In a further embodiment, at least one liquid line between the reagent delivery system and the reaction vessel is pre-cooled by the pre-cooling device located between the reagent delivery system and the reaction vessel.

In preferred embodiments of the present invention the pre-cooling device may comprise a metal cooling surface made of a metal heat exchange material. In such embodiment the building block delivery line and activator line may contact the metal cooling surface at a preset temperature. Thus, the metal cooling surface may be cooled to a predefined temperature. Preferably the predefined temperature corresponds to the temperature of the reaction mixture in the reaction vessel, thus preferably corresponds to the desired reaction temperature. The metal cooling surface may be made of any known metal heat exchange material. In further preferred embodiments the washing solvent delivery line may also contact the metal cooling surface at a preset temperature.

In preferred embodiments of the present invention the pre-cooling device may comprise a thermoelectric contact-cooling device such as a Peltier cooler. The cooling effect provided by a Peltier cooler is based on the well-known Peltier effect. The Peltier cooler is adapted to provide the means to reach the preset temperature. Thus, the Peltier cooler may be in thermal communication with the metal cooling surface.

In preferred embodiments the thermoelectric contact-cooling device is assisted by a line of coolant fluid, such as for example cooling water, preferably at a temperature of 15°C to cool down the warm side of the thermoelectric contact-cooling device. The cooling water line could be provided by a central cooling system or a compact commercial cooling unit.

In preferred embodiments of the present invention the pre-cooling device may also provide active cooling to the reaction vessel. In other words the pre-cooling device may be a part or component of the cooling device. In such embodiments the pre-cooling device may provide active cooling to the cooling jacket surrounding the reaction vessel. Thus, the pre-cooling device may be adapted to provide active cooling to the cooling circuit comprising the cooling jacket, cooling circuit pump and the coolant fluid reservoir. In such embodiments the cooling device for cooling the reaction vessel and the pre-cooling device for pre-cooling the reagents to be supplied are adjusted simultaneously to the same preset temperature. Thus, based on the preset temperature for cooling of the reaction vessel the reagents to be supplied will be pre-cooled to the temperature of the reaction vessel.

The cooling device serves as temperature control unit and should be capable of regulating and maintain the temperature of the inside of the reaction vessel at a desired temperature(s). To accomplish the automated solid-phase synthesis of many different types of oligosaccharides, the temperature control unit in form of the cooling device may be capable of maintaining the temperate inside of the reaction vessel at a set temperature of between room temperature and -30 °C. To achieve this the Peltier cooler (thermoelectric) contact-cooling device may provide the means to cool down a metal surface of the pre-cooling device reaching temperature equal or lower to the set reaction temperature to compensate later temperature increases downstream. Channels or groves on the cooling surface may enhance the contact with the reagent delivery lines improving the heat transfer. The pre-cooling device is placed external to a reaction chamber and before the inlets to the reaction vessel. Pre-cooling action may be provided by contacting the surface with lines for delivering the building blocks and activator solution. The washing solvents delivered by the solvent delivery line could be pre-cooled as well by the action of the Peltier cooler by contacting the cooling surface. An active cooling action may be given by a coolant liquid served from a coolant reservoir that is immediately adjacent to the cooling surface, which provides the heat-exchange surface. The coolant reservoir may contain a microwave transparent coolant that is driven by a cooling circuit pump and may be circulated through a cooling coil around the reactor vessel, in another embodiment the coil could be substituted by a cooling jacket surrounding the reaction vessel walls. The cooling coil or jacket may provide active cooling to the reaction vessel.

As an example, FIG. **9** or **11** show charts comparing temperature readings inside the reaction vessel, taken during a synthesis cycle, in the presence and absence of the pre-cooling device of the present invention. The three thermal stages are identified. It can be clearly recognized that temperature spikes appear when a liquid is dispensed in the reaction vessel. The dashed curve shows the temperature profile for the device without pre-cooling of the reagents to be supplied. The thermal spikes are remarkable at the pre-coupling regime (subzero temperatures). The solid line depicts the temperature profile with active pre-cooling. The incoming solutions of reagents are effectively pre-cooled and this suppresses the undesired temperature spikes.

Thus, the present invention preferably further relates to a device for automated synthesis of oligo- and polysaccharides on a solid support comprising a reaction vessel, a cooling jacket in thermal communication with the reaction vessel, a reagent storing component, a reagent delivery system in fluid communication with the reaction vessel, a pre-cooling device interposed between the reaction vessel and the reagent delivery system.

The present invention preferably further relates to a device for automated synthesis of oligo- and polysaccharides on a solid support comprising a reaction vessel, a cooling jacket in thermal communication with the reaction vessel and surrounding the reaction vessel, a reagent storing component, a reagent delivery system in fluid communication with the reaction vessel, a pre-cooling device interposed between the reaction vessel and the reagent delivery system.

The present invention preferably further relates to a device for automated synthesis of oligo- and polysaccharides on a solid support comprising a reaction vessel, a cooling jacket in thermal communication with the reaction vessel and surrounding the reaction vessel, a reagent storing component, a reagent delivery system in fluid communication with the reaction vessel through one or more reagents delivery lines, a pre-cooling device interposed between the reaction vessel and the reagent delivery system, the pre-cooling component in thermal communication with the one or more reagents delivery lines.

The present invention preferably further relates to a device for automated synthesis of oligo- and polysaccharides on a solid support comprising a reaction vessel, a cooling jacket in thermal communication with the reaction vessel and surrounding the reaction vessel, the cooling jacket arranged and/or integrated in a cooling circuit, a reagent storing component, a reagent delivery system in fluid communication with the reaction vessel through one or more reagent delivery lines, a pre-cooling device interposed between the reaction vessel and the reagent delivery system, the pre-cooling device in thermal communication with the one or more reagents delivery lines and in thermal communication with the cooling circuit and a computing device comprising at least one processor configured to control one or more components of the device.

Therefore, the present invention preferably relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel, and
(e) a pre-cooling device for pre-cooling the reagents to be supplied in thermal communication with the reagent delivery system.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel,
(d) a cooling device for cooling the reaction vessel, wherein the cooling device comprises a cooling jacket in thermal communication with the reaction vessel, and
(e) a pre-cooling device for pre-cooling the reagents to be supplied.

Therefore, the present invention preferably further relates to a device for automated synthesis of oligo- and polysaccharides on a solid support, the device comprising:
(a) a reaction vessel,
(b) a reagent storing component,
(c) a reagent delivery system in fluid communication with the reaction vessel,
(d) a cooling device for cooling the reaction vessel, wherein the cooling device comprises a cooling jacket in thermal communication with the reaction vessel, and
(e) a pre-cooling device in thermal communication with the reagent delivery system for pre-cooling the reagents to be supplied.

Therefore, the present invention preferably further relates to a device for automated synthesis of oligo- and polysaccharides on a solid support, the device comprising:
(a) a reaction vessel,
(b) a reagent storing component,
(c) a reagent delivery system in fluid communication with the reaction vessel,
(d) a cooling device for cooling the reaction vessel comprising a cooling coil in thermal communication with the reaction vessel, and
(e) a pre-cooling device for pre-cooling the reagents to be supplied.

Therefore, the present invention preferably further relates to a device for automated synthesis of oligo- and polysaccharides on a solid support, the device comprising:
(a) a reaction vessel,
(b) a reagent storing component,
(c) a reagent delivery system in fluid communication with the reaction vessel,
(d) a cooling device for cooling the reaction vessel, wherein the cooling device is a cooling jacket, which is in thermal communication with the reaction vessel, and
(e) a pre-cooling device in thermal communication with the reagent delivery system for pre-cooling the reagents to be supplied,
wherein the cooling device is upstream to the pre-cooling device.

Therefore, the present invention preferably further relates to a device for automated synthesis of oligo- and polysaccharides on a solid support, the device comprising:
(a) a reaction vessel,
(b) a reagent storing component,
(c) a reagent delivery system in fluid communication with the reaction vessel,
(d) a cooling device comprising a cooling jacket in thermal communication with the reaction vessel for maintaining the temperature of the reaction vessel during one or more synthesis steps of the automated synthesis cycle,
(e) a pre-cooling device interposed between the reaction vessel and the reagent delivery system for control of the temperature of the one or more reagents before entering the reaction vessel.

Therefore, the present invention preferably further relates to a device for automated synthesis of oligo- and polysaccharides on a solid support, the device comprising:
(a) a reaction vessel,
(b) a reagent storing component,
(c) a reagent delivery system in fluid communication with the reaction vessel through one or more reagent delivery lines,
(d) a cooling device comprising a cooling jacket in thermal communication with the reaction vessel,
(e) a pre-cooling device in thermal communication with the one or more reagent delivery lines.

Therefore, the present invention preferably relates to a device for automated synthesis of oligo- and polysaccharides on a solid support, the device comprising:
(a) a reaction vessel,
(b) a reagent storing component,
(c) a reagent delivery system comprising:
   a building block distribution component connected to the reaction vessel through a building block delivery line,
   an activator distribution component connected to the reaction vessel through an activator delivery line,
(d) a cooling device comprising a cooling jacket in thermal communication with the reaction vessel,
(e) a pre-cooling device in thermal communication with the activator delivery line and the building block delivery line.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel via one or more reagent delivery lines,
(d) a cooling device comprising a cooling jacket in thermal communication with the reaction vessel,
(e) a pre-cooling device in thermal communication with the one or more reagent delivery lines.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel through one or more reagent delivery lines,
(d) a cooling device for cooling the reaction vessel,
(e) a pre-cooling device in thermal communication with the one or more reagent delivery lines and in thermal communication with the cooling device.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel through one or more reagent delivery lines,
(d) a cooling device comprising a cooling circuit comprising a cooling jacket in thermal communication with the reaction vessel,
(e) a pre-cooling device in thermal communication with the one or more reagent delivery lines and in thermal communication with the cooling circuit comprising the cooling jacket.

The device for automated synthesis of oligo- or polysaccharide may further comprise a **microwave generator component.** Microwave-assisted organic chemical synthesis refers to the use of electromagnetic radiation within the microwave frequencies to provide the energy required to initiate, drive or accelerate certain chemical reactions. Several multimode or mono-mode / single-mode microwave generator components for microwave-assisted organic synthesis are known in the art, for example, in multimode instruments microwaves are reflected by the walls of a relatively large cavity, which may generate pockets of high and low energy as the moving waves either reinforce (hot spots) or cancel out each other (cold spots) leading to a non-homogeneous microwave field in the cavity. In order to provide a homogeneous field, multimode instruments may be equipped with a mechanical mode stirrer inside the cavity. In mono-mode cavities a standing wave may be created when the electromagnetic irradiation is passed by a waveguide that directs the microwaves directly through a reaction vessel that is positioned in a fixed distance from the radiation source or by generating travelling waves where the microwaves make a single pass through the reaction vessel, are reflected off the cavity wall and pass through the reaction vessel a second time.. Multimode microwave components enable performance of parallel synthesis, whereas mono-mode microwave components provide a high degree of reproducibility due to a very precise heating. The microwave generator component may be one of a magnetron, klystron and solid state devices. A solid-state microwave generator component uses a semiconductor transistor which generates single, defined microwave frequencies in a controlled manner.

Thus, in preferred embodiments of the present invention the device may further comprise a microwave generator component, preferably a mono-mode / single-mode microwave generator component. The microwave generator component may further comprise a waveguide. The microwave generator component preferably comprises a chamber for insertion and fixing of the reaction vessel to provide microwave radiation to the reaction vessel.

In comparison to conductive heating microwave heating (dielectric heating at 2.45 GHz) occurs by disposing the energy directly to the solvent and some reagents, due to interactions of the solvents and reagents with the alternating electric field. Material interacts with the electromagnetic field differently, i.e. materials store and convert the energy to heat to different extents, which have huge impact on their ability to be heated by microwaves. Besides the solvent, several other factors influence the dielectric properties, such as sample volume, vessel material, and the mode of stirring. The application of heat energy (thermal transfer) is one of the most significant factors in increasing the rate of a wide variety of chemical reactions. Microwave-assisted reactions, however, transfer energy to chemical reactions in a different, much faster manner than the conductive devices. Microwave energy directly interacts with polar or ionic molecules. This, effect, known as dipole rotation, is a result of the polar or ionic molecules trying to align themselves with the rapidly changing electric field of the microwaves. The rotational movement of molecules as they try to orient themselves with the electric field creates localized superheating and generates thermal energy as a by-product.

Thermal energy may be raised beyond a critical point for a given reaction, resulting in excess thermal energy. Excess thermal energy increases the temperature of a reaction mixture. This excess thermal energy may have detrimental effects on heat-sensitive reactions or compositions. Excess thermal energy can drive side reactions that degrade the reactants, catalysts and desired product of the desired reaction. Furthermore, some products may be unstable at room temperature. Therefore it is advantageous to maintain a low bulk reaction mixture temperature. The beneficial effects of simultaneous cooling during microwave irradiation have been described in the art to be useful for chemical synthesis with solid phase supports.

The inventors of the present invention have found that the utilization of a microwave generator component is not only instrumental for hastening the cooling to heating process, microwave-assisted synthesis has also been shown to drastically reduce chemical reaction time. During a typical AGA cycle there is the glycosylation coupling step as well as several auxiliary steps (acetyl capping and temporary group deprotection). These auxiliary steps increase the overall yield of the final glycan by terminating unglycosylated nucleophiles as well as they remove temporary protecting groups that allows the next coupling to occur. These auxiliary steps have been a bottleneck in the overall time required for one AGA cycle and the use of microwave radiation drastically reduces the reaction time. Under these rapid microwave-assisted conditions, the steps remained orthogonal and few side reactions were observed. With shortened reaction times for these auxiliary steps, the overall duration of a standard AGA cycle could be successfully reduced from 100 minutes to below 60 minutes and even to 45 minutes. Thus, the auxiliary steps carried out in the automated saccharide synthesis benefit from the use of microwave radiation.

A solid support or solid phase resin which is stable under microwave conditions or microwave irradiation is particularly preferred with regard to embodiments of the device of the present invention further comprising a microwave generator component. Such a microwave-stable solid support or solid phase resin is particularly suitable for performing microwave-assisted synthesis of oligo- and polysaccharides on a solid support. Therefore, it is particularly preferred that no decomposition of the solid phase resin occurs during the microwave-assisted synthesis steps of the synthesis cycles. In other words a solid support material is preferred which is stable under microwave conditions and also a linker is preferred which is stable under microwave conditions. Thus, a solid phase resin functionalized with an appropriate linker which is stable under microwave conditions is particularly preferred, which also means that no decoupling from the solid phase resin occurs during the microwave-assisted synthesis steps of the synthesis cycles.

In embodiments of the device of the present invention further comprising a microwave generator component it is particularly preferred that the reaction vessel is made of a microwave transparent material. Examples of microwave transparent materials include, but are not limited to, glass, quartz, PTFE (Teflon), perfluoroalkoxy alkane (PFA), polypropylene. A microwave transparent reaction vessel is particularly preferred in embodiments, wherein the device of the present invention further comprises a microwave generator component and the synthesis cycles comprise one or more microwave-assisted synthesis steps performed inside of the reaction vessel.

It is preferred that the microwave transparent reaction vessel further comprises at least one opening or aperture for insertion of a microwave compatible temperature sensor for monitoring and measuring the temperature inside of the reaction vessel. Thus, it is preferred that the temperature sensor is a microwave-compatible temperature sensor and suitable for monitoring and measuring the temperature inside of the reaction vessel during microwave-assisted synthesis steps.

The inventors have found that microwave-assisted synthesis steps advantageously reduce the overall period of time of the overall synthesis of the desired oligo- or polysaccharides, which was also shown to increase the yield of the desired oligo- or polysaccharide.

The cooling device may be a microwave transparent heating and/or cooling device equipped with a microwave transparent temperature sensor or thermometer, wherein the cooling device temperature may be adjusted either manually or preferably by a computing device. For example, the cooling device could be a heating bath, an external refrigerated circulator or a heating/cooling block. The heating/cooling block may be made of any microwave transparent heat transfer material known in the art. The block may have channels running through to pass microwave transparent coolant fluid through. The microwave transparent reaction vessel may be placed in a cavity of the microwave transparent heating/cooling block. In a preferred embodiment of the present invention a microwave transparent coolant fluid may be circulated around the microwave transparent reaction vessel via a microwave transparent sleeve surrounding the microwave transparent reaction vessel. In preferred embodiments the cooling device may comprise a microwave transparent cooling jacket. In preferred embodiments the microwave transparent cooling jacket may be in form of a microwave transparent cooling coil surrounding the microwave transparent reaction vessel. The microwave transparent cooling jacket is preferably in thermal communication with the microwave transparent reaction vessel. The cooling coil or cooling jacket may be further provided with a microwave transparent thermal isolation cover to cover the cooling jacket or cooling coil from exposure to the chamber to increase the heat exchange efficiency of the cooling jacket or cooling coil.

The cooling device may be connected to a coolant fluid reservoir. The cooling device may be connected to a cooling circuit pump. In preferred embodiments of the present invention the microwave transparent cooling jacket surrounding the microwave transparent reaction vessel may be connected to a coolant fluid reservoir and a coolant circuit pump. The coolant fluid reservoir, the coolant circuit pump and the microwave transparent cooling jacket surrounding the microwave transparent reaction vessel may form a closed cooling circuit. The microwave transparent cooling jacket may be provided in form of a cooling coil surrounding the microwave transparent reaction vessel. The coolant circuit pump may be under control of a computer comprising at least one processor. The cooling device may comprise a cooling unit configured to control the temperature of the cooling device. The cooling unit may be electronically coupled to a computing device comprising at least one processor. The cooling circuit pump may be electronically coupled to a computing device comprising at least one processor to control the flow of the coolant fluid through the cooling circuit.

The present invention relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component.

The present invention further relates to a device for automated synthesis of oligo- and polysaccharides on a solid support, the device comprising:
(a) a microwave transparent reaction vessel adapted for receiving the solid support,
(b) a reagent storing component,
(c) a reagent delivery system,
(d) a cooling device for cooling the reaction vessel,
(e) and a pre-cooling device for pre-cooling the reagents to be supplied,
(f) a microwave generator component.

Therefore the present invention preferably relates to a device for automated synthesis of oligo- and polysaccharides on a solid support, the device comprising:
(a) an interchangeable microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component.

Therefore the present invention preferably relates to a device for automated synthesis of oligo- and polysaccharides on a solid support, the device comprising:
(a) an interchangeable microwave transparent reaction vessel adapted for receiving the solid support,
(b) a reagent storing component,
(c) a reagent delivery system,
(d) a cooling device for cooling the reaction vessel,
(e) and a pre-cooling device for pre-cooling the reagents to be supplied,
(f) a microwave generator component.

Therefore the present invention preferably further relates to a device for automated synthesis of oligo- and polysaccharides on a solid support, the device comprising:
(a) a microwave transparent reaction vessel made of perfluoroalkoxy alkanes (PFA),
(b) a reagent storing component,
(c) a reagent delivery system,
(d) a cooling device for cooling the reaction vessel,
(e) and a pre-cooling device for pre-cooling the reagents to be supplied,
(f) a microwave generator component.

The present invention preferably further relates to a device for automated synthesis of oligo- and polysaccharides on a solid support, the device comprising:
(a) a microwave transparent reaction vessel,
(b) a reagent storing component for storing building blocks and/or building block solutions, activators and/or activator solutions, washing solutions and/or washing solvents and deprotection solutions,
(c) a reagent delivery system,
(d) a cooling device for cooling the reaction vessel,
(e) and a pre-cooling device for pre-cooling the reagents to be supplied,
(f) a microwave generator component.

The present invention preferably further relates to a device for automated synthesis of oligo- and polysaccharides on a solid support, the device comprising:
(a) a microwave transparent reaction vessel,
(b) a reagent storing component,
(c) a reagent delivery system for delivery of buildings blocks and/or building block solutions, activators and/or activator solutions, washing solvents and/or washing solutions and deprotection solutions,
(d) a cooling device for cooling the reaction vessel,
(e) and a pre-cooling device for pre-cooling the reagents to be supplied,
(f) a microwave generator component.

The present invention further relates to a device for automated synthesis of oligo- and polysaccharides on a solid support, the device comprising:
(a) a microwave transparent reaction vessel,
(b) a reagent storing component for storing building blocks and/or building block solutions, activators and/or activator solutions, washing solutions and/or washing solvents and deprotection solutions,
(c) a reagent delivery system for delivery of buildings blocks and/or building block solutions, activators and/or activator solutions, washing solvents and/or washing solutions and deprotection solutions,
(d) a cooling device for cooling the reaction vessel,
(e) and a pre-cooling device for pre-cooling the reagents to be supplied,
(f) a microwave generator component.

The present invention preferably relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied, and
(f) a microwave generator component,
wherein the reagent delivery system connects the reagent storing component with the reaction vessel.

The present invention preferably relates to a device for automated synthesis of oligo- and polysaccharides on a solid support, the device comprising:
(a) a microwave transparent reaction vessel,
(b) a reagent storing component,
(c) a reagent delivery system comprising a first reagent distribution component and a second reagent distribution component,
(d) a cooling device for cooling the reaction vessel,
(e) and a pre-cooling device for pre-cooling the reagents to be supplied,
(f) a microwave generator component.

The present invention preferably relates to a device for automated synthesis of oligo- and polysaccharides on a solid support, the device comprising:
(a) a microwave transparent reaction vessel,
(b) a reagent storing component,
(c) a reagent delivery system comprising a first reagent distribution component for delivery of washing solvents, building blocks and/or building block solutions and activators and/or activator solutions and a second reagent distribution component for delivery of deprotection solutions,
(d) a cooling device for cooling the reaction vessel,
(e) and a pre-cooling device for pre-cooling the reagents to be supplied,
(f) a microwave generator component.

The present invention preferably relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel,
(d) a cooling device for cooling the reaction vessel, wherein the cooling device comprises a microwave transparent cooling jacket in thermal communication with the reaction vessel, and
(e) a pre-cooling device for pre-cooling the reagents to be supplied,
(f) a microwave generator component.

The present invention preferably further relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel,
(d) a cooling device for cooling the reaction vessel, wherein the cooling device comprises a microwave transparent cooling jacket in thermal communication with the reaction vessel, and
(e) a pre-cooling device in thermal communication with the reagent delivery system for pre-cooling the reagents to be supplied,
(f) a microwave generator component.

The present invention preferably further relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel,
(d) a cooling device for cooling the reaction vessel comprising a microwave transparent cooling coil in thermal communication with the reaction vessel, and
(e) a pre-cooling device for pre-cooling the reagents to be supplied,
(f) a microwave generator component.

The present invention preferably further relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel,
(d) a cooling device for cooling the reaction vessel, wherein the cooling device is a microwave transparent cooling jacket, which is in thermal communication with the reaction vessel, and
(e) a pre-cooling device in thermal communication with the reagent delivery system for pre-cooling the reagents to be supplied,
(f) a microwave generator component,
wherein the cooling device is upstream to the pre-cooling device.

The present invention preferably further relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel,
(d) a cooling device comprising a microwave transparent cooling jacket in thermal communication with the reaction vessel for maintaining the temperature of the reaction vessel during one or more synthesis steps of the automated synthesis cycle,
(e) a pre-cooling device interposed between the reaction vessel and the reagent delivery system for control of the temperature of the one or more reagents before entering the reaction vessel.
(f) a microwave generator component.

The present invention preferably further relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel through one or more reagent delivery lines,
(d) a cooling device comprising a microwave transparent cooling jacket in thermal communication with the reaction vessel,
(e) a pre-cooling device in thermal communication with the one or more reagent delivery lines,
(f) a microwave generator component.

The present invention preferably relates to a device for automated synthesis of oligo- and polysaccharides on a solid support, the device comprising:
(a) a microwave transparent reaction vessel,
(b) a reagent storing component,
(c) a reagent delivery system comprising:
   a building block distribution component connected to the reaction vessel through a building block delivery line,
   an activator distribution component connected to the reaction vessel through an activator delivery line,
(d) a cooling device comprising a microwave transparent cooling jacket in thermal communication with the reaction vessel,
(e) a pre-cooling device in thermal communication with the activator delivery line and the building block delivery line,
(f) a microwave generator component.

Therefore, the present invention preferably relates to a device for automated synthesis of oligo- and polysaccharides on a solid support, the device comprising:
(a) a microwave transparent reaction vessel,
(b) a reagent storing component,
(c) a reagent delivery system in fluid communication with the reaction vessel via one or more reagent delivery lines,
(d) a cooling device comprising a microwave transparent cooling jacket in thermal communication with the reaction vessel,
(e) a pre-cooling device in thermal communication with the one or more reagent delivery lines,
(f) a microwave generator component.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel through one or more reagent delivery lines,
(d) a cooling device for cooling the reaction vessel,
(e) a pre-cooling device in thermal communication with the one or more reagent delivery lines and in thermal communication with the cooling device,
(f) a microwave generator component.

Therefore, the present invention preferably relates to a device for automated synthesis of oligo- and polysaccharides on a solid support, the device comprising:
(a) a reaction vessel,
(b) a reagent storing component,
(c) a reagent delivery system in fluid communication with the reaction vessel through one or more reagent delivery lines,
(d) a cooling device comprising a cooling circuit comprising a cooling jacket in thermal communication with the reaction vessel,
(e) a pre-cooling device in thermal communication with the one or more reagent delivery lines and in thermal communication with the cooling circuit comprising the cooling jacket,
(f) a microwave generator component.

The present invention preferably relates to a device for automated synthesis of oligo- and polysaccharides on a solid support in the range of 12.5 - 200 pmol per synthesis.

### Continuous flow process

In another embodiment of the present invention the reaction vessel may be provided as a flow reaction vessel to allow automated synthesis of oligo- and polysaccharides on a solid support in a continuous flow process. The reaction vessel may be provided in form of a tube reaction vessel or preferably may be provided as a packed-bed reaction vessel or column reaction vessel which allows heterogeneous transformations where reagents are on solid support.

Thus, in one embodiment the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is a flow reaction vessel.

The device may also comprise a microwave generator component. The flow reaction vessel may be made for example of glass, borosilicate, Teflon, fluoropolymer or SiC. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a microwave transparent flow reaction vessel.

Thus, the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is a packed-bed reaction vessel.

The device may also comprise a microwave generator component. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a microwave transparent packed-bed reaction vessel.

For performing the coupling reactions appropriate building blocks and activators may be provided in a continuous flow through the flow reaction vessel. Preferably, the building blocks and activators may be provided in a continuous flow through the flow reaction vessel already containing the solid support or solid phase resin. In such a case the flow reaction vessel is preferably a packed-bed reaction vessel. The coupling reactions preferably take place inside the flow reaction vessel followed by appropriate deprotection, capping and/or washing steps. All of these synthesis steps and treatments in the synthesis cycles are preferably performed inside of the flow reaction vessel of the device of the present invention and the reagents and/or solution and/or solvents are provided in a continuous flow through the flow reaction vessel. The synthesis cycles are repeated as often as necessary to achieve the desired length of the desired oligo- or polysaccharide. The synthesis cycle is preferably repeated at least three times, more preferably at least four times or more preferably more than four times for obtaining an oligo- or polysaccharide.

The reagent delivery system may be connected to the flow reaction vessel through one or more inlets of the flow reaction vessel for delivery of the reagents and/or reagents solution and/or solvents in a continuous flow through the continuous flow reaction vessel. The reagent delivery system may be connected to the flow reaction vessel through one or more reagent delivery lines. Thus, the reagent delivery system may be adapted to deliver reagents and/or reagent solutions and/or solutions and/or solvents in a continuous flow through the flow reaction vessel and may be further adapted to supply the reagents and/or reagent solution and/or solutions or solvents through the one or more inlets of the flow reaction vessel. Preferably the reagent delivery system may be adapted to deliver one or more reagents and/or one or more reagent solutions and/or one or more solutions in different amounts, at different points in time and in a specific sequence or specific order, and at a specific flow rate or different flow rates and further through specific inlets of the one or more inlets of the flow reaction vessel during the synthesis cycles of the automated synthesis of oligo- and polysaccharides on a solid support. It is therefore preferred that the reagent delivery system comprises suitable technical means, preferably suitable technical means electronically coupled to a computing device comprising at least one processor, for delivery of one or more reagents and/or one or more reagent solutions and/or one or more solutions in different amounts, at different points in time and in a specific sequence or specific order and at a specific flow rate or at different flow rates during the synthesis cycles of the automated synthesis of oligo- and polysaccharides on solid support. The reagent delivery system may comprise a pump system or one or more pumps such as syringe pumps, peristaltic pumps or other suitable pumps and may further comprise one or more valves or one or more valve assemblies, one or more manifolds, one or more distributing components and similar suitable technical means. It is preferred that the pump system or the one or more pumps, the one or more valves or the one or more valve assemblies, the one or more manifolds and/or the one or more distributing components and similar technical means are under control of a computing device comprising at least one processor.

In a further embodiment only specific reaction steps of the synthesis cycle may be performed in a continuous flow process. Thus, the automated synthesis of oligo- and polysaccharides on a solid support may be performed in a semi-continuous flow process. For example, the glycosylation reaction or coupling reaction may be performed without continuous flow of the reagents through the reactor, whereas the deprotection steps and/or capping steps may be performed in a continuous flow process. In such embodiments the building blocks and activators may be provided to the flow reaction vessel without simultaneous discharging the reagents to be supplied or without recirculating the reagents to be supplied. Thus, in the pre-coupling regime the building blocks and activators are allowed to impregnate the resin and to diffuse through the porous solid. As mentioned above the flow reaction vessel may be connected to one or more valves or one or more valve assemblies, one or more manifolds, one or more distributing components and similar suitable technical means. The one or more valves or one or more valve assemblies, one or more manifolds, one or more distributing components and similar suitable technical means may be adapted to hold the reagents, solvents or solutions inside the flow reaction vessel and may be further adapted to discharge the reagents, solvents or solutions from the flow reaction vessel and may even be adapted to allow recirculation of the reagents, solvents or solutions through the flow reaction vessel. Therefore, the flow reaction vessel may be operated in a continuous flow mode or may be operated in a non-continuous flow mode. Thus, the flow reactor may be switchable between a continuous flow mode and a non-continuous flow mode. Thus, the flow reactor may be switchable between a continuous flow mode and a semi-batch or batch mode. The modes may be switched via one or more valves or one or more valve assemblies or one or more manifolds and/or one or more distributing components or similar technical means.

In a further embodiment the flow reactor may comprise a frit or a filter to prevent canalization of fluid through. As already described above a frit allows for dispersion of inert gas bubbling for mixing purposes and the frit may also ensure that the solid support remains inside of the reaction vessel. Thus, in preferred embodiments of the present invention the flow reaction vessel may further comprise a frit or a filter. It is preferred that the frit is located in the bottom compartment of the flow reaction vessel. Thus, it is preferred that the frit is located close to the outlet where the flow of solvents and solutions leave the flow reaction vessel. Thus, to prevent solid support from being drawn from the bottom inlet, the end-part in the flow reaction vessel may be fitted with a frit or a filter. In a further embodiment a frit or filter may be used which only allows passage or through-flow of solvents and solutions through the frit or filter by applying an elevated pressure from the top or reduced pressure at the bottom of the frit or filter. For example the continuous flow of a solvent or solution may be achieved in that the solvent or solution is supplied under elevated pressure to the flow reaction vessel. In such embodiments the solvent or solution may pass the frit or filter to allow continuous flow of the solvent or solution through the flow reaction vessel. In another example the solvent or solution may be applied without elevated pressure or only at slightly elevated pressure such that the solvent or solution may not pass the frit or filter. In such a case the flow reaction vessel may be filled with the solvent and solution, for example, building blocks or activators, and in this example the building blocks and/or activators may impregnate the solid phase resin as it is beneficial in the pre-coupling stage as already described above. In such embodiment it is further preferred that bubbling gas for agitating the reaction mixture, such as inert gas is provided to the flow reaction vessel.

Thus, the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is a flow reaction vessel, wherein the flow reaction vessel is switchable between a continuous flow mode and a semi-batch mode.

The device may also comprise a microwave generator component. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a microwave transparent flow reaction vessel, wherein the microwave transparent flow reaction vessel is switchable between a continuous flow mode and a semi-batch mode.

Thus, the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is flow reaction vessel, wherein the flow reaction vessel is switchable between a continuous flow mode and a semi-batch or batch mode via one or more valves or one or more valve assemblies or one or more manifolds and/or one or more distributing components or similar technical means.

The device may also comprise a microwave generator component. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a flow reaction vessel, wherein the microwave transparent flow reaction vessel is switchable between a continuous flow mode and a semi-batch mode via one or more valves or one or more valve assemblies or one or more manifolds and/or one or more distributing components or similar technical means.

Thus, the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is a flow reaction vessel, wherein the flow reaction vessel comprises a frit, wherein the flow of solvent and/or solution through the frit is pressure-dependent.

The device may also comprise a microwave generator component. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a flow reaction vessel, wherein the microwave transparent flow reactor comprises a frit, wherein the flow of solvent and/or solution through the frit is pressure-dependent.

In a further embodiment the continuous flow process may be performed by recirculating the reagents, solvents and/or solutions through the flow reaction vessel. The flow reaction vessel may connected to itself via one or more valves, one or more valve assemblies, one or more manifolds and/or the one, one or more distributing components and similar technical means so that a flow can pass the flow reaction vessel repeated times. Thus, one or more outlets (or bottom inlets) of the flow reaction vessel may be connected through one or more valves, one or more valve assemblies, one or more manifolds, one or more distributing components and/or similar suitable technical means with one or more inlets of the flow reaction vessel. Preferably one or more inlets at the bottom of the flow reaction vessel are connected with one or more inlets at the top of the flow reaction vessel via one or more valves, one or more valve assemblies, one or more manifolds and/or the one, one or more distributing components and similar technical means so that a flow can pass the flow reaction vessel repeated times. For example the deprotection reagents and/or capping reagents may recirculate through the flow reaction vessel. In a further example all reagents, solvents and solutions may recirculate through the flow reaction vessel. In case of building blocks and activators it is preferred that the building blocks or building block solution and the activators or activator solution are mixed or merged before passing through the flow reaction vessel. The building blocks or building block solution and the activators or activator solutions may be mixed or merged directly at the time of supplying the building blocks or building block solution and the activators or activator solution to the flow reaction vessel.

Thus, the present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
wherein the reaction vessel is a flow reaction vessel and wherein the flow reaction vessel is connected to itself via one or more valves or one or more valve assemblies or one or more manifolds and/or one or more distributing components or similar technical means so that a flow can pass the flow reaction vessel repeated times.

The device may also comprise a microwave generator component. Thus, the present invention further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the reaction vessel,
(e) and a pre-cooling device for pre-cooling the reagents to be supplied,
(f) a microwave generator component,
wherein the microwave transparent reaction vessel is a microwave transparent flow reaction vessel and wherein the microwave transparent flow reaction vessel is connected to itself via one or more valves or one or more valve assemblies or one or more manifolds and/or one or more distributing components or similar technical means so that a flow can pass the microwave transparent flow reaction vessel repeated times.

The device of the present invention may further comprise a **computing device** comprising at least one processor and a computer-readable storage medium comprising computer readable instructions that may be executed by the processor. The processor may be configured to control the temperature setting of the pre-cooling device, may be configured to control the temperature setting of the cooling device, and may be configured to set the time, temperature and power of the microwave generator component, may be configured to control the amount and speed of delivering fluid from the reagent delivery system. The device of the present invention preferably comprises one or more or a plurality of technical means electronically coupled to the computing device comprising at least one processor to allow the processor to control the one or more or plurality of technical means based on computer readable instructions stored on a computer readable medium which may be executed by the processor to enable the automation of the synthesis of oligo- and polysaccharides on a solid support with the device of the present invention. The one or more technical means or plurality of technical means may be wired or wirelessly connected to the computing device. The one or more technical means or plurality of technical means may comprise one or more valves, valve assemblies, one or more vents and similar technical means of the one or more components and systems of the device of the present invention. The device of the present invention may further comprise one or more temperature monitoring means to allow temperature control of the cooling device and/or pre-cooling device and to allow temperature control of the reaction mixture inside of the reaction vessel. The computing device comprising the at least one processor may be configured to receive the temperature monitoring data from the cooling device, pre-cooling device and from one or more temperature monitoring means such as a temperature sensor or temperature probe. Based on the received temperature monitoring data the processor may be configured to adjust the cooling device and/or pre-cooling device to maintain a temperature inside of the reaction vessel.

Thus, the present invention preferably further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
(f) a computing device comprising at least one processor configured to control one or more components of the device.

The present invention preferably further relates to a device for automated synthesis of oligo- and polysaccharides on a solid support, the device comprising:
(a) a microwave transparent reaction vessel,
(b) a reagent storing component,
(c) a reagent delivery system,
(d) a cooling device for cooling the microwave transparent reaction vessel,
(e) and a pre-cooling device for pre-cooling the reagents to be supplied,
(f) a computing device comprising at least one processor configured to control one or more components of the device,
(g) a microwave generator component.

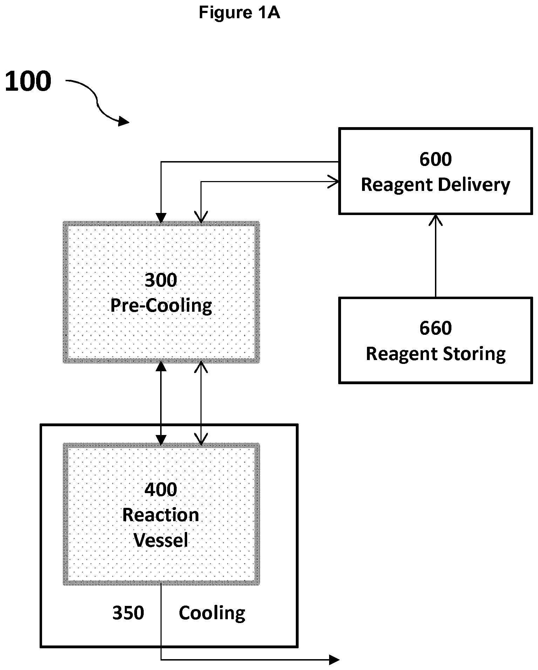

FIG. **1A-C** is a block diagram illustrating several examples of the inventive device for automated oligo- and polysaccharide synthesis on solid support.

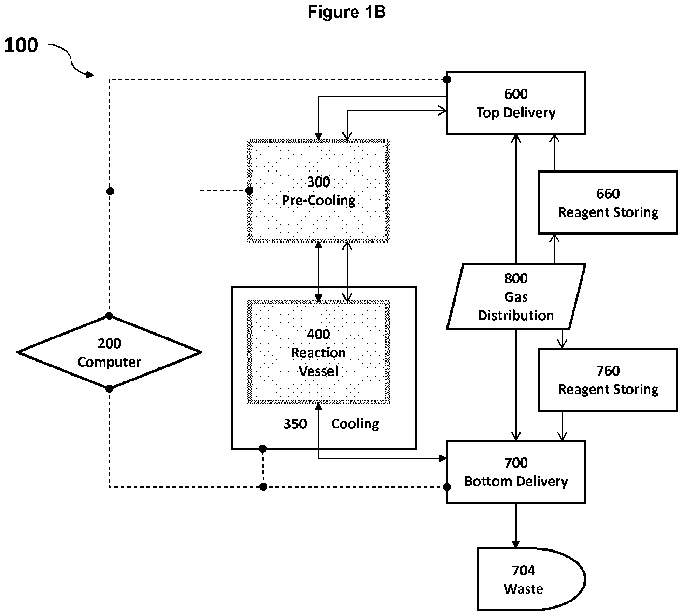

In Fig **1B**, two reagent delivery systems drive the flow of fluids to the reaction vessel **400.** The top delivery system **600** supplies the washing solvents, building blocks, and activating solutions to the pre-cooling component **300**, as well as a ventilation exit for the inert gas coming out of the reaction vessel **400.** The top delivery system **600** receives the washing solvents, building blocks and activating solutions from a reagent storing device **660.** The bottom delivery system **700** provides the deprotection reagent solutions, capping reagent solution and bubbling gas, which is used for mixing and creating an inert and anhydrous atmosphere inside the reaction vessel **400**, as well discharges the liquids after the reaction. The bottom delivery system **700** receives the deprotection reagent solutions and capping reagent solution from a reagent storing component **760.**

By splitting the reagent delivery system in a top and a bottom delivery system optimizes the number and the use of driving fluid components. The top delivery system feeds precisely the coupling reagents for instance by using syringe pump, while washing solvents and evacuating anhydrous gas are driven from the top for instance by gas differential pressure. The bottom delivery system is driven by gas differential pressure and directs the different deprotection and capping reagents into the reaction vessel **400.** This prevents potential mixing of incompatible chemicals (e.g. acids and bases) used at different steps of the synthesis program, and economizes the number of mechanical fluid driving devices.
An inert gas delivery system **800** provides anhydrous and inert gas to the device for mixing, atmosphere conditioning and driving fluids purposes.

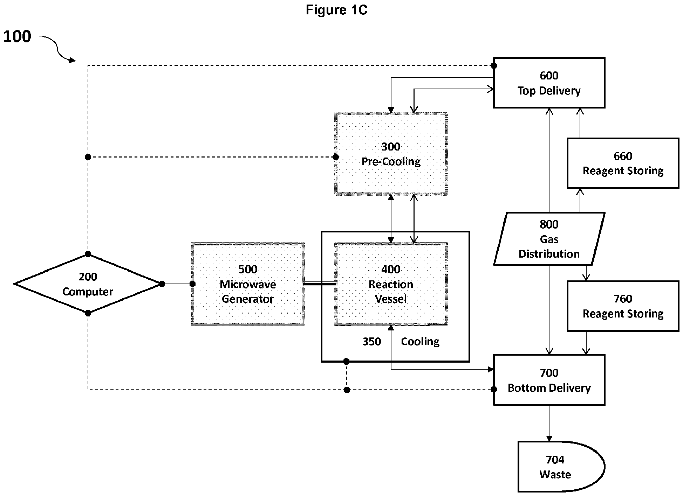

In the embodiment shown in Fig. **1C**, the reaction vessel **400** is fitted inside a microwave generator component **500**, which provides the microwave radiation in accordance to a further embodiment of the present invention.

The process is automated and controlled by a computing device comprising at least one processor **200.** The processor controls the execution of commands, which adjusts the temperature of pre-cooling component **300;** sets the time, temperature and power of the microwave generator component **500;** controls the amount and speed of delivering fluid from the top delivery system **600** and bottom delivery system **700.**

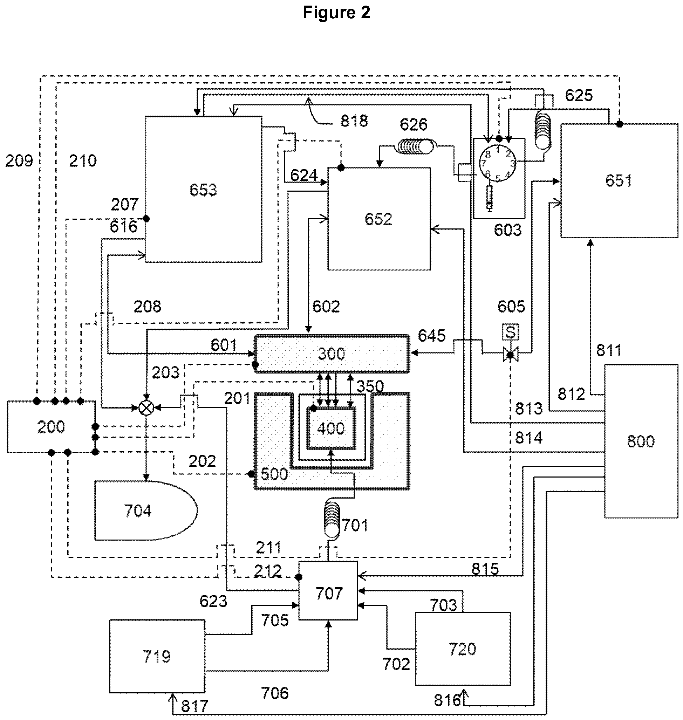

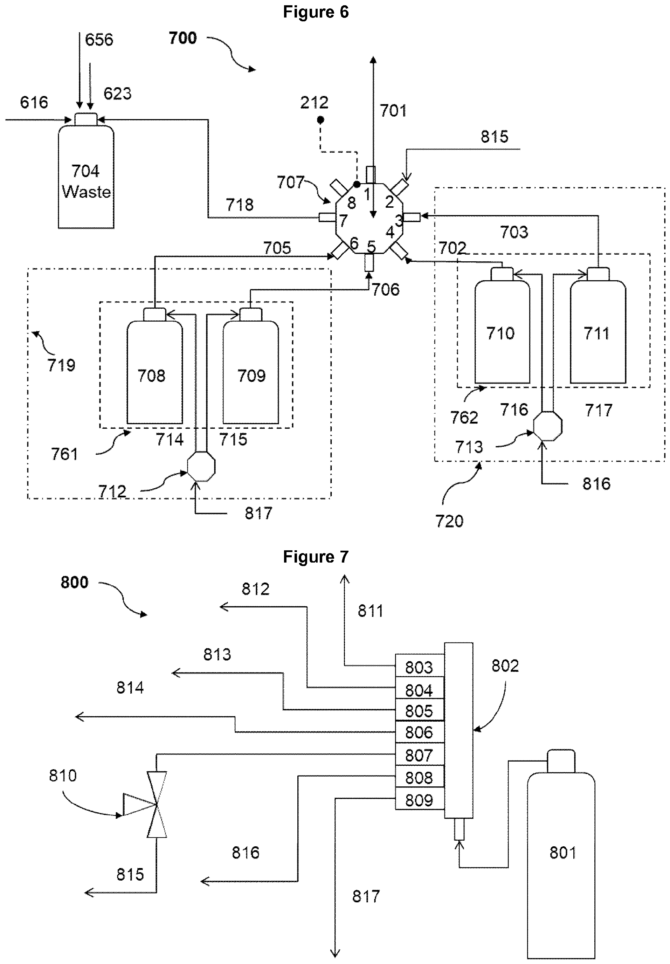

FIG. **2** is a flow diagram illustrating the liquid and gas distribution components of FIG. **1A-C** in more detail. The top delivery system **600** includes the building blocks distribution component **653** and the activator distribution component **652.** The syringe pump **603** drives the fluids within the top delivery system **600.** The loop lines **625** and **626** allow for careful delivery of sensitive and/or corrosive chemical solutions. The resting volume of the loops is defined as a function of the tubing length. From the washing solvents component **651**, a driving solvent is taken to fill up the loops ahead the withdrawing of chemical solutions from **652** and **653.** This avoids the direct contact of the reagents with the syringe pump **603**, which prevents the pump deterioration and cross-contamination. The bottom delivery system **700** includes the multi-port valve **707**, the deprotection distribution component **719**, the capping distribution component **720**, and the waste collector **704.** The loop line **701** allows for the delivery of chemical solutions from the deprotection storing component **761** (shown in Fig. 6) and capping storing component **762** (shown in Fig. 6) via components **719** and **720**, and bubbling gas from the inert gas delivery system **800.** The loop line 701 allows as well the discharge of waste liquids from the reaction vessel **400.**

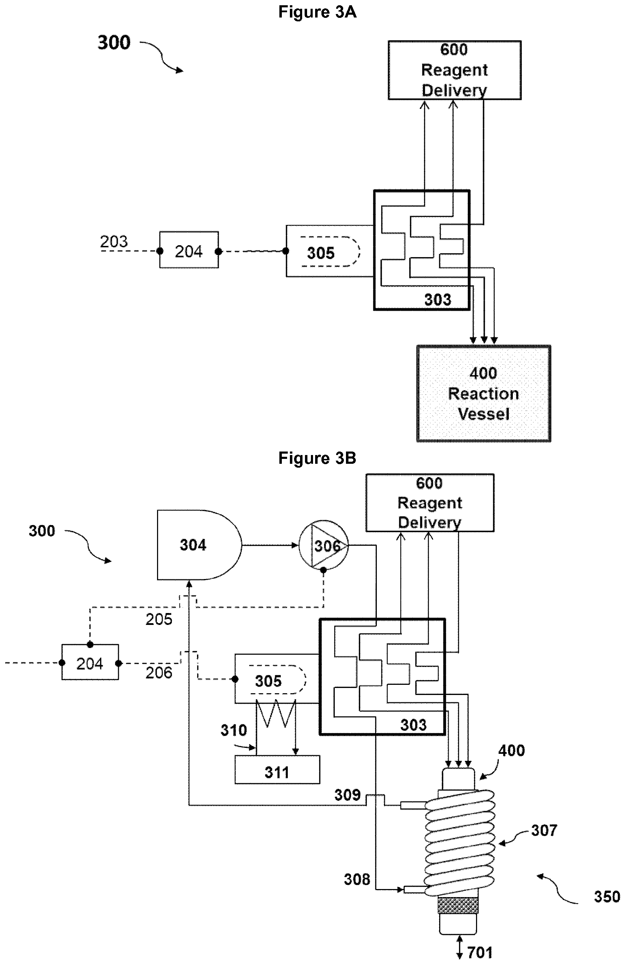

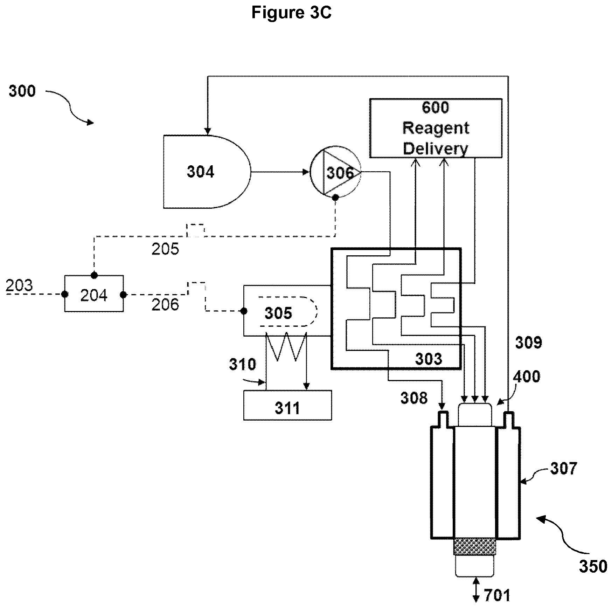

FIG. **3A** is a conceptual diagram illustrating a side view of an example implementation of a pre-cooling device **300.** Before the inlet to the reactor vessel **400**, the building block delivery line **601** and activator solution delivery line **602** contact a metal cooling surface **303** at the set temperature. The washing solvents delivered by the line **645** could be pre-cooled as well by the action of the Peltier cooler **305.**

FIG. **3B** is a conceptual diagram illustrating a side view of an example implementation of a compact pre-cooling device **300** and cooling device **350.** A coolant fluid reservoir **304** is immediately adjacent to the cooling surface **303**, which provides the heat-exchange material. A Peltier cooler (thermoelectric) contact-cooling device **305** provides the means to reach the set temperature. The thermoelectric contact-cooling device **305** may be assisted by a line of coolant fluid **310**, such as for example cooling water to cool down the warm side of the thermoelectric contact-cooling device **305.** The cooling water line **310** may be provided by a central cooling system or a compact commercial cooling unit **311.** The coolant reservoir **304** contains a microwave transparent coolant that is driven by a cooling circuit pump **306** and circulated through a cooling jacket or cooling coil **307.** The cooling jacket or cooling coil **307** provides active cooling to the reaction vessel **400.** The coolant gets in the cooling jacket or cooling coil **307** by the line **308** and exits through the line **309** to the coolant reservoir **304.** The connection line **203** communicates the commands from the computing device comprising at least one processor **200** to a signal processor **204** that integrates and delivers actions by electronic signal **205** to the pump **306**, and electronic signal **206** to the Peltier cooler **305.**

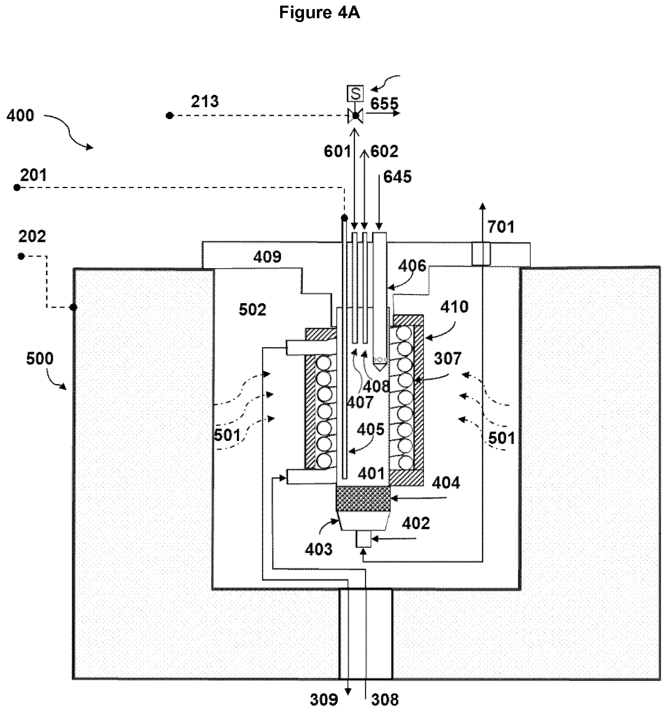

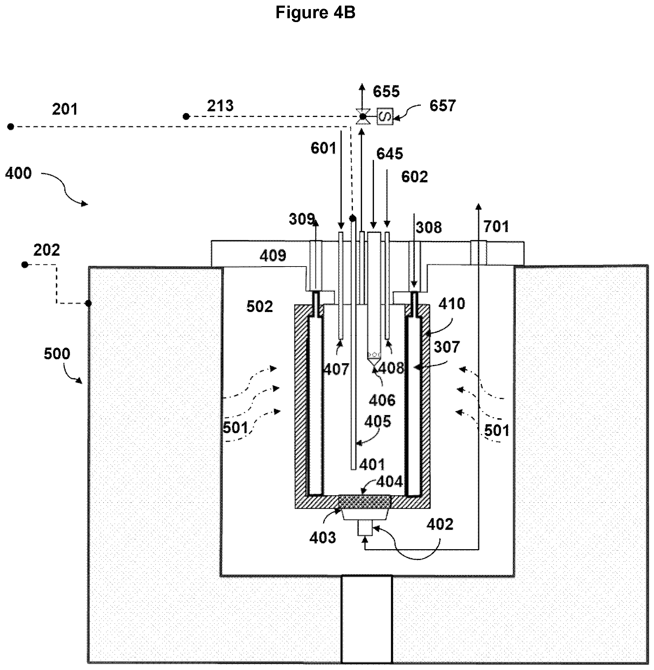

FIG. **4** is a conceptual diagram illustrating a cross-section view of the reaction vessel **400** in an embodiment of the inventive device comprising a microwave generator component **500.** The microwave generator component **500** houses the vessel **400** and produces microwave radiation **501** within the chamber **502.** The microwave generator **500** is connected by electric signal **202** to the computing device **200** in order to control the process parameters, radiation power, radiation time and maximum temperature. The effective loading space **401** is fenced by the bottom inlet **402.** The bottom compartment **403** prevents the canalization of the fluid through the frit **404.** The frit **404** allows for the dispersion of the inert gas bubbling for mixing purposes. A microwave-compatible temperature sensor **405** measures the temperature within the reactor vessel **400.** A signal line **201** communicates the computing device **200** with the temperature sensor **405** for monitoring and control of the temperature. The channel **406** sprays the washing solvents by splitting the liquid among the series of holes at the tip of the channel. The conical end of channel **406** assures the complete dropping of the solvent. The inlets for building block line **407** and activator solution line **408** delivers at certain distance from the bottom surface of the reactor vessel cap **409.** The outlet **655** serves as gas exhaust to prevent over pressurizing the reaction vessel **400** during bubbling or washing solvent delivery. The single way valve **657** opens or closes the top gas exhaust exit **655**, and regulating the pressure inside of the reaction vessel **400.** The single way valve **657** is controlled by the computing device **200** in communication with the valve **657** via the signal line **213.** The cap **409** closes the reaction vessel, protecting it from the external atmosphere, as well as to seal off the microwaves chamber **502**, preventing the escape of microwave radiation **501.** The cooling coil or cooling jacket may be further provided with a microwave transparent thermal isolation cover 410 to cover the cooling jacket or cooling coil from exposure to the chamber to increase the heat exchange efficiency of the cooling jacket or cooling coil.

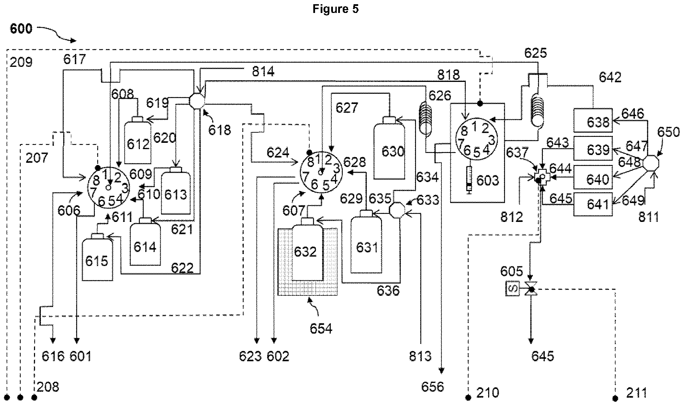

FIG. **5** is a conceptual diagram illustrating an example implementation of the liquid top delivery system **600.** The rotary valve **606** distributes the fluids within the building block distribution component **653.** The lines **608-611** allow to withdraw building block solutions from a building block storing component **663** comprising the containers **612-615.** The line **601** delivers building block solution to the reaction vessel by passing through the pre-cooling device **300.** The line **616** delivers excess building block to the waste container **704.** Line **617** supplies inert gas to the rotary valve distributor **606** from the splitter **618.** By the lines **619-622** the splitter **618** provides an anhydrous and inert gas atmosphere to the containers **612-615.** The signal line **207** delivers the command from the computing device **200** to the rotary valve distributor **606.** The loop line **625** communicates the rotary valve **606** with the syringe pump **603.** The line **656** discharges to the waste from the syringe pump **603.** The syringe pump **603** has incorporated a rotary valve that directs the withdrawing or dispensing action of the syringe pump to the lines **625-626**, **642**, **656**, **818**, respectively.

The rotary valve **607** distributes the fluids within the activator distribution component **652.** The lines **627-629** allows for withdrawal of activator solutions from an activator storing component **662** comprising the containers **630-632.** The line **602** delivers activator solution to the reaction vessel by passing through the pre-cooling component **300.** The line **623** delivers excess activator solution to the waste container **704.** The line **624** supplies inert gas to the rotary valve distributor **607** from the splitter **618.** By the lines **634-636** the splitter **633** provides an anhydrous and inert gas atmosphere to the containers **630-632.** The signal line **208** delivers the command from the computing device **200** to the rotary valve distributor **607.** The loop line **626** communicates the rotary valve **607** with the syringe pump **603.** The signal line **209** delivers the commands to the syringe pump **603.** One or more of the containers may be cooled for example by a cooling bath 654, which provides the mean to adjust the temperature of an activator reagent below the room temperature. For example, a Dewar filled with ice or a contact cooling device, such a Peltier cooling element may be used to provide cooling of the one or more containers.

The washing solvent distribution component **651** provides the reactor vessel **400** with the following exemplary washing solvents of a washing solvent storing component **661:** Dichloromethane line **643** from solvent container **639**, tetrahydrofuran line **644** from the solvent container **640**, dimethylformamide line **645** from solvent container **641.** The solvent container **638** provides 1,2-dichloroethane line to the syringe pump **603** as driving fluid by the line **642.** The order of delivery is controlled by the four way magnetic valve **637.** The washing line **645** delivers washing solvents to the top of the reaction vessel **400** by passing through the pre-cooling component **300**, which was regulated by the valve **605.** The magnetic valve **637** also serves to deliver inert gas to the top of the reactor vessel via the line **645.** The washing solvent containers **638-641** are pressurized with gas from the lines **646-649**, which is provided by the splitter **650.** The signal line **210** delivers the command from the computing device to the multiple ways valve **637** while the line **211** communicates to the valve **605.** The line **818** serves with gas the syringe pump **603.**

FIG. **6** is a conceptual diagram illustrating an example implementation of the liquid bottom delivery system **700.** The multiple ways magnetic valve **707** delivers and allows discharging fluids from the reaction vessel **400** by the line **701.** It is under control of the computing device **200** and the commands are delivered by the signal line **212.** A deprotection storing component **761** with the containers **708-709** provides deprotection reagents. The deprotection reagents are delivered from the containers **708-709** by the lines **705-706.** The splitter **712** provides anhydrous and inert gas to the containers **708-709** by the lines **714-715.** A capping storing component **762** with the containers **710-711** provides capping reagents. The capping reagents are delivered from the containers **710-711** by the lines **702-703.**

The splitter **713** provides anhydrous and inert gas to the containers **710-711** by the lines **716-717.** The line **718** delivers excess reagents to the waste container **704.**

FIG. **7** is a conceptual diagram illustrating an example implementation of the inert gas delivery system **800.** A gas container **801** supplies argon to a manifold **802** with seven valves **803-809** to control the pressure within the different lines. The gas lines that serve the driving fluid system or reagent delivery system are the solvent line **811**, the top pressure line **812** to push the liquid out of the reaction vessel **400** by the bottom exit **402**, the capping line **816** and the deprotection line **817.** The gas line **813** serves the activator distribution component **652**, and the gas line **814** serves the building block distribution component **653** to provide an anhydrous atmosphere within the reagent containers. The valve **810** controls the flow in the gas line **815** that provides the bubbling gas for the reaction vessel **400.**

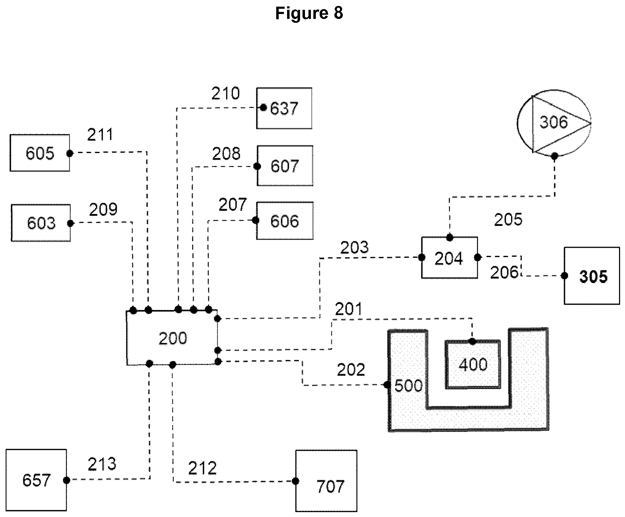

FIG. **8** is a block diagram illustrating a process of controlling components of the device of the present invention through a computing device **200** comprising at least one processor. The computing device **200** comprises a computer readable storage medium comprising computer readable instructions, which may be executed by the processor to allow to set the automated process sequences and the process variables, volume delivered, flow, temperature, microwave irradiation time and power. The signal line **201** communicates the computing device **200** with the sensor **405** for monitoring and control of the temperature. The line **202** communicates the microwave generator component **500** with the computing device **200.** The connection line **203** communicates the commands from the computing device comprising at least one processor **200** to a signal processor **204** that integrates and delivers commands by electronic signals **205** to the pump **306** and the line **206** to the Peltier cooler **305.** The lines **207-211** control the top delivery system **600** by communicating the rotary valves **606-607**, the syringe pump **603** and the rotary valve within, the single way valve **605**, and the multiple ways valve **637** with the computer **200.** The line **212** allows controlling of the bottom delivery system by connecting the multiple ways valve **707** with the computer **200.** The signal line **213** allows controlling of pressure inside of the reaction vessel through the single way valve **657.**

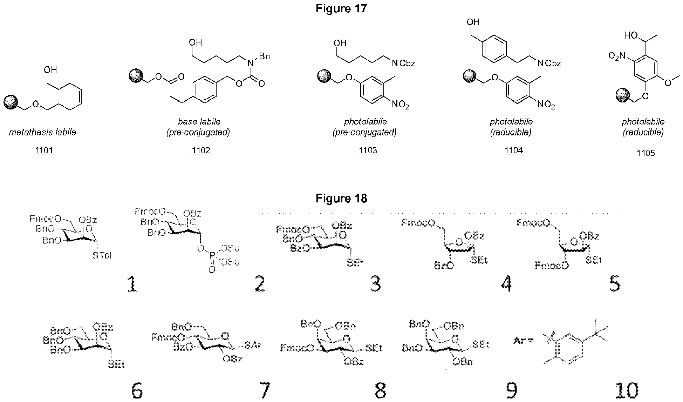

FIG. **17** depicts certain commonly used Merrifield resins functionalized with organic linkers, which were developed for the automated synthesis of oligo- and polysaccharides on a solid support. Resin **1101** is cleaved in the presence of metathesis-inducing Grubbs' second-generation catalyst to furnish an n-pentenyl glycoside that can serve as glycosylating agent. Resin **1102** is cleaved after treatment with sodium methoxide solution, affords conjugation-ready glycans to be printed on glycan arrays or for use in glycoconjugates. Photocleavable resins **1103**, **1104** and **1105** are considered "traceless" since the on-resin removal of photosensitive groups only require exposure to light to release the product into the solution. They have proven stable under both acidic and basic conditions and are compatible with a wide range of protecting groups such as carbonates (9-flourenylmethoxycarbonyl, Fmoc), esters (levulinoyl, Lev and benzoate, Bz) as well as ethers (benzyl, Bn). Resin **1103** is fast, easily and chemoselectively removed by photocleavage in a commercial flow photoreactor, and affords conjugation-ready glycans after global deprotection. Resin **1104**, a modified version of resin **1103**, was developed to obtain glycans with a free reducing end. These photocleavable linkers offer strategic and practical advantages, but cleavage efficiency is often below 50%. The development of modified linkers with higher cleavage efficiency led to resin **1105.** Modification of the ortho-nitrobenzyl scaffold is key to high yields and compatibility with the AGA workflow. Resin **1105** retains the excellent chemical orthogonality and enables access to protected glycans with a free reducing end. These in turn can be transformed into the glycosyl fluorides, phosphates, and trichloroacetimidates. Oligosaccharide fragments produced in this way can be united in block couplings to prepare more complex polysaccharides. Furthermore, free reducing glycans are valuable analytical standards to inspect fragmentation patterns in tandem mass spectrometry, or for glycosynthase assays that demand linker-free glycan epitopes as precursors.
FIG. **18** depicts a set of common building blocks (glycosyl donors) used for automated synthesis in the device of the present invention.

In preferred embodiments of the present invention the device may further comprise a light source for cleaving photo-cleavable linkers.

The present invention further relates to a device for automated synthesis of oligo- and polysaccharides on a solid support, the device comprising:
(a) a reaction vessel,
(b) a reagent storing component,
(c) a reagent delivery system,
(d) a cooling device for cooling the reaction vessel,
(e) a pre-cooling device for pre-cooling the reagents to be supplied, and
(f) a light source for cleaving photo-cleavable linkers.

In one embodiment the device may further comprise a microwave generator component and the reaction vessel may be microwave transparent.

### Method for automated synthesis of oligo- and polysaccharides on a solid support

The present invention further relates to a method for automated synthesis of oligo- and polysaccharides on a solid support with a device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied.
In one embodiment of the inventive method the device may further comprise a microwave generator component and the reaction vessel may be microwave transparent.

The present invention further relates to a method for automated synthesis of oligo- and polysaccharides on a solid support with a device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied.
(f) a computing device comprising at least one processor configured to control one or more components of the device.
In one embodiment of the inventive method the device may further comprise a microwave generator component and the reaction vessel may be microwave transparent.

The present invention further relates to a method for automated synthesis of oligo- and polysaccharides on a solid support with a device comprising:
(a) a microwave transparent reaction vessel,
(b) a microwave generator component,
(c) a reagent storing component,
(d) a reagent delivery system,
(e) a cooling device for cooling the microwave transparent reaction vessel,
(f) and a pre-cooling device for pre-cooling the reagents to be supplied,

the method comprising at least the following steps:
cooling, by the cooling device, the microwave transparent reaction vessel to a temperature range of -40°C to -10°C,
delivering, by the reagent delivery system, one or more reagents to the pre-cooling device,
adding, by the reagent delivery system, one or more pre-cooled reagents to the microwave transparent reaction at a temperature range of -40°C to -10°C.

The present invention further relates to a method for automated synthesis of oligo- and polysaccharides on a solid support with a device comprising:
(a) a reaction vessel,
(b) a reagent storing component,
(c) a reagent delivery system,
(d) a cooling device for cooling the reaction vessel,
(e) and a pre-cooling device for pre-cooling the reagents to be supplied,
the method comprising the steps of cooling, by the cooling device, the reaction vessel to a temperature range of -40°C to -10°C, receiving, by the reagent delivery system, one or more reagents from the reagent storing component, delivering, by the reagent delivery system, one or more reagents to the pre-cooling device, adding, by the reagent delivery system, one or more pre-cooled reagents to the reaction vessel for impregnation of the solid phase resin, adjusting, by the cooling device, the temperature of the reaction vessel to a temperature range of -1°C to +1°C for performing the coupling reaction, receiving, by the reagent delivery system, a deprotection solution from the reagent storing component, supplying, by the reagent delivery system, deprotection solution to the reaction vessel, repeating the previous steps for performing a further coupling cycle or terminating the synthesis process, and wherein one or more of the components of the device are under control of a computing device comprising at least one processor.

The present invention further relates to a method for automated synthesis of oligo- and polysaccharides on a solid support with a device comprising:
(a) a microwave transparent reaction vessel,
(b) a microwave generator component,
(c) a reagent storing component,
(d) a reagent delivery system,
(e) a cooling device for cooling the microwave transparent reaction vessel,
(f) and a pre-cooling device for pre-cooling the reagents to be supplied,
the method comprising the steps of cooling, by the cooling device, the microwave transparent reaction vessel to a temperature range of -40°C to -10°C, receiving, by the reagent delivery system, one or more reagents from the reagent storing component, delivering, by the reagent delivery system, one or more reagents to the pre-cooling device, adding, by the reagent delivery system, one or more pre-cooled reagents to the microwave transparent reaction vessel for impregnation of the solid phase resin, adjusting, by the cooling device, the temperature of the microwave transparent reaction vessel in the temperature range of -1°C to +1°C for performing the coupling reaction, receiving, by the reagent delivery system, deprotection solution from the reagent storing component, supplying, by the reagent delivery system, deprotection solution to the reaction vessel, providing, by the microwave generator component, microwave radiation to the reaction vessel, repeating the previous steps for performing a further coupling cycle or terminating the synthesis process, wherein one or more of the components of the device are under control of a computing device comprising at least one processor.

### Description of the figures

FIG. **1A** is a block diagram illustrating the inventive device for automated oligo- and polysaccharide synthesis on solid support; **B** shows a diagram of an example of the device comprising two reagent delivery systems (600, 700) in accordance with one or more aspects of the invention; **C** shows a diagram of an example of the device comprising a microwave generator component (500).
FIG. **2** is a flow diagram illustrating components of FIG. **1A-C** in more details, and more particularly, the inert gas and liquid distribution, in accordance with one or more aspects of the invention.
FIG. **3A** is a conceptual diagram illustrating a side view of an example implementation of the pre-cooling device (300), in accordance with one or more aspects of the invention; **B** shows a compact embodiment of the pre-cooling device (300) and cooling device (350) comprising a cooling jacket (307) forming together a single component of the inventive device; **C** shows a compact embodiment of the pre-cooling device (300) and cooling device (350) comprising a cooling coil (307) forming together a single component of the inventive device.
FIG. **4A** is a first conceptual diagram illustrating a cross-section view of an example implementation of a reaction vessel, in accordance with one or more aspects of the invention; **4B** is a second conceptual diagram illustrating a cross-section view of an example implementation of a reaction vessel, in accordance with one or more aspects of the invention.
FIG. **5** is a conceptual diagram illustrating an example implementation of the liquid top delivery system, in accordance with one or more aspects of the invention.
FIG. **6** is a conceptual diagram illustrating an example implementation of the liquid bottom delivery system, in accordance with one or more aspects of the invention.
FIG. **7** is a conceptual diagram illustrating an example implementation of the inert gas delivery system, in accordance with one or more aspects of the invention.
FIG. **8** is a block diagram illustrating a process of controlling components of the device through a computer program, in accordance with one or more aspects of the invention.
FIG. **9** shows the thermal profile during the synthesis of a mannose-α-1,6-tetramer "standard test" in the presence and absence of pre-cooling. The reaction vessel is made of glass, using thioglycoside donor as building block, and active cooling is ON in both experiments. By using a precooling device, the thermal perturbation due to the delivery of reagents is suppressed (solid line), specifically the delivery of activator reagents and donor solution. The washing solvent was not pre-cooled to highlight the stark contrast in the thermal spike.
FIG. **10** shows the analytical results of the experiments described in FIG. 9 and mass spectrometry (MALDI) and HPLC chromatograms.
FIG. **11** shows the thermal profile during the synthesis of a mannose-α-1,6-tetramer "standard test" in the presence and absence of pre-cooling. The reaction vessel is made of glass, using glycosyl phosphate donor as building block, and active cooling is ON in both experiments. By using a pre-cooling device, the thermal perturbation due to the delivery of reagents is suppressed (solid line), specifically the delivery of activator reagents and donor solution. The washing solvent was not pre-cooled to highlight the stark contrast in the thermal spike.
FIG. **12** shows the analytical results of the experiments described in FIG. 11 and mass spectrometry (MALDI) and HPLC chromatogram. The reaction without the pre-cooling shows a HPLC chromatogram with more side product traces (peaks at 2.3 min and 3.6 min).
FIG. **13** shows the thermal profile during the synthesis of a mannose-α-1,6-tetramer "standard test" in the presence and absence of precooling. The reaction vessel is made of PFA, using glycosyl phosphate donor as building block, and active cooling is OFF in both experiments. By using a pre-cooling device, there is a brief drop in temperature when the pre-cooled reagents/building blocks are delivered (solid line). In both cases the temperature oscillates around 20 °C.
FIG. **14** shows the analytical results of the experiments described in FIG. 13 and HPLC chromatograms. In both experiments no desired product was observed. Only side products are visible in HPLC chromatogram (peaks at 2.3 min and 3.4 min).
FIG. **15** shows the temperature differential (maximum temperate reached minus the actual reactor temperature) within a glass reaction vessel when 1 mL of dichloromethane (DCM) is delivered at different flow rates to a reactor vessel at -18 °C. The white circle series refers to the solvent delivered without precooling; the black triangle series refers to precooled solvent. By using the precooling, the reagent can be delivered 3 times faster than the current state of the art with a thermal perturbation lesser than ±3 °C (highlighted in box).
FIG. **16** shows the thermal perturbation in the reaction vessel when different volumes of Dichloromethane are delivered at 1.05 mL/min in a reaction vessel at -18 °C in the presence or absence of pre-cooling. The solid line refers to the thermal perturbation with precooling. The dash line refers to the thermal perturbation without pre-cooling. The pre-cooling reduces the thermal perturbation even when 4 mL are delivered which is 4 times the current scale of the current state of the art synthesizer.
FIG. **17** depicts certain commonly used Merrifield resins 1101, 1102, 1103, 1104 functionalized with organic linkers, which were developed for the automated synthesis of oligo- and polysaccharides on a solid support.
FIG. **18** shows a set of common building blocks (glycosyl donors) used for automated synthesis in the inventive device.

### Abbreviations

Ac₂O= Acetic anhydride
ClAc = Chloroacetyl
DBU = 1,8-Diazabicyclo[5.4.0]undec-7-ene
DDQ = 2,3-Dichloro-5,6-dicyano-1,4-benzoquinone
EGME = 2-Methoxyethanol
Fmoc = Fluorenylmethyloxycarbonyl
HFIP = Hexafluoroisopropanol
Lev = Levulinic ester
MsOH = Methanesulfonic acid
NAP = 2-Naphthylmethyl ether
NDM = 1-Dodecanethiol
TIS = Triisopropylsilane
DCM = Dichloromethane
DMF = N-N-Dimethylformamide
DCE = 1,2-Dichloroethane
THF = Tetrahydrofuran

### List of Reference Signs

- 100: device
- 200: computing device, processor
- 201-203: connection line / communication line
- 204: signal processor
- 207-213: communication lines
- 300: pre-cooling device / pre-cooling component
- 303: metal cooling surface
- 304: coolant fluid reservoir / coolant reservoir
- 305: Peltier cooler / contact-cooling device
- 307: cooling coil / cooling jacket
- 308,309: lines
- 310: cooling line
- 311: cooling means
- 350: cooling device for reaction vessel
- 400: reaction vessel
- 402: bottom inlet / bottom exit
- 403: bottom compartment
- 404: frit
- 405: temperature sensor / sensor
- 407: building block line
- 408: activator solution line
- 409: cap
- 500: microwave generator component / microwave generator
- 502: chamber / microwaves chamber
- 600: reagent delivery system, top delivery system
- 601: building block delivery line
- 602: activator solution delivery line
- 603: syringe pump
- 606: rotary valve / rotary valve distributor
- 608-611: lines
- 612-615: containers
- 616,617: lines
- 618: splitter
- 619-622: lines
- 625, 626: loop lines
- 627-629: lines
- 630-632: containers
- 637: multiple ways valve / four way magnetic valve
- 639-641: solvent containers
- 645: delivery line
- 646-649: lines
- 651: washing solvent distribution component/washing solvents component
- 652: activator distribution component
- 653: building blocks distribution component
- 654: cooling means
- 655: exhaust gases exit
- 656: waste delivery line
- 657: single valve
- 660, 760: reagent storing component
- 661: washing solvent storing component
- 662: activator storing component
- 663: building block storing component
- 700: reagent delivery system, bottom delivery system
- 701: loop line / line
- 702, 703: lines
- 704: waste container / waste collector
- 705,706: lines
- 707: multi-port valve / multiple ways magnetic valv
- 708-711: containers
- 712: splitter
- 714-718: lines
- 719: deprotection distribution component
- 720: capping distribution component
- 761: deprotection storing component
- 762: capping storing component
- 800: inert gas delivery system
- 801: gas container
- 802: manifold
- 803-809: seven valves
- 810: valve
- 811: solvent line
- 812: pressure line
- 813-815: gas lines
- 816: capping line
- 817: deprotection line
- 1001-1005: resins

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Example 1: Investigation of microwave-assisted capping and deprotection steps

The improvement in conditions and reaction time for the capping and deprotection steps with microwave radiation was investigated.

During a typical AGA cycle there is the glycosylation coupling step as well as several auxiliary steps (acetyl capping and temporary group deprotection). These auxiliary steps increase the overall yield of the final oligo- or polysaccharide (glycan) by terminating unglycosylated nucleophiles as well as they remove temporary protecting groups that allows the next coupling to occur. These auxiliary steps have been a bottleneck in the overall time required for one AGA cycle. The investigation of the overall time required for one AGA cycle by using microwave radiation during these auxiliary steps has shown that microwave assisted deprotection and capping steps drastically reduce the reaction time. The results shown in Table **1** demonstrate that the utilization of a microwave generator component is not only instrumental for hastening the cooling to heating process, microwave-assisted synthesis also drastically reduces chemical reaction time. Under these rapid microwave-assisted conditions, the steps remained orthogonal and few side reactions were observed. With shortened reaction times for these auxiliary steps the overall duration of a standard AGA cycle was successfully reduced from 100 minutes to below 60 minutes and even to 45 minutes. First results have also shown that the glycosylation reaction benefits from the use of microwave radiation.

**Table 1**

| **Module** | **Without Microwave** | | **With Microwave Radiation** | |
|---|---|---|---|---|
| | **Conditions** | **Time** | **Conditions** | **Time** |
| Capping | 2% MsOH in CH₂Cl₂/Ac₂O (5:1), 25°C | 25 min | 2% MsOH in CH₂Cl₂/Ac₂O (5:1), 45 °C | 1 min |
| Deprotection: Lev | 7% N₂H₄ HOAc in CH₂Cl₂/Pyr/HOAc/H₂O (20:16:4:1), 25°C | 90 min | 7% N₂H₄ HOAc in CH₂Cl₂/Pyr/HOAc/H₂O (20:16:4:1), 40 °C | 1 min |
| Deprotection: NAP | 2% DDQ in DCE/MeOH/H₂O (64:16:1), 40°C | 120 min | 0.5% MsOH in CH₂Cl₂/HFIP/TIS (20:2:1), 40 °C | 1 min |
| Deprotection: Fmoc | 20% Piperidine in DMF, 25 °C | 5 min | 20% Piperidine in DMF, 40 °C | 1 min |
| Deprotection: ClAc | Thiourea in EGME, 80°C | 360 min | Thiourea in EGME, 80°C | 45 min |

### Example 2: Investigation of temperature fluctuations of the reaction mixture by addition of pre-cooled building block and activator solutions

The temperature development inside of the reaction vessel during a synthesis cycle in the presence and absence of pre-cooling device was investigated. FIG. **9** shows the results charts comparing temperature readings inside the reaction vessel, taken during a synthesis cycle, in the presence and absence of a pre-cooling device.

The three thermal stages are shown. Temperature spikes appear when a liquid is dispensed in the reaction vessel. The dashed curve shows the temperature profile for the device without pre-cooling of the reagents. The thermal spikes are remarkable at the pre-coupling regime (subzero temperatures). The solid line depicts the temperature profile with active pre-cooling. The incoming solution of reagents is pre-cooled and this suppresses the temperature spikes. Since there are three thermal stages during a glycosylation cycle: (1) The pre-coupling regime (-40 °C to -10 °C). The building block and activator are allowed to impregnate the resin and for diffusion through the porous solid. The low temperature prevents the early decomposition of the intermediate before the actual coupling; (2) the coupling regime (around 0°C). The increase in the temperature allows for initiating the coupling reaction by promoting the formation of the intermediates; and (3) the post-coupling regime (room temperate or above). The capping and deprotection reactions take place at higher temperature. These reactions close out the glycosylation cycle. Then, the next coupling or process termination takes place.

In Fig. 9 the thermal profile during the synthesis of a mannose-α-1,6-tetramer "standard test" in the presence and absence of pre-cooling is shown. Here, the reactor is made of glass and a thioglycoside donor is used as building block, and active cooling of the reaction vessel by a cooling device is ON in both experiments. By using a pre-cooling device according to the present invention, the thermal perturbation due to the delivery of reagents is suppressed (solid line), specifically the delivery of activator reagents and donor solution. In this example, the washing solvent was not pre-cooled to highlight the strong contrast in the thermal spike.

In Fig. 10 the analytical results of the experiments as described in Fig 9 are shown. Mass spectrometry (MALDI) and HPLC chromatogram are provided. The experiment performed in presence of a pre-cooling device shows superior results: only the desired product was obtained. An overall yield of 60% was achieved. The reaction without the pre-cooling delivers a mixture of tetra-mannose and some partially deprotected tetra-mannose. This further complicates the purification process and results in lower overall yield.

In Fig. 11 the thermal profile during the synthesis of a mannose-α-1,6-tetramer "standard test" in the presence and absence of pre-cooling is demontrated. The reaction vessel is made of glass, and a glycosyl phosphate donor is used as building block, and active cooling of the reaction vessel by a cooling device is ON in both experiments. By using a pre-cooling element, the thermal perturbation due to the delivery of reagents is suppressed (solid line), specifically the delivery of activator reagents and donor solution. The washing solvent was not pre-cooled to highlight the stark contrast in the thermal spike.

In Fig. 12 the analytical results of the experiments as shown in Fig.11 are shown. Mass spectrometry (MALDI) and HPLC chromatogram are provided. The experiment performed in presence of a pre-cooling device shows superior results: only the desired product was obtained. An overall yield of 60% was achieved. The reaction without the precooling shows a HPLC chromatogram with more side product traces (peaks at 2.3 min and 3.6 min).

In Fig. 13 the thermal profile during the synthesis of a mannose-α-1,6-tetramer "standard test" in the presence and absence of pre-cooling is demonstrated. The reaction vessel is made of PFA, and a glycosyl phosphate donor is used as building block, and active cooling of the reaction vessel by a cooling device is OFF in both experiments. By using a pre-cooling element, there is a brief drop in temperature when the pre-cooled reagents/building blocks are delivered (solid line). In both cases the temperature oscillates around 20 °C. In Fig. 14 the analytical results of the experiments as described in Fig 13 are shown. HPLC chromatogram are provided. In both experiments no desired product was observed. Only side products are visible in HPLC chromatogram (peaks at 2.3 min and 3.4 min). Precise control of the temperature is instrumental during the automated synthesis of oligosaccharides.

In Fig. 15 the temperature differential (maximum temperate reached minus the actual reaction vessel temperature) within a glass the reaction vessel when 1 mL of Dichloromethane is delivered at different flow rates to a reactor vessel at -18 °C is shown. The white circle series refer to the solvent delivered without pre-cooling; the black triangle series refers to pre-cooled solvent. By using the pre-cooling device, the reagent can be delivered 3 times faster than the current state of the art with a thermal perturbation lesser than ±3 °C (highlighted in box).

In Fig. 16 the thermal perturbation in the reaction vessel when different volumes of Dichloromethane are delivered at 1.05 mL/min in a reactor vessel at -18 °C in the presence or absence of pre-cooling is shown. The solid line refers to the thermal perturbation with pre-cooling. The dash line refers to the thermal perturbation without pre-cooling. The pre-cooling reduces the thermal perturbation even when 4 mL are delivered which is 4 times the current scale of the current state of the art synthesizer. This presents a major advantage of the present invention in the up-scaling the synthesis of oligosaccharides.

The advantages and improvement of the automated glycosylation via pre-cooling of the reagents are most apparent on Figure 10 and 12. Furthermore, it has been found that by pre-cooling of the reagents, the overall duration of a synthesis cycle could be reduced as the pre-cooling device allows rapid delivery of pre-cooled reagents. By using the pre-cooling device of the present invention, the donor could be delivered 3 times faster than the current state of the art devices; this was achieved without a thermal perturbation larger than 3 °C compared to the actual reaction vessel temperatures. In addition, the precooling reduces the thermal perturbation even when 4 mL are delivered which is 4 times the current scale of the current state of the art synthesizers. This presents a major advantage of the present invention in the up-scaling the synthesis of oligosaccharides.

### Example 3: Synthesis of mannose tetramer via thioglycoside donor

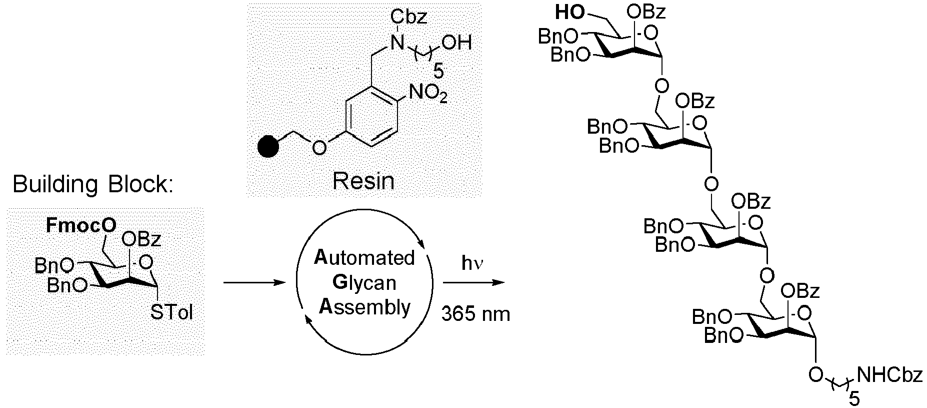

### Automated synthesis working modules

The current example was performed with and without precooling of the activators solution and building block/donor solution; within a glass reaction vessel.

The timing and quantity of solvents/reagents transferred to the reaction vessel in each step is controlled by the software. The reagent delivery system is based on valve-pressured control in which the entire platform is constantly pressurized so that the specific solvent/reagent is transferred from the respective storage components by timing the opening and closing of the appropriate valves. Some of the reagents are delivered by the withdrawing and/or dispensing action of a syringe pump. Before start the modules, the program initializes all the components (valves, active cooling, precooling, pump, sensors and actuators); renews the driving solvent in the loops **625** and **626;** the program empties and flushes the reagents delivery lines.
Module 1: Acidic Wash: The resin loaded into the reaction vessel is washed with DMF, THF, DCM (six times each with 3 mL for 15 s). The resin is swollen in 2 mL DCM, and the temperature of the reaction vessel was adjusted in the range of -18 °C to -17 °C. For acidic washing, 1 mL of the solution of TMSOTf is delivered to the reaction vessel via the pre-cooling device which reaches a temperature of -15 °C. After three minutes, the solution is drained. Finally, 3 mL DCM is added to the reaction vessel, incubated for 15 s, and drained out.
Module 2: Glycosylation: Thioglycoside building block is dissolved in the proper solvent mixture (4 mL for four glycosylation cycles) in the designated building block storing component. The reaction vessel is set to reach the initial glycosylation temperature. During the adjustment of the temperature, the DCM in the reaction vessel is drained and 1 mL of thioglycoside building block (6.5 eq. in 1.0 mL DCM) is delivered from the building block storing component to the reaction vessel via the pre-cooling device which cools the solution of thioglycoside building block to a temperature of -15 °C. After the temperature reached the range of -18 °C to -17 °C °C, 1.0 mL NIS and TfOH solution in DCM and dioxane (v/v, 2:1) is delivered to the reaction vessel from the respective activator storing component via the pre-cooling device which cools the activator solution down to a temperature of -15 °C. The glycosylation mixture is incubated for 5 min at the temperature range of -18 °C to -17 °C, linearly ramped to 0 °C, and after reaching 0 °C the reaction mixture is incubated for an additional 20 min. Once incubation time is finished, the reaction mixture is drained and the resin is washed with DCM (once, 2 mL for 15 s). Then the resin is washed with 2 mL of Dioxane. Finally the resin is washed twice with DCM (2 mL for 15 s).
Module 3: Capping: The temperature of the reaction vessel is adjusted to 25 °C. The resin is washed twice with DMF (3 mL for 15 s). 2 mL of Pyridine solution (10% in volume in DMF) was delivered and the resin is incubated for one minute. The resin is washed three times with DCM (2 mL for 15 s). The capping of the unreacted acceptor groups is done by delivering 4 mL of Methanesulfonic acid (2% in volume) and Acetic anhydride (10% in volume) in DCM. The resin and the reagents are incubated for 10 min; then 1 mL of DCM in added to dilute the solution and the incubation continues for another 10 min. The solution is drained from the reaction vessel and the resin is washed 3 time with DCM (2 mL for 15 s).
Module 4: Fmoc Deprotection: The resin is washed with DMF (three times with 3 mL for 15 s), swollen in 2 mL DMF and the temperature of the reaction vessel is adjusted to 25 °C. For Fmoc deprotection the DMF is drained and 2 mL of a solution of 20% Piperidine in DMF was delivered to the reaction vessel. After 5 min the reaction solution is drained from the reactor vessel. Then, the resin is washed with DMF (three times with 3 mL for 15 s) and DCM (five times with 3 mL). After this module the resin is ready for the next glycosylation cycle.

Resin **1103** (see Fig **17**) functionalized with a photo-cleavable linker (40 mg; loading 0.33 mmol/g; 25.1 µmol) was loaded into the reaction vessel of the synthesizer and swollen in 2 mL DCM. The sequence of reaction steps for the formation of mannose tetramer was as follows:
1. Module 1 was performed with 1 mL TMSOTf solution at -20 °C for 3 min.
2. Module 2 was performed with 5 equiv. building block **1** (see Fig. **18**) and 5 equiv. NIS solution.
3. Module 3 was carried out in two steps; first 2 mL of Pyridine solution (10% in volume in DMF); then in a second step with 4 mL of Methanesulfonic acid (2% in volume) and Acetic anhydride (10% in volume) in DCM.
4. Module 4 was carried out with 20% Piperidine in DMF.
5. Subsequently, modules 1 -4 were repeated four times in the same manner as described in steps 1-4 in order to obtain a tetramer.

After buildup of the tetramer on the resin the oligosaccharide was cleaved from solid support in a photoreactor: A mercury lamp is turned on 30 min prior to the first cleavage event. The fluorinated-ethylene-propylene (FEP) tubing was washed with 20 mL DCM at a flow rate of 5 mL/min before cleavage. The solid support was pre-swelled in the dark in DCM for 30 min at least before being taken up with a 20 mL disposable syringe. The suspension of solid support in DCM was slowly injected from the disposable syringe (20 mL) into the FEP tubing using a syringe pump. The suspension was pushed through the FEP tubing into the photoreactor with additional 18 mL DCM (flow rate: 700 µL/min). The photocleavage took place inside the reactor while solid support travelled toward the exit point of the reactor. The suspension leaving the reactor was directed into a syringe equipped with polyethylene filter frit where the resin was filtered off and the solution containing the cleaved oligosaccharide is collected in a separate glass vial. The tubing as washed with 20 mL DCM (flow rate: 2 mL/min) until any remaining resin exited the reactor and the remaining oligosaccharide solution is collected. The tubing was reequilibrated with 20 mL DCM using a flow rate of 5 mL/min and the entire cleavage procedure was repeated. The combined solution that was collected in the photocleavage process was evaporated in vacuo and the crude material was analyzed by MALDI-TOF, and HPLC.

### Example 4: Synthesis of mannose tetramer via phosphate donor

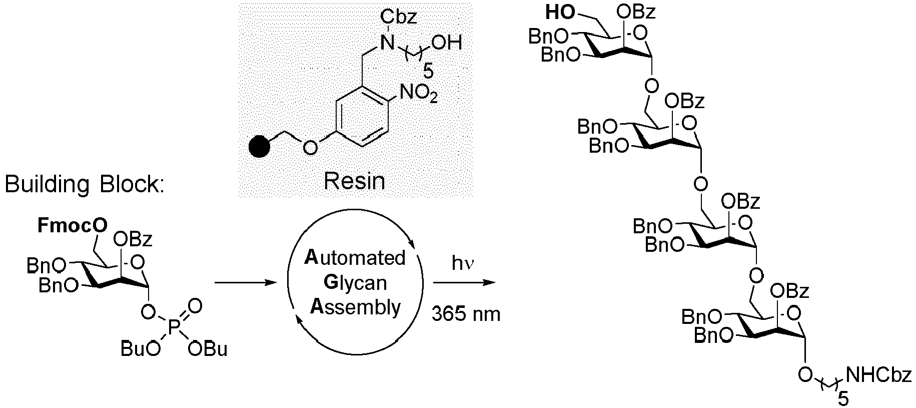

### Automated synthesis working modules

The current example was performed within a PFA/glass reactor, in a factorial experimental design with two factors: active cooling of the reaction vessel, and precooling action of the activators solution and building block/donor solution, each factor had two levels with and without active temperature control (on and off stage of the corresponding device).

The timing and quantity of solvents/reagents transferred to the reaction vessel in each step is controlled by the software. The reagent delivery system is based on valve-pressured control in which the entire platform is constantly pressurized so that the specific solvent/reagent is transferred from the respective storage components by timing the opening and closing of the appropriate valves. Some of the reagents are delivered by the withdrawing and/or dispensing action of a syringe pump. Before start the modules, the program initializes all the components (valves, active cooling, precooling, pump, sensors and actuators); renews the driving solvent in the loops **625** and **626;** the program empties and flushes the reagents delivery lines.
Module 1: Acidic Washing: The resin loaded into the reaction vessel is washed with DMF, THF, DCM (six times each with 3 mL for 15 s). The resin is swollen in 2 mL DCM, and the temperature of the reaction vessel was adjusted in the range of -18 °C to -17 °C by cooling device (when it is programed to do so). For acidic washing 1 mL of the solution of 2% TMSOTf in DCM is delivered to the reaction vessel via the pre-cooling device which cools the solution to a temperature of -15°C (when it is programed to do so). After three minutes, the solution is drained. Finally, 3 mL DCM is added to the reaction vessel.
Module 2: Glycosylation: Phosphate building block is dissolved in the proper solvent mixture e.g. DCM (5 mL for one initial double cycle for the first coupling and three more single glycosylation cycles to build up the tetramer) in the designated building block storing component. The reaction vessel is set to reach the initial glycosylation temperature. During the adjustment of the temperature in the reactor vessel (when it is programed to do so), the DCM in the reaction vessel is drained and 1 mL of phosphate building block 2 (5.0 eq. in 1.0 mL DCM) is delivered from the building block storing component to the reaction vessel via the pre-cooling device which cools the solution of phosphate building block (when it is programed to do so) to a temperature of -15 °C. After the set temperature in the range of -18 °C to -17 °C is reached the resin is incubated in the solution of phosphate building block for 10 min. Then 1.0 mL of the solution of 2% TMSOTf in DCM is delivered to the reaction vessel from the respective activator storing component via the pre-cooling device which cools the activator solution down to a temperature of -15 °C (when it is programed to do so). The glycosylation mixture is incubated for 30 min in the temperature range of -18 °C to -17 °C, linearly ramped to 0 °C (when it is programed to do so), and after reaching 0 °C the reaction mixture is incubated for an additional 10 min. Once incubation time is finished, the reaction mixture is drained and the resin is washed with DCE (once, 2 mL for 5 s).
Module 3: Capping: The temperature of the reactor vessel is adjusted to 25 °C. The resin is washed twice with DMF (3 mL for 15 s). 2 mL of Pyridine solution (10% in volume in DMF) was delivered and the resin is incubated for one minute. The resin is washed three times with DCM (2 mL for 15). The capping of the unreacted acceptor groups is done by delivering 4 mL of Methanesulfonic acid (2% in volume) and Acetic anhydride (10% in volume) in DCM. The resin and the reagents are incubated for 10 min; then 1 mL of DCM in added to dilute the solution and the incubation continues for another 10 min. The solution is drained from the reactor vessel and the resin is washed 3 time with DCM (2 mL for 15 s).
Module 4: Fmoc Deprotection: The resin is washed with DMF (three times with 3 mL for 15 s), swollen in 2 mL DMF and the temperature of the reaction vessel is adjusted to 25 °C. For Fmoc deprotection the DMF is drained and 2 mL of a solution of 20% Piperidine in DMF was delivered to the reaction vessel. After 5 min the reaction solution is drained from the reactor vessel. Then, the resin is washed with DMF (three times with 3 mL for 15 s) and DCM (five times with 3 mL). After this module the resin is ready for the next glycosylation cycle.

Resin **1103** (see Fig **17**) functionalized with a photo-cleavable linker (40 mg; loading 0.33 mmol/g; 25.1 µmol) was loaded into the reaction vessel of the synthesizer and swollen in 2 mL DCM. The sequence of reaction steps for the formation of mannose tetramer was as follows:
1. Module 1 was performed with 1 mL TMSOTf solution at the temperature range of -18 °C to -17 °C (when it is programed to do so) for 3 min.
2. Module 2 was performed with 5 equiv building block **2** (see Fig. **18**) and 2% TMSOTf in DCM solution.
3. Module 3 was carried out in two steps; first 2 mL of Pyridine solution (10% in volume in DMF); then in a second step with 4 mL of Methanesulfonic acid (2% in volume) and Acetic anhydride (10% in volume) in DCM.
4. Module 4 was carried out with 20% Piperidine in DMF.
5. After Module 1 took place the Module 2 repeated twice (for the first coupling). The Modules 3 and 4 were then performed.
6. Subsequently, modules 1-4 were repeated three times in the same manner as described in steps 1-4 in order to obtain a tetramer.

After buildup of the tetramer on the resin the oligosaccharide was cleaved from solid support in a photoreactor: A mercury lamp is turned on 30 min prior to the first cleavage event. The fluorinated-ethylene-propylene (FEP) tubing was washed with 20 mL DCM at a flow rate of 5 mL/min before cleavage. The solid support was pre-swelled in the dark in DCM for 30 min at least before being taken up with a 20 mL disposable syringe. The suspension of solid support in DCM was slowly injected from the disposable syringe (20 mL) into the FEP tubing using a syringe pump. The suspension was pushed through the FEP tubing into the photoreactor with additional 18 mL DCM (flow rate: 700 µL/min). The photocleavage took place inside the reactor while solid support travelled toward the exit point of the reactor. The suspension leaving the reactor was directed into a syringe equipped with polyethylene filter frit where the resin was filtered off and the solution containing the cleaved oligosaccharide is collected in a separate glass vial. The tubing as washed with 20 mL DCM (flow rate: 2 mL/min) until any remaining resin exited the reactor and the remaining oligosaccharide solution is collected. The tubing was reequilibrated with 20 mL DCM using a flow rate of 5 mL/min and the entire cleavage procedure was repeated. The combined solution that was collected in the photocleavage process was evaporated in vacuo and the crude material was analyzed by MALDI-TOF, and HPLC.

## Claims

1. A device for automated synthesis of oligo- and polysaccharides on a solid support, the device (100) comprising:
(a) a reaction vessel (400),
(b) a reagent storing component (660),
(c) a reagent delivery system (600),
(d) a cooling device (350) for cooling the reaction vessel (400),
(e) and a pre-cooling device (300) for pre-cooling the reagents to be supplied.

2. The device according to claim 1 further comprising a computing device (200) comprising at least one processor configured to control one or more components of the device.

3. The device according to claim 1 or 2, the device being adapted for reactions under anhydrous and inert atmosphere.

4. The device according to any one of claims 1 - 3, further comprising a microwave generator component (500) having a chamber in which the reaction vessel (400) is located, and wherein the reaction vessel is microwave transparent.

5. The device according to any one of claims 1 - 4, the pre-cooling device (300) being in thermal communication with the reagent delivery system (600).

6. The device according to any one of claims 1 - 5, wherein the pre-cooling device (300) is configured to cool the reagents to be supplied to the reaction vessel a temperature which is not higher than 3°C below the temperature of the reaction mixture in the reaction vessel.

7. The device according to any one of claims 1 - 6, wherein the pre-cooling device (300) is configured to cool the reagents to be supplied to the reaction vessel to a temperature in the range of -14°C to -10°C.

8. The device according to any one of claims 1 - 7, wherein the cooling device (350) comprises a cooling jacket.

9. The device according to any one of claims 1 - 8 further comprising an inert gas delivery system (800).

10. The device according to any one of claims 1 - 9, wherein the pre-cooling device (300) is upstream to the cooling device (350).

11. The device according to any one of claims 1 - 10, wherein the reaction vessel (400) is interchangeable.

12. The device according to any one of claims 1 - 11, wherein the reaction vessel (400) is made of a fluoropolymer such as perfluoroalkoxy alkanes (PFA) or glass.

13. The device according to any one of claims 1 - 12, wherein the reaction vessel (400) comprises one or more inlets at the top of the reaction vessel and one or more inlets at the bottom of the reaction vessel.

14. The device according to any one of claims 1 - 13, wherein the reagent delivery system (600) connects the reagent storing component (660) with the reaction vessel (400) via the pre-cooling device (300).

15. The device according to any one of claims 1 - 14, wherein the pre-cooling device (300) is a part of the cooling device (350) using the same cooling circuit together.
